# EUROPEAN PATENT APPLICATION

(11) **EP 3 335 720 A1**
(43) Date of publication of application: **20.06.2018**
(21) Application number: 16205009.0
(22) Date of filing: 19.12.2016
(51) Int. Cl.: A61K 38/00

(54) **C-DI-GMP BINDING PEPTIDES**

(71) Applicant: Universität Basel, 4001 Basel (CH)
(72) Inventor: Hee, Chee Seng, 4056 Basel (CH); Schmutz, Christoph, 4052 Basel (CH); Jenal, Urs, 4054 Basel (CH); Schirmer, Tilman, 79400 Kandern (DE); Habazettl, Judith Maria, 4125 Riehen (CH); Grzesiek, Stephan, 79599 Wittlingen (DE)
(74) Representative: Latscha Schöllhorn Partner AG

(57) **Abstract**

The present invention is directed to a peptide comprising at least one amino acid sequence (I), wherein said amino acid sequence (I) comprises, preferably consists of, the amino sequence D1-X2-X3-X4-X5-X6-X7-X8-X9-Y10-X11-G12-X13-R14-R15-R16-X17-X18-D19, a composition and a pharmaceutical composition comprising the peptide of the invention, the peptide of the invention for use as a medicament in the treatment or prevention of a disease, non-medical use of the peptide of the invention, and a nucleic acid encoding the peptide of the invention.

## Description

The present invention relates to the field of peptides, preferably of c-di-GMP binding peptides. In particular, the present invention concerns a peptide comprising at least one amino acid sequence (I), wherein said amino acid sequence (I) comprises, preferably consists of, the amino sequence D1-X2-X3-X4-X5-X6-X7-X8-X9-Y10-X11-G12-X13-R14-R15-R16-X17-X18-D19, a composition and a pharmaceutical composition comprising the peptide of the invention, the peptide of the invention for use as a medicament in the treatment or prevention of a disease, non-medical use of the peptide of the invention and a nucleic acid encoding the peptide of the invention.

### RELATED ART

Bacteria can occur as planktonic organisms (i.e. free-floating) or as biofilm (i.e. mentioned herein as "biofilm bacteria"), in which bacteria are clustered and encapsulated in an extracellular polymeric substance. Compared to planktonic bacteria, biofilm bacteria predominate in virtually all ecosystems under sufficient-nutrient conditions. If biofilm bacteria were simply planktonic cells that had adhered to a surface, this revelation would be unimportant, but biofilm bacteria are profoundly different to planktonic bacteria. For example, biofilm bacteria are up to 1.000 times more resistant to antibiotics than planktonic bacteria. The reason why antibiotics fail to eradicate biofilm bacteria is the compact nature of biofilm structures and the protection conferred by biofilm polymers. Moreover, biofilm bacteria are highly resistant to innate as well as adaptive immune system responses and clearance. Thus, bacteria can persist despite an intact host immune defense and frequent antibiotic treatments (Costerton et al., Annu. Rev. Microbiol. 1995; Davies, Nat. Rev. Drug Discov. 2003).

The number of diseases associated with bacterial biofilms is considered to be quite large, with colitis, vaginitis, urethritis, conjunctivitis and otitis being just a short list of common examples. Biofilms are also important as colonizers of medical devices, including urinary, venous and arterial catheters, shunts and the like. Since bacteria present in a biofilm resist antibiotics as well as immune defense, biofilm-related infections tend to be chronic, such as bronchopulmonary *Pseudomonas aeruginosa* (*P. aeruginosa*) infections in cystic fibrosis (CF) patients.

To date, there are no drugs available that specifically target bacteria in biofilms. Therefore, treatment of biofilm-related infections has proven a considerable unmet clinical need (Davies, Nat. Rev. Drug Discov. 2003; Bjarnsholt et al., Nat. Rev. Drug Discov. 2013).

An approach for promoting biofilm dispersal is the interference with intracellular levels of cyclic dimeric guanosine monophosphate (c-di-GMP). Intracellular signaling mediated by the second messenger c-di-GMP has been identified as a mechanism for switching between planktonic and bio-film associated "lifestyles" of bacteria and regulation of biofilm formation (Schirmer et al., Nat. Rev. Microbiol. 2009; Hengge, Nat. Rev. Microbiol. 2009). For example, reducing intracellular c-di-GMP levels by overproducing c-di-GMP specific phosphodiesterases, which break down c-di-GMP, has been shown to prevent and eradicate P. *aeruginosa* biofilms (Christensen et al., Infect. Immun. 2013; Chua et al., Nat. Commun. 2014). Further, decreasing c-di-GMP levels using proteins that effectively bind and block c-di-GMP, such as the c-di-GMP-binding protein BdcA of *Escherichia coli* (*E. coli*), has been shown to lead to biofilm dispersal *in vitro* (Bjarnsholt et al., Nat. Rev. Drug Discov. 2013).

Therefore, one objective of the invention is to provide novel compounds that target the signaling molecule c-di-GMP. A further objective is to provide compounds having anti-biofilm activity and compounds for the treatment of biofilm associated diseases.

### SUMMARY OF THE INVENTION

To solve above-mentioned problems, the present invention provides in a first aspect a peptide, wherein said peptide comprises, preferably consists of, at least one, preferably exactly one, amino acid sequence (I), wherein said amino acid sequence (I) comprises, preferably consists of, the amino sequence
D1-X2-X3-X4-X5-X6-X7-X8-X9-Y10-X11-G12-X13-R14-R15-R16-X17-X18-D19 (SEQ ID NO: 59),
wherein D1 and D19 are independently of each other (i) a deletion, (ii) an amino acid having a negative net charge at pH 7, wherein preferably said amino acid having a negative net charge at pH 7 is a D-amino acid or L-amino acid, and further preferably an L-amino acid having a negative net charge at pH 7, or (iii) glycine or a glycine derivative selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine, ethyl acetamidoacetate, alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, wherein preferably said glycine derivative is a D-stereoisomer or L-stereoisomer, further preferably an L-stereoisomer;
X2, X3, X4, X5, X6, X7, X8, X9, X11, X13, X17 and X18 are independently of each other an amino acid, preferably a D-amino acid or L-amino acid, and further preferably an L-amino acid, or a deletion, wherein at most one of X2, X3, X4, X5, X6, X7, X8, X9, X11, X13, X17 or X18 is a deletion;
R14, R15 and R16 are independently of each other a basic amino acid, wherein preferably said basic amino acid is a D-amino acid or an L-amino acid, and further preferably a basic L-amino acid;
Y10 is an aromatic amino acid, wherein preferably said aromatic amino acid is D-amino acid or an L-amino acid, and further preferably an aromatic L-amino acid;
G12 is glycine or a glycine derivative, wherein the glycine derivative is selected from 2-aminobutyric acid, vinylglycine, benzylglycine, allylglycine, glycine C1-C3 alkylester, C1-C3 alkylglycine, C2-C3 alkenylglycine, formylglycine, N-formylglycine or ethyl acetamidoacetate, and wherein said glycine derivative is preferably a D-stereoisomer or L-stereoisomer, further preferably an L-stereoisomer;
and wherein said peptide has a length of at most 160 amino acids, preferably of at most 100 amino acids, further preferably of at most 50 amino acids and most preferably of at most 30 amino acids.

In a second aspect, the present invention provides a composition comprising or preferably consisting of:
(1) at least one peptide according to the invention; and
(2) at least one antimicrobial agent, at least one anti-infective, or a combination of at least one antimicrobial agent and at least one anti-infective.

In a third aspect, the present invention provides a pharmaceutical composition comprising of at least one peptide according to the invention. In a further aspect, the present invention provides a peptide according to the invention for use as a medicament in the treatment or prevention of a disease.

In another aspect, the present invention provides the use of the peptide according to the invention for preventing or reducing biofilm formation or for eradicating or reducing an existing biofilm.

In an additional aspect, the present invention provides a nucleic acid encoding the peptide of the invention, preferably the nucleic acid comprises or preferably consists of at least one nucleic acid sequence selected from SEQ ID NO: 54 to 58.

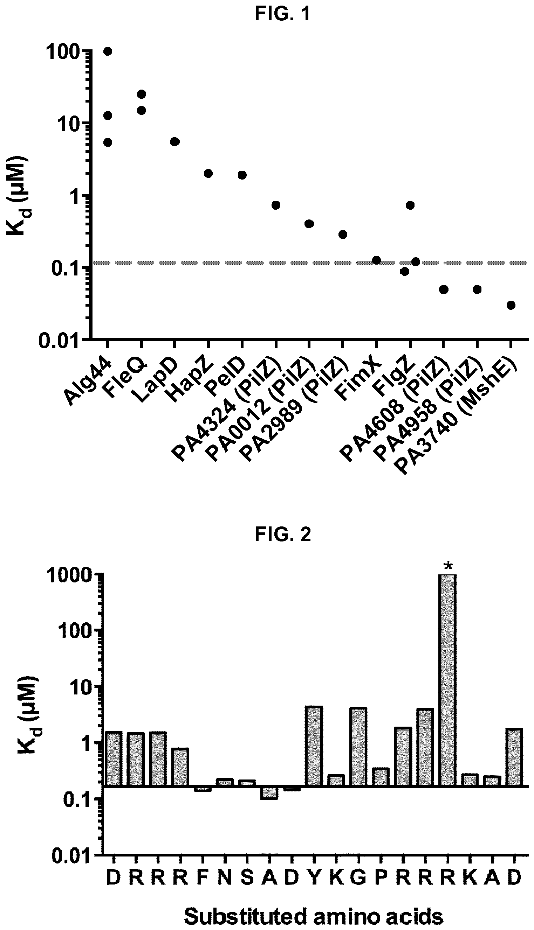

The invention provides peptides, which bind c-di-GMP with high affinity, higher than most c-di-GMP effectors known in the prior art. For example, dissociation constants (K_{d}) measured for c-di-GMP effectors of *Pseudomonas aeruginosa,* such as Alg44, FleQ, LapD and HapZ are much higher (meaning a lower affinity) than for peptides of the invention (cf. Figure 1). The inventors further demonstrated that the peptides of the invention which are derived from a small modified protein fragment specifically selected from a protein sequence of *Caulobacter crescentus*, bind specifically to c-di-GMP and are highly stable, especially against bacterial proteases.

Moreover, the invention provides a novel approach to address bacterial biofilms using c-di-GMP sequestrating peptides. Decreasing cellular c-di-GMP promotes planktonic bacteria. The peptides of the present invention bind c-di-GMP with high affinities and can thus compete with and displace other bacterial proteins that bind c-di-GMP. It is believed that c-di-GMP signaling network is disrupted and bacteria switch into planktonic lifestyle if the peptide of the invention sequesters c-di-GMP. Without being bound to this theory, the peptides of the present invention disrupt c-di-GMP signaling by binding and sequestrating c-di-GMP, which results in disruption of biofilms and/or inhibition or prevention of biofilm formation.

Disrupting or weakening a biofilm or avoiding or decreasing biofilm formation via the peptide of the invention will lead to an improved efficacy of simultaneously or subsequently applied biocides, especially antibiotics. This is a highly synergistic effect, since liberated planktonic bacteria are much more (up to 1.000-fold) susceptible to biocides and antibiotics. By a simultaneous or subsequent antibiotic treatment, planktonic bacterial cells can be eliminated, and spreading of the infection to other parts of the body can be avoided. Moreover, liberated bacteria are no longer resistant to host's immune defense mechanisms and thus, can be cleared by the host's own immune system.

Remarkably, the peptides of the invention do not affect cell growth and are not bactericidal, i.e. do not kill bacterial cells, but promote instead a change from biofilm to planktonic lifestyle. This property is responsible for a low risk of developing resistances, which is a devastating problem worldwide in healthcare. Therefore, without being bound to this theory, it is believed that the peptides and compositions of the invention lead to little or no bacterial resistance against said peptides.

Since the mode of action of the peptides of the invention does not involve killing cells, said peptides show no cytotoxic effects at low and medium concentrations, and only minor cytotoxic effects at high concentrations. It is thus believed that the peptides and compositions of the invention are safe and very well tolerated in medical and non-medical applications.

### DESCRIPTION OF FIGURES

In the following, the content of the figures comprised in this specification is described. In this context, it is also referred to the description of the invention above and below.
- FIG. 1:: Comparison of binding affinities of known *P. aeruginosa* c-di-GMP effectors with the peptide of SEQ ID NO: 4 (shown as dashed line with K_{d} = 0.12 µM). Note that more than one value known from different studies is reported for Alg44, FleQ and FlgZ.
- FIG. 2:: Influence of amino acid substitution on affinity to bind c-di-GMP: Comparison of binding affinities of the peptide of SEQ ID NO: 12 with peptides of SEQ ID NO: 13 to 31, which are based on SEQ ID NO: 12, but include a single amino acid substitution. Affinity of peptide SEQ ID NO: 12 is shown as solid line with K_{d} = 0.17 µM. SEQ ID NO: 12 is shown below the x-axis to indicate the position of the substituted amino acid. Asterisk indicates no binding detected for the peptide of SEQ ID NO: 28.
- FIG. 3:: Influence of amino acid deletion on affinity to bind c-di-GMP: Comparison of binding affinities of the peptide of SEQ ID NO: 12 reported peptides of SEQ ID NO: 32 to 38 having a single amino acid deletion compared to SEQ ID NO: 12. Affinity of peptide SEQ ID NO: 12 is shown as solid line with K_{d} = 0.17 µM. SEQ ID NO: 12 is shown below the x-axis to indicate the position of the deleted amino acid.
- FIG. 4:: Specific binding of FITC-labeled peptide of SEQ ID NO: 43 to c-di-GMP (solid line).
- FIG. 5:: Comparison of stability of peptides of SEQ ID NO: 12 and SEQ ID NO: 28 having different affinities in the presence or absence of c-di-GMP.
- FIG. 6:: Uptake of FITC-labelled peptides of SEQ ID NO: 43 to 51 in *P. aeruginosa* PAO1 cells as determined by flow cytometry.
- FIG. 7:: Localization of FITC-labeled peptides of SEQ ID NO: 48 and 51 in *P. aeruginosa* PAO1 cells as determined by fluorescence microscopy (scale bar indicates 5 µm).
- FIG. 8:: FITC-labelled peptides of SEQ ID NO: 43, 48 and 51 detected in cytoplasmic and membrane fractions of *P*. *aeruginosa* PAO1 cells.
- FIG. 9:: Effects of peptides of SEQ ID NO: 12, 28, 43 and 51 on biofilm formation.
- FIG. 10:: Bacterial growth curves: Peptides of SEQ ID NO: 12, 28, 43 and 51 do not show an effect on bacterial growth.
- FIG. 11:: Cytotoxic effects of peptides of SEQ ID NO: 12, 43, 48 and 51 on HeLa cells determined by resazurin reduction assay normalized to a Triton X control.
- FIG. 12:: Cytotoxic effects of peptides of SEQ ID NO: 12, 43, 48 and 51 on HeLa cells determined by LDH (lactate dehydrogenase) release assay normalized to a Triton X control.

### DETAILED DESCRIPTION OF THE INVENTION

Unless defined otherwise, all technical and scientific terms used herein have the same meaning as commonly understood by one of ordinary skill in the art to which this invention belongs.

Throughout this specification and the claims which follow, unless the context requires otherwise, the term "comprise" and its variations such as "comprises" and "comprising" etc., are to be understood as a non-exhaustive wording and imply the inclusion of a stated feature or element but not the exclusion of any other feature or element. The term "comprise" and its variations cover the term "consisting of". As used in this specification and the appended claims, the singular forms "a", "an", and "the" include plural referents, unless the content clearly dictates otherwise. The terms "reduce", "inhibit" or "decrease", as used herein, include a just detectable reduction but also a reduction down to zero (reduction by 100%). The term "is effective" refers herein to any kind and any degree of effect and also an effect that is only believed to occur according to the common general knowledge.

A peptide, as defined herein, is any peptide-bond-linked chain of amino acids, regardless of length, secondary and tertiary structure, number of subunits or post-translational modification. Thus, the term "peptide" is to be understood as covering the terms "polypeptide", "protein", "amino acid chain" and "polypeptide chain". Amino acids included in the peptide of the invention are proteinogenic, non-proteinogenic and synthetic amino acids. Peptides in accordance with the invention can be an open linear peptide chain or cyclic peptides; alternatively or additionally, peptides of the invention may include at least one chemical modification, such as lipidation, glycosylation and phosphorylation. Peptides, as understood herein, especially peptides of the invention can be produced by chemical synthesis, RNA translation and/or recombinant processes. Preferably the peptide of the invention is an isolated peptide.

The term "amino acid", as used herein, refers to organic compounds containing the functional groups amine (-NH2) and carboxylic acid (-COOH) and its zwitterions, typically and preferably, along with a side chain specific to each amino acid. The term "amino acid" typically and preferably includes amino acids that occur naturally, such as proteinogenic amino acids (produced by RNA-translation), non-proteinogenic amino acids (produced by other metabolic mechanisms, e.g. posttranslational modification), standard or canonical amino acids (that are directly encoded by the codons of the genetic code) and non-standard or non-canonical amino acids (not directly encoded by the genetic code). Naturally occurring amino acids include non-eukaryotic and eukaryotic amino acids. The term "amino acid", as used herein, also includes unnatural amino acids that are chemically synthesized. Moreover, the term covers alpha- (α-), beta- (β-), gamma- (γ-) and delta- (δ-) etc. amino acids as well as mixtures thereof in any ratio, and, if applicable, any isomeric form of an amino acid, i.e. its D- and L-stereoisomers (alternatively addressed by the (R) and (*S*) nomenclature) as well as mixtures thereof in any ratio, preferably in a racemic ratio of 1:1. Amino acids in this invention are typically and preferably in L-configuration. The term "D-stereoisomer", "L-stereoisomer", "D-amino acid" or "L-amino acid" refers to the chiral alpha carbon of the amino acids. Basic amino acids in accordance with the invention have an additional basic group (e.g. another amine). Aromatic amino acids in accordance with the invention include an aromatic group. Amino acid can include modifications and/or attached compounds and residues, for example residues used for peptide synthesis, such as Boc, Fmoc or both.

In the context of this invention, amino acid sequences that differ from each other in at least one amino acid are compared by means of determining the percentage of sequence identity. The difference between the sequences is selected from insertion, deletion and amino acid exchange. The term "sequence identity", as used herein, is determined based on an alignment of both sequences. Algorithms for the determination of sequence identity are available to the artisan. Preferably, the sequence identity of two amino acid sequences is determined using publicly available computer homology programs such as the "BLAST" program (http://blast.ncbi.nlm.nih.gov/Blast.cgi) or the "CLUSTALW" (http://www.genome.jp/tools/clustalw/), and hereby preferably by the "BLAST" program provided on the NCBI homepage at http://blast.ncbi.nlm.nih.gov/Blast.cgi, using the default settings provided therein. Typical and preferred standard settings are: expect threshold: 10; word size: 3; max matches in a query range: 0; matrix: BLOSUM62; gap costs: existence 11, extension 1; compositional adjustments: conditional compositional score matrix adjustment.

The term "amino acid exchange", as used herein, refers to the exchange of a given amino acid in an amino acid sequence by a deletion or any other amino acid having a different residues or chemical structure, preferably by another proteinogenic amino acid.

The term "N-terminus", as used herein, refers to an end of a peptide having a free (-NH₂) or modified amino or amine group. Preferred N-terminal modifications are those that protect the N-terminus from proteolytic degradation. N-terminal modifications in accordance with the invention include but are not limited to acetylation, attachment of at least one, preferably exactly one amino acid, preferably of at least one, preferably exactly one D-amino acids, or attachment of at least one, preferably exactly one compound, such as a peptide, preferably a cell penetrating peptide, but also a nucleic acid, methyl, myristoyl, prenyl group, palmitoyl, ubiquitin, 7-methoxycoumarin acetic acid (Mca), dansyl, formyl, 4-diniphenyl, pyroglutamyl, urea, carbamate, sulphonamide, alkylamine, fatty acids, such as palmitic acid, radioligand, quencher, fluorescein or another dye or label, such as biotin.

The term "C-terminus", as used herein, refers to an end of a peptide having a free (-COOH) or modified carboxyl group. Preferred C-terminal modifications are those that protect the C-terminus from proteolytic degradation. C-terminal modifications in accordance with the invention include but are not limited to amidation or attachment of at least one, preferably exactly one amino acid, preferably of at least one, preferably exactly one D-amino acids, or attachment of at least one, preferably exactly one compound, such as a peptide, preferably a cell penetrating peptide, but also a nucleic acid, polyethylene glycol (PEGylation), thiol, ester, aldehyde, pNA (para-nianilide), Amc (7-amino-4-methylcoumarinyl), hydrazide, hydroxamic acid, chloromethyl ketone, biotin, radioligand, quencher, Abz or other dyes and labels. Herein and by general convention, peptide sequences are written from N-terminal on the left to C-terminal on the right (according to the direction of translation).

Each alkyl moiety either alone or as part of a larger group, such as alkylester is a straight or branched chain and is preferably C1-C4 alkyl, more preferably C1-C3 alkyl. Examples include methyl, ethyl, n-propyl, prop-2-yl. The integer after the capital letter "C", e.g. in the term "C1-C3", refers to the number of carbons included in the carbon chain, i.e. the term "C1-C3" refers to a carbon chain including 1 to 3 carbons.

Each alkenyl moiety either alone or as part of a larger group, such as alkenylglycine is a straight or branched chain and is preferably C2-C4 alkenyl, more preferably C2-C3 alkenyl. Each moiety can be of either the (*E*)- or (*Z*)-configuration. Examples include vinyl and allyl. A compound of the present invention comprising an alkenyl moiety thus may include, if applicable, either said compound with said alkenyl moiety in its (*E*)-configuration, said compound with said alkenyl moiety in its (*Z*)-configuration or mixtures thereof in any ratio. The integer after the capital letter "C", e.g. in the term "C1-C3", refers to the number of carbons included in the carbon chain, i.e. the term "C1-C3" refers to a carbon chain including 1 to 3 carbons.

The term "deletion" refers herein to a position in an amino acid sequence or nucleic acid sequence that is not occupied by an amino acid and nucleic acid, respectively.

The term "cell penetrating peptide", as used herein, refers to any peptide that facilitates transfer across membranes or internalization of compounds into cells or cellular compartments. Penetration may occur by different mechanisms, such as penetration endocytosis mediated translocation, translocation through the formation of a transitory structure or directly by an energy-independent process. Preferably, cell penetrating peptides have an amino acid composition that may contain a high abundance of positively charged amino acids, such as lysine or arginine (polycationic cell penetrating peptides). In another embodiment, the cell penetrating peptide includes an alternating pattern of polar, charged amino acids and non-polar, hydrophobic amino acids (amphipathic cell penetrating peptide) or apolar residues with low net charge or hydrophobic amino acid groups (hydrophobic cell penetrating peptides). Preferred examples of cell penetrating peptides in accordance with the invention are trans-activating transcriptional activator peptides (TAT), preferably TAT from human immunodeficiency virus 1 (TAT-HIV1), pVEC, penetratin, pep-1, MPG and polyarginines.

The term "linker", as used herein, is at least one molecule or sequence that is capable of connecting or associating at least two molecules, preferably two peptides or a peptide and a non-peptide. Connection or association can occur by all possible ways, preferably chemical interaction, such as covalent or non-covalent interaction, or physical interaction. Preferably, a linker acts by at least one covalent bond. Preferred linkers in accordance with the invention are linkers containing at least one amino acid, more preferably an amino acid sequence including a number of N amino acids, wherein N is an integer of preferably, 1-100, 1-50, 1-20, 1-10, 1-5, 1, 2, 3, 4, 5 or more. A linker couples preferably multiple domains within a single polypeptide. A linker is associated with the peptide preferably by way of at least one peptide bond. Preferred examples of linkers in accordance with the invention are amino acids, such as beta-alanine, polyethylene glycol of different suitable lengths, maleimide, NHS-esters. Other linkers in accordance with the invention are O1Pen, aminohexanoic acid, O2Oc, O1Pen-O1Pen, 12-amino-dodecanoic acid, Ttds, molecules comprising a sulfhydryl group or a cysteine residue, molecules comprising alkyl, preferably C1-C6 alkyl, cycloalkyl, such as cyclopentyl or cyclohexyl, cycloalkenyl, aryl or heteroaryl moiety. Alkyl, cycloalkyl, aryl, alkenyl or a combination thereof can also be combined with at least one additional amino acid to form a linker. The term "linker", as used herein, includes also a combination of two or more linkers.

The parameter "dissociation constant" (K_{d}), as used herein, refers to an equilibrium constant that measures the propensity of a molecule, herein the peptide of the invention, to dissociate from the binding partner, herein c-di-GMP. Thus, the dissociation constant is a quantitative measure of the binding affinity. K_{d} and affinity are inversely related, i.e. the lower the K_{d} value, which means the lower the concentration, the higher the affinity of the molecule to its binding partner. Preferred K_{d} values are in the µM range and more preferably in the nM range.

The parameter "MBIC₅₀", as used herein, is defined as the peptide concentration leading to 50% biofilm reduction in a standard biofilm assay. Preferably, the medium used for the assay is Mueller-Hinton broth (MHB), since most bacteria can be grown in this medium, and it allows a reliable comparison of MBIC values. MBIC₅₀ values in accordance with the invention are obtained by growing *P. aeruginosa* at 37°C in presence of different concentrations of peptides, without agitation, for 24 h, followed by staining the adherent biofilm, dissolving the biofilm and measuring the OD600.

The term "antimicrobial agent", as used herein, refers to an agent that kills microorganisms or inhibits or disrupts growth and/or metabolism of microorganisms. The general definition of the term "antimicrobial agent", as used herein, thus covers antibiotics, antimycotics, antiviral agents and antiprotozoal agent.

The term "disinfectant", as used herein, refers to an antimicrobial agent that is applied to non-living objects to destroy or inhibit microorganisms. Disinfectants in accordance with the invention may work, e.g., by destroying the cell wall or interfering with the metabolism of microbes and include but are not limited to alcohols, aldehydes, oxidizing agents, phenolics, quaternary ammonium compounds, silver and silver alloys, copper alloys, thymol compounds, polyaminopropyl biguanide, sodium bicarbonate (NaHCO₃), hydrochloric acid and lactic acid.

The term "antiseptic", as used herein, refers to an antimicrobial agent that is applied to living objects or tissue to destroy or inhibit microorganisms. Antiseptics in accordance with the invention include but are not limited to alcohols, most commonly ethanol (60-90%), 1-propanol (60-70%) and 2-propanol/isopropanol (70-80%) or mixtures of these alcohols; quaternary ammonium compounds, such as benzalkonium chloride, cetyltrimethylammonium bromide, cetylpyridinium chloride, and benzethonium chloride; chlorhexidine gluconate; octenidine; boric acid; hydrogen peroxide; iodine; manuka honey; octenidine dihydrochloride; phenol; polyhexanide (polyhexamethylene biguanide); sodium hypochlorite, calcium hypochlorite; sodium bicarbonate; and Balsam of Peru.

The term "anti-infective", as used herein, refers to an agent that is capable of acting against an infection, for example, by inhibiting the spread of an infective microorganism, killing the infective microorganism, stimulating the immune system via an immunostimulating agent or therapeutic or prophylactic vaccine. The herein used general definition of the term "anti-infective" thus also covers antibiotics, antimycotics, antiviral agents and antiprotozoal agents.

The term "immunostimulating agent" (synonymous to "immunostimulatory" or "immunostimulant"), as used herein, refers to a pharmaceutically active agent (including drugs, nutrients and others) that is capable of inducing and/or enhancing an immune response. Examples of immunostimulating agents in accordance with the invention include but are not limited to interferon, such as interferon beta, e.g., interferon beta-1a, interferon beta-1b, peginterferon beta-1a; interferon alpha, e.g., interferon alpha-2a, interferon alpha-2b, peginterferon alpha-2a, peginterferon alpha-2b; glatiramer acetate; interferon gamma, such as interferon gamma-1b; interleukin, such as aldesleukin, e.g. aldesleukin G-CSF; bacterial immunostimulating agents, e.g. of *Enterococcus,* such as *Enterococcus faecalis*, *Escherichia coli* and combinations thereof; plant derived and complementary immunostimulating agents, e.g. of *Echinacea purpurea, Eleutherococcus*, *Panax ginseng*; CXCR-4 antagonist, such as plerixafor; histamine dihydrochloride; tasonermin; bacillus Calmette-Guérin (BCG) vaccines; mifamurtide; colony stimulating factors and other cytokines, such as filgrastim, lenograstim, pegfilgrastim, lipefilgrastim, granulocyte-macrophage colony-stimulating factor (GM-CSF); toll-like receptor activating substances and substances inducing cytokine secretion; stimulatory nucleic acids, e.g. containing at least one CpG motif, peptidoglycans, lipopolysaccharides, lipoteichoic acids, imidazoquinoline compounds, flagellins, lipoproteins, and immunostimulatory organic substances, such as taxol.

The term "antibiotic", as used herein, refers to a type of antimicrobial agent or anti-infective that kills bacteria (bactericidal antibiotics) or inhibits growth and/or metabolism of bacteria (bacteriostatic antibiotics). Antibiotics in accordance with the invention can also possess antiprotozoal activity. Antibiotics can be classified, e.g., based on their mechanism of action. Antibiotics in accordance with the invention can target the bacterial cell wall (e.g. penicillins, cephalosporins) or cell membrane (e.g. polymyxins) or can interfere with essential bacterial enzymes (e.g. rifamycins, lipiarmycins, quinolones, and sulfonamides) or protein synthesis (e.g. macrolides, lincosamides and tetracyclines).

The term "antiviral agent", as used herein, refers to a type of antimicrobial agent or anti-infective that inactivates viruses or viral articles and/or inhibits or blocks viral development, replication and/or spreading. An anti-viral strategy is impairing the ability of a virus to infiltrate a target cell. Other strategies relate to impairing the processes that synthesize virus components, such as viral nucleic acids, polypeptides and particles, after a virus has entered a target cell, or to preventing or inhibiting assembly or release of viral particles from the host cell. Antiviral agents in accordance with the invention include but are not limited to aciclovir, foscarnet, imunovir, imiquimod, indinavir, inosine, integrase inhibitor, interferon type I, II, III, ribavirin, trizivir, adamantane derivatives amantadine and rimantadine, and neuraminidase inhibitors oseltamivir and zanamivir.

The term "antimycotic", as used herein, is synonymous to an antifungal agent and refers to a certain type of antimicrobial agent or anti-infective that kills fungi (fungicide) or inhibits growth and/or metabolism of fungi (fungistatic). Antimycotics in accordance with the invention include but are not limited to polyene, imidazole, triazole, thiazole, allylamines, echinocandins, cyclosporin A and diclofenac agents.

The term "antiprotozoal agent", as used herein, refers to a certain type of antimicrobial agent or anti-infective that kills protozoans or inhibits growth and/or metabolism of protozoans. Antiprotozoal agents in accordance with the invention include but are not limited to eflornithine, furazolidone, melarsoprol, menidazole, nifursemizone, nitazoxanide, ornidazole, paromomycin sulfate, pentamidine, pyrimethamine and tinidazole.

The term "pharmaceutically active agent", as used herein, refers to any agent that causes or assists in causing a physiological change in an organism, such as a pharmacological, immunological, biological or metabolic effect. A pharmaceutical agent can be, e.g., an anti-infective, antiseptic and the like.

The term "vaccine", as used herein, refers to a pharmaceutically active agent that provides active or passive immunity against a certain disease. A vaccine in accordance with the invention includes also a nucleic acid vaccine, which expresses a therapeutic peptide or an antigen derived from a pathogen.

The term "adjuvant", as used herein, refers to a pharmaceutically active agent that modifies, preferably enhances the effect of other agents, such as a vaccine or pharmaceutically active agent. Adjuvants are preferably added to a vaccine in order to modify, preferably enhance the response of the immune system to the vaccine, e.g. by stimulating the immune system non-specifically. Further, by means of the adjuvant, a depot can be generated in the host which when combined with the peptide, vaccine or pharmaceutically active agent in the pharmaceutical composition of the invention provides a sustained efficiency. Adjuvants in accordance with the invention are complete and incomplete Freund's adjuvant, aluminum containing adjuvant, preferably alum or aluminum hydroxide, modified muramyldipeptide, mineral gels, surface active substances, such as lysolecithin, pluronic polyols, polyanions, peptides, oil emulsions, keyhole limpet hemocyanins, diniphenol, and human adjuvants such as BCG (bacillus *Calmette Guerin*) and *Corynebacterium parvum*. The adjuvant in accordance with the invention can also comprise mixtures of these adjuvants.

A "biofilm" is herein defined as population of bacterial and/or fungal cells adherent to each other, to surfaces, interfaces including biotic and abiotic surfaces and interfaces, or associated to tissue or mucus. This definition also includes small and great microbial aggregates and floccules and also adherent populations within the pore spaces of porous media. The term "anti-biofilm activity" or only "anti-biofilm", as used herein, refers to prevention or reduction of biofilm formation or eradication or reduction of an existing biofilm, at least partial liberation of bacteria or at least partially dissolving a biofilm matrix. The term "biofilm bacteria", as used herein, refers to non-planktonic bacteria present in a biofilm.

The term "coating", as used herein, refers to an absorbed or adsorbed lining. Coatings in accordance with the invention include layers applied to a surface or interface of a product having a smooth, solid or straight surface, but also substances applied to textile, wadding, matrix, net, porous material that absorb the coating. Coatings in accordance with the invention can be applied, e.g., as liquid, solid, paste, gaseous phase, dust or the like, and may optionally change their aggregate state after application.

The term "medical device", as used herein, refers to products whose main purpose is primarily achieved by physical means. Medical devices do not act via pharmacological, biological, immunological or metabolic mechanisms, and thus the term "medical device" excludes medical products, such as pharmaceuticals or drugs. Medical devices in accordance with the invention include but are not limited to implants, products for injection, infusion, transfusion and dialysis, medical instruments intended for use in humans, software, catheters, artificial cardiac and neural pacemakers, dental devices, bandaging material, mattresses, pillows, disposal gloves and textiles, corrective lenses, x-ray machines, condoms, contraceptive devices, laboratory instruments, instruments for use by physicians as well as *in vitro* diagnostic agents.

The term "preservative", as used herein, is at least one compound that prevents or inhibits decomposition caused by microbial growth or chemical changes. It is preferably added or applied to products, such as food, beverages, pharmaceutical drugs, paints, biological samples, cosmetics and wood. Preservatives in accordance with the invention are preferably antimicrobial preservatives that prevent degradation by bacteria, including but not limited to benzoic acid, sodium benzoate, hydroxybenzoates, lactic acid, nitrite, nitrate, propionic acid, sodium propionate, sulfur dioxide, sulfites, sorbic acid and sodium sorbate. The term "preservative", as understood herein, also covers antioxidants, including but not limited to ascorbic acid, butylated hydroxytoluene, butylated hydroxyanisole, gallic acid, sodium gallate, oxygen scavenger, sulfur dioxide, sulfites and tocopherols; and non-synthetic and natural compounds, such as citric and ascorbic acids, rosemary extract, hops, salt, sugar, vinegar, alcohol, diatomaceous earth and castor oil.

A disease is defined herein as an abnormal condition that affects part or all of an organ, organ system, structure, tissue or organism, such as a person, animal, or plant. A disease includes also a disorder or an abnormal condition of an, cell or an abnormal function.

The term "cosmetic", as used herein, includes any non-medical cosmetic and excludes any medical product.

The term "nucleic acid", as used herein, refers to an N-meric, oligomeric or polymeric macromolecule of any length made from nucleotide monomers. The term "nucleic acid" is herein to be understood as covering the terms "polynucleotide", "(poly)nucleic (acid) chain", "polynucleotide chain", "oligonucleotide", "oligonucleic (acid) chain" and "oligonucleotide chain". Nucleotide monomers in accordance with the invention are composed of a nucleobase, a five-carbon sugar (such as but not limited to ribose or 2'-deoxyribose), and one to three phosphate groups. Typically and preferably, a nucleic acid is formed through phosphodiester bonds between the individual nucleotide monomers. In the context of the present invention, the term "nucleic acid" includes but is not limited to ribonucleic acid (RNA), deoxyribonucleic acid (DNA), mixtures thereof such as e.g. RNA-DNA hybrids, and artificial nucleic acid analogues, such as peptide nucleic acid, morpholino and locked nucleic acid, glycol nucleic acid, and threose nucleic acid. Nucleic acids in accordance with the invention can be synthesized chemically, e.g. in accordance with the phosphotriester method, or can be synthesized by transcription.

The term "vector", as used herein, refers to a protein or a polynucleotide or a mixture thereof which is capable of being introduced or of introducing the proteins and/or nucleic acid comprised therein into a cell. In the context of the present invention, it is preferred that the genes of interest encoded by the introduced polynucleotide are expressed within the cell upon introduction of the vector or vectors. Examples of suitable vectors include but are not limited to plasmids, cosmids, phages, viruses and artificial chromosomes.

In a first aspect, the invention provides for a peptide, wherein said peptide comprises, preferably consists of, at least one, preferably exactly one, amino acid sequence (I), wherein said amino acid sequence (I) comprises, preferably consists of, the amino sequence:
D1-X2-X3-X4-X5-X6-X7-X8-X9-Y10-X11-G12-X13-R14-R15-R16-X17-X18-D19 (SEQ ID NO: 59)
   wherein D1 and D19 are independently of each other (i) a deletion, (ii) an amino acid having a negative net charge at pH 7, wherein preferably said amino acid having a negative net charge at pH 7 is a D-amino acid or L-amino acid, and further preferably an L-amino acid having a negative net charge at pH 7, or (iii) glycine or a glycine derivative selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine, ethyl acetamidoacetate, alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, wherein preferably said glycine derivative is a D-stereoisomer or L-stereoisomer, further preferably an L-stereoisomer;
X2, X3, X4, X5, X6, X7, X8, X9, X11, X13, X17 and X18 are independently of each other an amino acid, preferably a D-amino acid or L-amino acid, and further preferably an L-amino acid, or a deletion, wherein at most one of X2, X3, X4, X5, X6, X7, X8, X9, X11, X13, X17 or X18 is a deletion;
R14, R15 and R16 are independently of each other a basic amino acid, wherein preferably said basic amino acid is a D-amino acid or an L-amino acid, and further preferably a basic L-amino acid;
Y10 is an aromatic amino acid, wherein preferably said aromatic amino acid is a D-amino acid or an L-amino acid, and further preferably an aromatic L-amino acid;
G12 is glycine or a glycine derivative, wherein the glycine derivative is selected from 2-aminobutyric acid, vinylglycine, benzylglycine, allylglycine, glycine C1-C3 alkylester, C1-C3 alkylglycine, C2-C3 alkenylglycine, formylglycine, N-formylglycine or ethyl acetamidoacetate, and wherein said glycine derivative is preferably a D-stereoisomer or L-stereoisomer, further preferably an L-stereoisomer;
and wherein said peptide has a length of at most 160 amino acids, preferably of at most 100 amino acids, further preferably of at most 50 amino acids and most preferably of at most 30 amino acids.

In another embodiment, the peptide of the invention has a length of at most 160 amino acids, preferably of at most 150 amino acids, more preferably of at most 140 amino acids, again more preferably of at most 130 amino acids, again more preferably of at most 120 amino acids, and again more preferably of at most 110 amino acids. In another embodiment, the peptide of the invention has a length of at most 100 amino acids, preferably of at most 80 amino acids, more preferably of at most 70 amino acids, again more preferably of at most 50 amino acids. In a preferred embodiment, the peptide of the invention has a length of 100 to 17 amino acids. In a another preferred embodiment, the peptide of the invention has a length of at most 100 amino acids, preferably of at most 90 amino acids, more preferably of at most 80 amino acids, again more preferably of at most 70 amino acids, again more preferably of at most 60 amino acids, and again more preferably of at most 50 amino acids. In an even more preferred embodiment, the peptide of the invention has a length of 50 to 18 amino acids, more preferably 50 to 17 amino acids. In another preferred embodiment, the peptide of the invention has a length of at most 40 amino acids, preferably of at most 30 amino acids, more preferably of at most 20 amino acids, again more preferably of at most 19 amino acids..

In one embodiment, D1 and D19 of the peptide of the invention are independently of each other a deletion.

In another preferred embodiment, D1 and D19 of the peptide of the invention are independently of each other an amino acid having a negative net charge at pH 7, preferably a D-amino acid or L-amino acid having a negative net charge at pH 7, and further preferably an L-amino acid having a negative net charge at pH 7. Preferably, D1 and D19 are independently of each other a D- or L-amino acid, preferably an L-amino acid, wherein the D- or L-amino acid has a side chain comprising, or alternatively consisting of at least one carboxyl group. More preferably, D1 and D19 are independently of each other an aspartate derivative, wherein the aspartate derivative is selected from 2-amino-6-methylene-pimelic acid, 4-fluoro-glutamic acid, carboxymethylated cysteine, 2-amino-6-oxopimelic acid, 3,3-dimethyl aspartic acid, 4-methylglutamate, 3-methyl-glutamic acid, 2-aminoadipic acid, 5-o-methyl-glutamic acid, 3-methyl-aspartic acid, glutamate, 2-amino-propanedioic acid, 4-hydroxy-glutamic-acid, beta-hydroxyaspartic acid, 6-carboxylysine or aspartate, wherein preferably the aspartate derivative is a D-stereoisomer or L-stereoisomer, more preferably an L-stereoisomer. In an even more preferred embodiment, D1 and D19 are independently of each other an aspartate derivative, wherein the aspartate derivative is selected from aspartate, 3-methyl-aspartic acid, beta-hydroxyaspartic acid or 3,3-dimethyl aspartic acid, wherein the aspartate derivative is preferably a D-stereoisomer or L-stereoisomer, more preferably an L-stereoisomer. In an again more preferred embodiment, D1 and D19 are independently of each other D-aspartate or L-aspartate, more preferably L-aspartate. In an again more preferred embodiment, D1 and D19 are L-aspartate.

In another embodiment, D1 and D19 of the peptide of the invention are independently of each other glycine or a glycine derivative, wherein the glycine derivative is selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine, ethyl acetamidoacetate, alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, wherein the glycine derivative is preferably a D-stereoisomer or L-stereoisomer, more preferably an L-stereoisomer. In a preferred embodiment, D1 and D19 are independently of each other glycine or a glycine derivative, wherein the glycine derivative is selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine, ethyl acetamidoacetate or alanine, wherein the glycine derivative is preferably a D-stereoisomer or L-stereoisomer, more preferably an L-stereoisomer. In an even more preferred embodiment, D 1 and D19 are independently of each other L-aspartate, L-alanine or glycine. In a more preferred embodiment, D1 and D19 are independently of each other L-aspartate or glycine. In a further more preferred embodiment, D1 and D19 are independently of each other glycine. In a most preferred embodiment, D1 and D19 are glycine.

R14, R15 and R16 of the peptide of the invention are independently of each other a basic amino acid, wherein preferably said basic amino acid is a D-amino acid or an L-amino acid, preferably a basic L-amino acid. In a preferred embodiment, R14, R15 and R16 are independently of each other a basic D- or a basic L-amino acid, preferably a basic L-amino acid, wherein the D- or L-amino acid has a side chain comprising at least one amine. In a more preferred embodiment, said R14, R15 and R16 are independently of each other a lysine derivative, wherein said lysine derivative is selected from diaminobutyric acid, 2,3-diaminopropanoic acid, 2,8-diaminooctanoic acid, lysine, ornithine or thialysine, and wherein preferably said lysine derivative are a D-stereoisomer or L-stereoisomer, further preferably an L-stereoisomer. In an even more preferred embodiment, R14, R15 and R16 are independently of each other D-lysine or L-lysine, preferably L-lysine. In an even more preferred embodiment, R14, R15 and R16 are D-lysine or L-lysine, preferably L-lysine.

In an again more preferred embodiment, R14, R15 and R16 of the peptide according to the invention are independently of each other a basic amino acid, wherein preferably said basic amino acid is a D-amino acid or L-amino acid, preferably an L-amino acid, wherein the basic D- or L-amino acid has a side chain comprising, or alternatively consisting of, at least one guanidine group. Again more preferably, R14, R15 and R16 are independently of each other an arginine derivative, wherein the arginine derivative is selected from arginine, citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline or 4-guanidinobutryric acid, and wherein preferably said arginine derivative is a D-stereoisomer or L-stereoisomer, further preferably an L-stereoisomer. In an even more preferred embodiment, said arginine derivative is selected from a D-stereoisomer or L-stereoisomer of arginine, 2-amino-4-guanidinobutryric acid, 2-amino-3-guanidinopropionic acid, 5-methyl-arginine, c-gamma-hydroxy arginine or homoarginine, wherein the L-stereoisomer is preferred.

In another preferred embodiment, R14, R15 and R16 are independently of each other a D-amino acid or L-amino acid, preferably an L-amino acid, wherein said D- or L-amino acid is selected from a D-stereoisomer or L-stereoisomer of arginine, citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline, 4-guanidinobutryric acid, lysine, diaminobutyric acid, 2,3-diaminopropanoic acid, 2,8-diaminooctanoic acid, ornithine or thialysine. In a more preferred embodiment, R14, R15 and R16 are independently of each other D-lysine, L-lysine, D-arginine or L-arginine, again more preferably D-arginine or L-arginine, and again more preferably L-arginine. In a most preferred embodiment, R14, R15 and R16 are D-lysine, L-lysine, D-arginine or L-arginine, again more preferably D-arginine or L-arginine, and again more preferably L-arginine.

In one embodiment, X2, X3 and X4 of the peptide according to the invention are an alanine derivative, wherein said alanine derivative is independently of each other selected from alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, and wherein preferably said alanine derivative is a D-stereoisomer or L-stereoisomer, further preferably an L-stereoisomer. Preferably, the alanine derivative is D-alanine or L-alanine, preferably L-alanine. In a more preferred embodiment, X2, X3 and X4 are independently of each other a D-amino acid or L-amino acid, preferably an L-amino acid, which has a positive net charge at pH 7. In an even more preferred embodiment, X2, X3 and X4 are independently of each other (i) an arginine derivative, wherein the arginine derivative is selected from arginine, citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline or 4-guanidinobutryric acid, and wherein preferably said arginine derivative is a D-stereoisomer or L-stereoisomer, further preferably an L-stereoisomer; or (ii) a lysine derivative, wherein the lysine derivative is selected from lysine, diaminobutyric acid, 2,3-diaminopropanoic acid, 2,8-diaminooctanoic acid, ornithine or thialysine, and wherein preferably said lysine derivative is a D-stereoisomer or L-stereoisomer, further preferably an L-stereoisomer. Again more preferably, X2, X3 and X4 are independently of each other a D-amino acid or L-amino acid, preferably an L-amino acid, wherein the D- or L-amino acid is selected from a D-stereoisomer or L-stereoisomer of ornithine, diaminobutyric acid, 2,3-diaminopropanoic acid, 2,8-diaminooctanoic acid, lysine, thialysine, 5-methyl-arginine, c-gamma-hydroxy arginine, homoarginine, 2-amino-3-guanidinopropionic acid, 2-amino-4-guanidinobutryric acid or arginine. In an even more preferred embodiment, X2, X3 and X4 are independently of each other selected from a D-stereoisomer or L-stereoisomer of 5-methyl-arginine, c-gamma-hydroxy arginine, homoarginine, 2-amino-3-guanidinopropionic acid, 2-amino-4-guanidinobutryric acid or arginine, wherein the L-stereoisomer is preferred. In an again more preferred embodiment, X2, X3 and X4 are independently of each other D-lysine, L-lysine, D-arginine or L-arginine, preferably L-lysine or L-arginine. In an again more preferred embodiment, X2, X3 and X4 are independently of each other D-arginine or L-arginine, preferably L-arginine. In a most preferred embodiment, X2, X3 and X4 are D-arginine or L-arginine, preferably L-arginine.

In one embodiment, X5 of the peptide of the invention is an alanine derivative, wherein the alanine derivative is selected from alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, and wherein preferably said alanine derivative is a D-stereoisomer or L-stereoisomer, further preferably an L-stereoisomer. Preferably, said alanine derivative is D-alanine or L-alanine, preferably L-alanine. In another embodiment, X5 is a D-amino acid or L-amino acid, preferably an L-amino acid, wherein the D- or L-amino acid has a side chain comprising, or alternatively consisting of, at least one benzyl group. Preferably, X5 is a phenylalanine derivative, wherein the phenylalanine derivative is selected from tyrosine, 4-methyl-phenylalanine, homophenylalanine, alanine or phenylalanine, and wherein preferably said phenylalanine derivative is a D-stereoisomer or L-stereoisomer, further preferably an L-stereoisomer. In a more preferred embodiment, the phenylalanine derivative is a D-stereoisomer or L-stereoisomer of tyrosine, phenylalanine or alanine, wherein the L-stereoisomer is preferred. In a most preferred embodiment, the phenylalanine derivative is D-phenylalanine or L-phenylalanine, preferably L-phenylalanine.

In one embodiment, X6 of the peptide of the invention is an alanine derivative, wherein the alanine derivative is selected from alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, and wherein preferably said alanine derivative is a D-stereoisomer or L-stereoisomer, further preferably an L-stereoisomer. Preferably, the alanine derivative is D-alanine or L-alanine, preferably L-alanine. In another embodiment, X6 is a D-amino acid or L-amino acid, preferably an L-amino acid, wherein the D- or L-amino acid has a side chain comprising, or alternatively consisting of, at least one carboxamide. Preferably, X6 is an asparagine derivative, wherein the asparagine derivative is selected from asparagine, cysteine-s-acetamide, glutamine hydroxamate, n5-methyl-glutamine, 3-methyl-glutamine, beta-hydroxyasparagine, n-methyl-asparagine or 2,5-diamino-4-hydroxy-5-oxopentanoic acid, and wherein preferably said asparagine derivative is a D-stereoisomer or L-stereoisomer, further preferably an L-stereoisomer. More preferably, said asparagine derivative is selected from a D-stereoisomer or L-stereoisomer of asparagine, glutamine, beta-hydroxyasparagine or 3-methyl-glutamine, wherein the L-stereoisomer is preferred. In a most preferred embodiment, X6 is D-asparagine or L-asparagine, wherein L-asparagine is preferred.

In another embodiment, X7 of the peptide of the invention is an alanine derivative, wherein the alanine derivative is selected from alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, and wherein preferably said alanine derivative is a D-stereoisomer or L-stereoisomer, further preferably an L-stereoisomer. Preferably the alanine derivative is D-alanine or L-alanine, preferably L-alanine. In another embodiment, X7 is a D-amino acid or L-amino acid, preferably an L-amino acid, wherein the D- or L-amino acid has a side chain comprising, or alternatively consisting of, at least one alcohol group. More preferably, X7 is a serine derivative, wherein the serine derivative is selected from serine 6-hydroxy-norleucine, 2-amino-3-hydroxy-4-methylpentanoic acid, beta-hydroxyleucine, 4-hydroxy-isoleucine, hydroxynorvaline, 2-amino-5-hydroxypentanoic acid, 3-hydroxy-valine, 2-hydroxyethyl-cysteine, 4-chloro-threonine, 4,5-dihydroxy-isoleucine, homoserine, allo-threonine, threonine, 3,3-dihydroxy-alanine, 4-hydroxy-threonine, phosphoserine or phosphothreonine, and wherein preferably said serine derivative is a D-stereoisomer or L-stereoisomer, further preferably an L-stereoisomer. Even more preferably, the serine derivative is selected from a D-stereoisomer or L-stereoisomer of serine, threonine, 3,3-dihydroxy-alanine or homoserine, wherein the L-stereoisomer is preferred. In a most preferred embodiment, X7 is D-serine or L-serine, preferably L-serine.

In one embodiment, X8 and X18 are independently of each other a D-amino acid or L-amino acid, preferably an L-amino acid, wherein the D- or L-amino acid has a side chain comprising, or alternatively consisting of, at least one aliphatic hydrocarbon. Preferably, X8 and X18 of the peptide of the invention are independently of each other a leucine derivative, wherein the leucine derivative is selected from leucine, alpha-amino-2-indanacetic acid, isoleucine, tertleucine, leucine, allo-isoleucine, norleucine, 5-bromo-isoleucine, homoleucine, 5,5,5-trifluoro-leucine, 3-methyl-alloisoleucine, 4-hydroxy-isoleucine, 4,5-dihydroxy-isoleucine, beta-hydroxyleucine, 6-hydroxy-norleucine, (4r)-5-oxo-leucine, 5-oxo-norleucine, (4s)-5-fluoro-leucine, 2-aminobutyric acid, 2-aminoheptanoic acid, diethylalanine, norvaline, (2s)-2-amino-4,4-difluorobutanoic acid, hydroxynorvaline, valine, 3-fluoro-valine, 3-hydroxy-valine, 4-oxo-valine or 3-hydroxy-valine, and wherein preferably said leucine derivative is a D-stereoisomer or L-stereoisomer, further preferably an L-stereoisomer. More preferably, the leucine derivative is D-leucine or L-leucine, preferably L-leucine. In another preferred embodiment, X8 and X18 of the peptide of the invention are independently of each other an alanine derivative, wherein the alanine derivative is selected from alanine, adamanthane, 3-cyclohexyl-alanine, cyclohexylglycine, diethylalanine, 2-aminoheptanoic acid, 3-cyclopentyl-alanine, alpha-amino-2-indanacetic acid, isoleucine, tertleucine, leucine, allo-isoleucine, norleucine, 5-bromo-isoleucine, homoleucine, 5,5,5-trifluoro-leucine, 3-methyl-alloisoleucine, norvaline, valine, 2-aminobutyric acid, 2-allyl-glycine, 5-fluoro-leucine, cis-amiclenomycin, 3-fluoro-valine, glycine, vinylglycine, 3-chloro-alanine, 2-amino-4,4-difluorobutanoic acid or trifluoro-alanine, and wherein preferably said alanine derivative is a D-stereoisomer or L-stereoisomer, further preferably an L-stereoisomer. Preferably, said alanine derivative is selected from a D-stereoisomer or L-stereoisomer of alanine, glycine, 2-aminobutyric acid or vinylglycine, wherein the L-stereoisomer is preferred. In a further preferred embodiment, X8 and X18 are independently of each other D-alanine or L-alanine, preferably L-alanine. In a most preferred embodiment, X8 and X18 are D-alanine or L-alanine, preferably L-alanine.

In one embodiment, X9 of the peptide of the invention is an alanine derivative, wherein the alanine derivative is selected from alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, and wherein preferably said alanine derivative is a D-stereoisomer or L-stereoisomer, further preferably an L-stereoisomer. Preferably the alanine derivative is D-alanine or L-alanine, preferably L-alanine. In another preferred embodiment, X9 is a D-amino acid or L-amino acid, preferably an L-amino acid, wherein the D- or L-amino acid has a negative net charge at pH 7. Preferably, X9 is an aspartate derivative, wherein the aspartate derivative is selected from aspartate, 2-amino-6-methylene-pimelic acid, 4-fluoro-glutamic acid, carboxymethylated cysteine, 2-amino-6-oxopimelic acid, 3,3-dimethylaspartic acid, 4-methylglutamate, 3-methyl-glutamic acid, 2-aminoadipic acid, 5-o-methylglutamic acid, 3-methylaspartic acid, glutamate, 2-amino-propanedioic acid, 4-hydroxyglutamic acid, beta-hydroxyaspartic acid or 6-carboxylysine, and wherein preferably said aspartate derivative is a D-stereoisomer or L-stereoisomer, further preferably an L-stereoisomer. More preferably, the aspartate derivative is selected from a D-stereoisomer or L-stereoisomer of aspartate, glutamate, 3-methyl-aspartic acid, beta-hydroxyaspartic acid or 3,3-dimethyl aspartic acid, wherein the L-stereoisomer is preferred. In a most preferred embodiment, X9 is D-aspartate or L-aspartate, preferably L-aspartate.

Y10 of the peptide of the invention is an aromatic D-amino acid or an aromatic L-amino acid, wherein the aromatic L-amino acid is preferred. In a preferred embodiment, Y10 is a D-amino acid or L-amino acid, preferably an L-amino acid, wherein the D- or L-amino acid has a side chain comprising, or alternatively consisting of, at least one phenyl group. More preferably, Y10 is a tyrosine derivative, wherein preferably said tyrosine derivative is a D-stereoisomer or L-stereoisomer, further preferably an L-stereoisomer. In a further embodiment, the tyrosine derivative is selected from a D-stereoisomer or L-stereoisomer of phenylalanine, 4-methylphenylalanine, homophenylalanine or tyrosine. Even more preferably, the tyrosine derivative is D-tyrosine or L-tyrosine, preferably L-tyrosine. In another preferred embodiment, Y10 is a D-amino acid or L-amino acid, preferably an L-amino acid, wherein the D- or L-amino acid has a side chain comprising, or alternatively consisting of, at least one imidazole group. More preferably, Y10 is a histidine derivative, wherein the histidine derivative is selected from 2-fluoro-histidine, 2-fluoro-histidine, 2-fluoro-histidine, (2-furyl)-alanine, histidine, 3-(1-pyrazolyl)-alanine, 3-(2-tetrazolyl)-alanine, 3-(3-thienyl)-alanine, 3-(2-thienyl)-alanine, 3-(1,2,4-triazol-1-yl)-alanine or (4-thiazolyl)-alanine, and wherein preferably said histidine derivative is a D-stereoisomer or L-stereoisomer, further preferably an L-stereoisomer. Even more preferably, the histidine derivative is D-histidine or L-histidine, preferably L-histidine. In another preferred embodiment, Y10 is a D-amino acid or L-amino acid, preferably an L-amino acid, wherein the D- or L-amino acid has a side chain comprising, or alternatively consisting of, at least one indole group. More preferably, Y10 is a tryptophan derivative, wherein the tryptophan derivative is selected from 7-hydroxy-tryptophan, 3-(4H-thieno[3,2-b]pyrrol-6-yl)-alanine, 4-fluoro-tryptophan, 4-hydroxy-tryptophan, 4-amino-tryptophan, 6-chloro-tryptophan, 3-(3-benzothienyl)-alanine, 6-bromo-tryptophan, 7-chloro-tryptophan, 6-fluoro-tryptophan, 5-fluoro-tryptophan, 5-hydroxy-tryptophan, beta-hydroxy-tryptophan, 5-methoxy-tryptophan, 5-methyl-tryptophan, 6-methyl-tryptophan, 2-hydroxy-tryptophan, tryptophan, 6-hydroxy-tryptophan or 6-amino-7-hydroxy-tryptophan, and wherein preferably said tryptophan derivative is a D-stereoisomer or L-stereoisomer, further preferably an L-stereoisomer. Even more preferably, the tryptophan derivative is D-tryptophan or L-tryptophan, preferably L-tryptophan. In an again more preferred embodiment, Y10 is a D-amino acid or L-amino acid, preferably an L-amino acid, wherein the D- or L-amino acid is selected from a D-stereoisomer or L-stereoisomer of tyrosine, phenylalanine, histidine or tryptophan. Most preferably, Y10 is D-tyrosine or L-tyrosine, preferably L-tyrosine.

In one embodiment, X11 and X17 of the peptide of the invention are independently of each other an alanine derivative, wherein the alanine derivative is selected from alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, and wherein preferably said alanine derivative is a D-stereoisomer or L-stereoisomer, further preferably an L-stereoisomer. Preferably the alanine derivative is D-alanine or L-alanine, preferably L-alanine. In another embodiment, X11 and X17 are independently of each other a D-amino acid or L-amino acid, preferably an L-amino acid, wherein the D- or L-amino acid has a positive net charge at pH 7. In a preferred embodiment, X11 and X17 are independently of each other (i) an arginine derivative, wherein the arginine derivative is selected from of arginine, citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline or 4-guanidinobutryric acid, and wherein preferably said arginine derivative is a D-stereoisomer or L-stereoisomer, further preferably an L-stereoisomer; or (ii) a lysine derivative, wherein the lysine derivative is selected from lysine, diaminobutyric acid, 2,3-diaminopropanoic acid, 2,8-diaminooctanoic acid, ornithine or thialysine, and wherein preferably said lysine derivative is a D-stereoisomer or L-stereoisomer, further preferably an L-stereoisomer. More preferably, X11 and X17 are independently of each other selected from a D-stereoisomer or L-stereoisomer of arginine, canavanine, 2-amino-4-guanidinobutryric acid, diaminobutyric acid, 2,3-diaminopropanoic acid, ornithine, 2,8-diaminooctanoic acid, lysine, thialysine, 5-methyl-arginine, c-gamma-hydroxy arginine, homoarginine or 2-amino-3-guanidinopropionic acid, wherein the L-stereoisomer is preferred. Even more preferably, X11 and X17 are independently of each other selected from a D-stereoisomer or L-stereoisomer of diaminobutyric acid, 2,3-diaminopropanoic acid, 2,8-diaminooctanoic acid, ornithine, thialysine or lysine, wherein the L-stereoisomer is preferred. In an again further preferred embodiment, X11 and X17 are independently of each other D-lysine or L-lysine, preferably L-lysine. In a most preferred embodiment, X11 and X17 are D-lysine or L-lysine, preferably L-lysine.

G12 of the peptide of the invention is glycine or a glycine derivative, wherein the glycine derivative is selected from alanine, N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine or ethyl acetamidoacetate, wherein the glycine derivative is a D-stereoisomer or L-stereoisomer, more preferably an L-stereoisomer. Most preferably, G12 is glycine.

In one embodiment, X13 of the peptide of the invention is an alanine derivative, wherein the alanine derivative is selected from alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, and wherein preferably said alanine derivative is a D-stereoisomer or L-stereoisomer, further preferably an L-stereoisomer. Preferably the alanine derivative is D-alanine or L-alanine, preferably L-alanine. In a preferred embodiment, X13 is an L-amino acid or D-amino acid, preferably an L-amino acid, wherein the D- or L-amino acid has a side chain comprising, or alternatively consisting of, at least one pyrrolidine. More preferably, X13 is a proline derivative, wherein the proline derivative is selected from proline, 4-hydroxyproline, pipecolic acid, 1-acetyl-4-hydroxy-proline or alpha-methyl-proline, and wherein preferably said proline derivative is a D-stereoisomer or L-stereoisomer, further preferably an L-stereoisomer. In a most preferred embodiment, X13 is D-proline or L-proline, preferably L-proline.

In a preferred embodiment, D1 and D19 of the peptide of the invention are independently of each other a (i) deletion; or (ii) glycine or a glycine derivative selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine, ethyl acetamidoacetate, alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, wherein the glycine derivative is a D-stereoisomer or L-stereoisomer, more preferably an L-stereoisomer; or (iii) D1 and D19 are independently of each other an aspartate derivative selected from 2-amino-6-methylene-pimelic acid, 4-fluoro-glutamic acid, carboxymethylated cysteine, 2-amino-6-oxopimelic acid, 3,3-dimethyl aspartic acid, 4-methylglutamate, 3-methyl-glutamic acid, 2-aminoadipic acid, 5-o-methyl-glutamic acid, 3-methyl-aspartic acid, glutamate, 2-amino-propanedioic acid, 4-hydroxy-glutamic-acid, beta-hydroxyaspartic acid, 6-carboxylysine or aspartate, wherein the aspartate derivative is a D-stereoisomer or L-stereoisomer, more preferably an L-stereoisomer, wherein alternative (iii) is preferred;

R14, R15 and R16 are independently of each other (i') an arginine derivative, wherein said arginine derivative is selected from arginine, citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline or 4-guanidinobutryric acid, and wherein preferably said arginine derivative is a D-stereoisomer or L-stereoisomer, further preferably an L-stereoisomer, and wherein further preferably said arginine derivative is D-arginine or L-arginine; or (ii') a lysine derivative, wherein the lysine derivative is selected from lysine, diaminobutyric acid, 2,3-diaminopropanoic acid, 2,8-diaminooctanoic acid, ornithine or thialysine, and wherein preferably said lysine derivative is a D-stereoisomer or L-stereoisomer, further preferably an L-stereoisomer, and wherein further preferably said lysine derivative is D-lysine or L-lysine;

Y10 is (i") a tyrosine derivative, wherein the tyrosine derivative is selected from phenylalanine, 4-methylphenylalanine, homophenylalanine or tyrosine, and wherein preferably said tyrosine derivative is a D-stereoisomer or L-stereoisomer, further preferably an L-stereoisomer; or Y10 is (ii") a histidine derivative, wherein the histidine derivative is selected from 2-fluoro-histidine, 2-fluoro-histidine, 2-fluoro-histidine, (2-furyl)-alanine, histidine, 3-(1-pyrazolyl)-alanine, 3-(2-tetrazolyl)-alanine, 3-(3-thienyl)-alanine, 3-(2-thienyl)-alanine, 3-(1,2,4-triazol-1-yl)-alanine or (4-thiazolyl)-alanine, and wherein preferably said histidine derivative is a D-stereoisomer or L-stereoisomer, further preferably an L-stereoisomer; or Y10 is (iii") a tryptophan, wherein the tryptophan derivative is selected from 7-hydroxy-tryptophan, 3-(4H-thieno[3,2-b]pyrrol-6-yl)-alanine, 4-fluoro-tryptophan, 4-hydroxy-tryptophan, 4-amino-tryptophan, 6-chloro-tryptophan, 3-(3-benzothienyl)-alanine, 6-bromo-tryptophan, 7-chloro-tryptophan, 6-fluoro-tryptophan, 5-fluoro-tryptophan, 5-hydroxy-tryptophan, beta-hydroxy-tryptophan, 5-methoxy-tryptophan, 5-methyl-tryptophan, 6-methyl-tryptophan, 2-hydroxy-tryptophan, tryptophan, 6-hydroxy-tryptophan or 6-amino-7-hydroxy-tryptophan, and wherein preferably said tryptophan derivative is a D-stereoisomer or L-stereoisomer, further preferably an L-stereoisomer; and wherein preferably Y10 is an aromatic D-amino acid or L-amino acid, preferably an aromatic L-amino acid, wherein said aromatic D- or L-amino acid is selected from a D-stereoisomer or L-stereoisomer of phenylalanine, 4-methylphenylalanine, homophenylalanine, tyrosine, histidine or tryptophan; and

G12 is glycine or a glycine derivative selected from 2-aminobutyric acid, vinylglycine, benzylglycine, allylglycine, glycine C1-C3 alkylester, C1-C3 alkylglycine, C2-C3 alkenylglycine, formylglycine, N-formylglycine or ethyl acetamidoacetate, wherein the glycine derivative is a D-stereoisomer or L-stereoisomer, preferably an L-stereoisomer; wherein G12 is preferably glycine.

In another preferred embodiment, D1 and D19 of the peptide of the invention are independently of each other an aspartate derivative, wherein the aspartate derivative is selected from 2-amino-6-methylene-pimelic acid, 4-fluoro-glutamic acid, carboxymethylated cysteine, 2-amino-6-oxopimelic acid, 3,3-dimethyl aspartic acid, 4-methylglutamate, 3-methyl-glutamic acid, 2-aminoadipic acid, 5-o-methyl-glutamic acid, 3-methyl-aspartic acid, glutamate, 2-amino-propanedioic acid, 4-hydroxy-glutamic-acid, beta-hydroxyaspartic acid, 6-carboxylysine or aspartate, wherein preferably the aspartate derivative is aspartate, and wherein said aspartate derivative is a D-stereoisomer or L-stereoisomer, more preferably an L-stereoisomer;

R14, R15 and R16 are independently of each other an arginine derivative, wherein the arginine derivative is selected from citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline or 4-guanidinobutryric acid or arginine, and wherein said arginine derivative is a D-stereoisomer or L-stereoisomer, more preferably an L-stereoisomer, preferably R14, R15 and R16 are independently of each other D-arginine or L-arginine, preferably L-arginine;

Y10 is an aromatic amino acid, wherein the amino acid is selected from 4-methylphenylalanine, homophenylalanine, phenylalanine, tyrosine, histidine or tryptophan, preferably said aromatic acid is selected from phenylalanine, tyrosine, histidine or tryptophan, and wherein said aromatic amino acid is a D-stereoisomer or L-stereoisomer, more preferably an L-stereoisomer; and

G12 is a glycine derivative, wherein the glycine derivative is selected from 2-aminobutyric acid, vinylglycine, benzylglycine, allylglycine, glycine C1-C3 alkylester, C1-C3 alkylglycine, C2-C3 alkenylglycine, formylglycine, N-formylglycine or ethyl acetamidoacetate, wherein the glycine derivative is a D-stereoisomer or L-stereoisomer, more preferably an L-stereoisomer; even more preferably, G12 is glycine.

In another preferred embodiment, D1 and D19 of the peptide of the invention are independently of each other glycine or a D- or L-amino acid, wherein the D- or L-amino acid is selected from vinylglycine, 2-allyl-glycine, alanine, 2-aminobutyric acid, diethylalanine, allo-isoleucine, isoleucine, norleucine, leucine, valine, norvaline, 3-methyl-aspartic acid, beta-hydroxyaspartic acid, 3,3-dimethyl aspartic acid or aspartate, wherein preferably D1 and D19 are independently of each other glycine or D- or L-aspartate, more preferably glycine or L-aspartate; R14, R15 and R16 are independently of each other an arginine derivative or a lysine derivative, and wherein preferably said arginine derivative and said lysine derivative is a D-stereoisomer or L-stereoisomer, further preferably wherein said arginine derivative and said lysine derivative is an L-stereoisomer, wherein the arginine derivative or lysine derivative is selected from a D-stereoisomer or L-stereoisomer of 5-methyl-arginine, c-gamma-hydroxy arginine, homoarginine, citrulline, 2-amino-4-guanidinobutryric acid, 2-amino-3-guanidinopropionic acid, lysine, ornithine, 2,8-diaminooctanoic acid or arginine; preferably R14, R15 and R16 are independently of each other D-arginine or L-arginine, preferably L-arginine; Y10 is an aromatic D- or L-amino acid preferably an aromatic L-amino acid, wherein the aromatic D- or L-amino acid is selected from phenylalanine, histidine, tryptophan or tyrosine, preferably Y10 is D- or L-tyrosine, preferably L-tyrosine; and G12 is glycine.

In another preferred embodiment, D1 and D19 of the peptide of the invention are independently of each other an aspartate derivative, wherein the aspartate derivative is selected from 3-methyl-aspartic acid, beta-hydroxyaspartic acid, 3,3-dimethyl aspartic acid or aspartate, and wherein the aspartate derivative is a D-stereoisomer or L-stereoisomer, more preferably an L-stereoisomer; R14, R15 and R16 are independently of each other an arginine derivative, wherein the arginine derivative is selected from 5-methyl-arginine, c-gamma-hydroxy arginine and homoarginine, citrulline, 2-amino-4-guanidinobutryric acid, 2-amino-3-guanidinopropionic acid or arginine, and wherein preferably said arginine derivative is a D-stereoisomer or L-stereoisomer, further preferably an L-stereoisomer; Y10 is an aromatic D-or L-amino acid, preferably an aromatic L-amino acid, wherein the aromatic D- or L-amino acid is selected from a D-stereoisomer or L-stereoisomer of phenylalanine, histidine, tryptophan or tyrosine; and G12 is glycine.

In another preferred embodiment, D1 and D19 of the peptide of the invention are independently of each other an aspartate derivative, wherein the aspartate derivative is selected from 2-amino-6-methylene-pimelic acid, 4-fluoro-glutamic acid, carboxymethylated cysteine, 2-amino-6-oxopimelic acid, 3,3-dimethyl aspartic acid, 4-methylglutamate, 3-methyl-glutamic acid, 2-aminoadipic acid, 5-o-methyl-glutamic acid, 3-methyl-aspartic acid, glutamate, 2-amino-propanedioic acid, 4-hydroxy-glutamic-acid, beta-hydroxyaspartic acid, 6-carboxylysine or aspartate; preferably the aspartate derivative is selected from 3-methyl-aspartic acid, beta-hydroxyaspartic acid, 3,3-dimethyl aspartic acid or aspartate; R14, R15 and R16 are independently of each other a lysine derivative, wherein the lysine derivative is selected from lysine, diaminobutyric acid, 2,3-diaminopropanoic acid, 2,8-diaminooctanoic acid, ornithine or thialysine; more preferably the lysine derivative is lysine; Y10 is an aromatic amino acid, wherein the aromatic acid is selected from phenylalanine, 4-methylphenylalanine, homophenylalanine, tyrosine, histidine, tryptophan, 7-hydroxy-tryptophan, 4-hydroxy-tryptophan, 4-amino-tryptophan, 6-chloro-tryptophan, 5-hydroxy-tryptophan, beta-hydroxy-tryptophan, 5-methoxy-tryptophan, 5-methyl-tryptophan, 6-methyl-tryptophan, 2-hydroxy-tryptophan, 6-hydroxy-tryptophan or 6-amino-7-hydroxy-tryptophan; preferably the aromatic amino acid is selected from phenylalanine, tyrosine, histidine or tryptophan; and G12 is glycine or a glycine derivative, wherein the glycine derivative is selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine or ethyl acetamidoacetate; preferably G12 is glycine, and wherein D1, D19, R14, R15, R16, Y10 and G12 are independently of each other a D-amino acid or L-amino acid, more preferably an L-amino acid.

In another preferred embodiment, D1 and D19 of the peptide of the invention are independently of each other a glycine derivative, wherein the glycine derivative is selected from glycine, N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine, ethyl acetamidoacetate, alanine, adamanthane, 3-cyclohexyl-alanine, cyclohexylglycine, diethylalanine, 2-aminoheptanoic acid, 3-cyclopentyl-alanine, alpha-amino-2-indanacetic acid, isoleucine, tertleucine, leucine, allo-isoleucine, norleucine, 5-bromo-isoleucine, homoleucine, 5,5,5-trifluoro-leucine, 3-methyl-alloisoleucine, norvaline, valine, 2-aminobutyric acid, 2-allyl-glycine, 5-fluoro-leucine, cis-amiclenomycin, 3-fluoro-valine, glycine, vinylglycine, 3-chloro-alanine, 2-amino-4,4-difluorobutanoic acid or trifluoro-alanine; preferably, the glycine derivative is selected from glycine, alanine, N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine or ethyl acetamidoacetate; R14, R15 and R16 are independently of each other a lysine derivative, wherein the lysine derivative is selected from lysine, diaminobutyric acid, 2,3-diaminopropanoic acid, 2,8-diaminooctanoic acid, ornithine or thialysine; preferably R14, R15 and R16 are independently of each other lysine; Y10 is an aromatic amino acid, wherein the aromatic amino acid is selected from phenylalanine, 4-methylphenylalanine, homophenylalanine, tyrosine, histidine, tryptophan, 7-hydroxy-tryptophan, 4-hydroxy-tryptophan, 4-amino-tryptophan, 6-chloro-tryptophan, 5-hydroxy-tryptophan, beta-hydroxy-tryptophan, 5-methoxy-tryptophan, 5-methyl-tryptophan, 6-methyl-tryptophan, 2-hydroxy-tryptophan, 6-hydroxy-tryptophan or 6-amino-7-hydroxy-tryptophan; preferably the aromatic amino acid is selected from phenylalanine, tyrosine, histidine or tryptophan; and G12 is glycine or a glycine derivative selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine or ethyl acetamidoacetate; more preferably G12 is glycine; and wherein D1, D19, R14, R15, R16, Y10 and G12 are independently of each other a D-amino acid or L-amino acid, more preferably an L-amino acid.

In another preferred embodiment, D1 and D19 of the peptide of the invention are independently of each other a glycine derivative, wherein the glycine derivative is selected from glycine, N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine, ethyl acetamidoacetate, alanine, adamanthane, 3-cyclohexyl-alanine, cyclohexylglycine, diethylalanine, 2-aminoheptanoic acid, 3-cyclopentyl-alanine, alpha-amino-2-indanacetic acid, isoleucine, tertleucine, leucine, allo-isoleucine, norleucine, 5-bromo-isoleucine, homoleucine, 5,5,5-trifluoro-leucine, 3-methyl-alloisoleucine, norvaline, valine, 2-aminobutyric acid, 2-allyl-glycine, 5-fluoro-leucine, cis-amiclenomycin, 3-fluoro-valine, glycine, vinylglycine, 3-chloro-alanine, 2-amino-4,4-difluorobutanoic acid or trifluoro-alanine; preferably, D 1 and D 19 of the peptide of the invention are independently of each other a glycine or a glycine derivative selected from alanine, N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine or ethyl acetamidoacetate; R14, R15 and R16 are independently of each other an arginine derivative, wherein the arginine derivative is selected from arginine, citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline or 4-guanidinobutryric acid; preferably R14, R15 and R16 are independently of each other an arginine derivative, wherein the arginine derivative is selected from 5-methyl-arginine, c-gamma-hydroxy arginine, homoarginine, citrulline, 2-amino-4-guanidinobutryric acid, 2-amino-3-guanidinopropionic acid or arginine; Y10 is an aromatic amino acid, wherein the aromatic amino acid is selected from phenylalanine, 4-methylphenylalanine, homophenylalanine, tyrosine, histidine, tryptophan, 7-hydroxy-tryptophan, 4-hydroxy-tryptophan, 4-amino-tryptophan, 6-chloro-tryptophan, 5-hydroxy-tryptophan, beta-hydroxy-tryptophan, 5-methoxy-tryptophan, 5-methyl-tryptophan, 6-methyl-tryptophan, 2-hydroxy-tryptophan, 6-hydroxy-tryptophan or 6-amino-7-hydroxy-tryptophan; preferably the aromatic amino acid is selected from phenylalanine, tyrosine, histidine or tryptophan; and G12 is glycine or a glycine derivative selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine or ethyl acetamidoacetate; preferably G12 is glycine; and wherein D1, D19, R14, R15, R16, Y10 and G12 are independently of each other a D-amino acid or L-amino acid, more preferably an L-amino acid.

In another preferred embodiment, D1 and D19 of the peptide of the invention are independently of each other a glycine derivative, wherein the glycine derivative is selected from glycine, N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine, ethyl acetamidoacetate, alanine, adamanthane, 3-cyclohexyl-alanine, cyclohexylglycine, diethylalanine, 2-aminoheptanoic acid, 3-cyclopentyl-alanine, alpha-amino-2-indanacetic acid, isoleucine, tertleucine, leucine, allo-isoleucine, norleucine, 5-bromo-isoleucine, homoleucine, 3-methyl-alloisoleucine, norvaline, valine, 2-aminobutyric acid, 2-allyl-glycine or vinylglycine; preferably, D1 and D19 are independently of each other glycine or a glycine derivative selected from alanine, N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine or ethyl acetamidoacetate; R14, R15 and R16 are independently of each other an arginine derivative or lysine derivative, wherein the arginine derivative or lysine derivative is selected from arginine, citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline, 4-guanidinobutryric acid, lysine, diaminobutyric acid, 2,3-diaminopropanoic acid, 2,8-diaminooctanoic acid, ornithine or thialysine; preferably, R14, R15 and R16 are independently of each other an arginine derivative selected from 5-methyl-arginine, c-gamma-hydroxy arginine, homoarginine, citrulline, 2-amino-4-guanidinobutryric acid, 2-amino-3-guanidinopropionic acid or arginine; Y10 is an aromatic amino acid, wherein the aromatic amino acid is selected from phenylalanine, tyrosine, histidine or tryptophan; more preferably Y10 is tyrosine; and G12 is glycine or a glycine derivative, wherein the glycine derivative is selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine or ethyl acetamidoacetate; more preferably G12 is glycine, and wherein D1, D19, R14, R15, R16, Y10 and G12 are independently of each other a D-amino acid or L-amino acid, more preferably an L-amino acid.

In another preferred embodiment, D1 and D19 of the peptide of the invention are independently of each other glycine or alanine, more preferably glycine; R14, R15 and R16 are independently of each other lysine or arginine, more preferably arginine; Y10 is an aromatic amino acid, wherein the aromatic amino acid is selected from phenylalanine, tyrosine, histidine or tryptophan, more preferably Y10 is tyrosine; and G12 is glycine or a glycine derivative selected from N-acetylglycine, 2-aminobutyric acid, vinylglycine, 2-allylglycine, C1-C2 N-alkylglycine or N-formylglycine; more preferably G12 is glycine; and wherein D1, D19, R14, R15, R16, Y10 and G12 are independently of each other a D-amino acid or L-amino acid, more preferably an L-amino acid.

In another preferred embodiment, D1 and D19 of the peptide of the invention are independently of each other alanine; R14, R15 and R16 are independently of each other arginine; Y10 is an aromatic amino acid, wherein the aromatic amino acid is selected from phenylalanine, tyrosine, histidine or tryptophan, more preferably Y10 is tyrosine; and G12 is glycine or a glycine derivative, wherein the glycine derivative is selected from N-acetylglycine, 2-aminobutyric acid, vinylglycine, 2-allylglycine, C1-C2 N-alkylglycine or N-formylglycine; more preferably G12 is glycine; and wherein D1, D19, R14, R15, R16, Y10 and G12 are independently of each other a D-amino acid or L-amino acid, more preferably an L-amino acid.

In another preferred embodiment, D1 and D19 of the peptide of the invention are independently of each other alanine; R14, R15 and R16 are independently of each other lysine; Y10 is an aromatic amino acid, wherein the aromatic amino acid is selected from phenylalanine, tyrosine, histidine or tryptophan, more preferably Y10 is tyrosine; and G12 is glycine or a glycine derivative selected, wherein the glycine derivative is selected from N-acetylglycine, 2-aminobutyric acid, vinylglycine, 2-allylglycine, C1-C2 N-alkylglycine or N-formylglycine; more preferably G12 is glycine; and wherein D1, D19, R14, R15, R16, Y10 and G12 are independently of each other a D-amino acid or L-amino acid, more preferably an L-amino acid.

In another preferred embodiment, D1 and D19 of the peptide of the invention are independently of each other glycine; R14, R15 and R16 are independently of each other lysine; Y10 is an aromatic amino acid, wherein the aromatic amino acid is selected from phenylalanine, tyrosine, histidine or tryptophan, more preferably Y10 is tyrosine; and G12 is glycine or a glycine derivative, wherein the glycine derivative is selected from N-acetylglycine, 2-aminobutyric acid, vinylglycine, 2-allylglycine, C1-C2 N-alkylglycine or N-formylglycine; more preferably G12 is glycine; and wherein D1, D19, R14, R15, R16, Y10 and G12 are independently of each other a D-amino acid or L-amino acid, more preferably an L-amino acid.

In a more preferred embodiment of the invention, D1 and D19 are independently of each other D-aspartate or L-aspartate, preferably L-aspartate, X2, X3, X4, X5, X6, X7, X8, X9, X11, X13, X17 and X18 are independently of each other an amino acid or a deletion, wherein at most one of X2, X3, X4, X5, X6, X7, X8, X9, X11, X13, X17 or X18 is a deletion; R14, R15 and R16 are independently of each other L-arginine, Y10 is L-tyrosine, and G12 is glycine.

In again a more preferred embodiment of the invention, D1 and D19 are L-aspartate, X2, X3, X4, X5, X6, X7, X8, X9, X11, X13, X17 and X18 are independently of each other an amino acid or a deletion, wherein at most one of X2, X3, X4, X5, X6, X7, X8, X9, X11, X13, X17 or X18 is a deletion; R14, R15 and R16 are L-arginine, Y10 is L-tyrosine, and G12 is glycine.

In again a more preferred embodiment of the invention, D1 and D19 are L-aspartate, X2 and X3 are arginine, and X4, X5, X6, X7, X8, X9, X11, X13, X17 and X18 are independently of each other an amino acid or a deletion, wherein at most one of X2, X3, X4, X5, X6, X7, X8, X9, X11, X13, X17 or X18 is a deletion; R14, R15 and R16 are L-arginine, Y10 is L-tyrosine, and G12 is glycine.

In again a more preferred embodiment of the invention, D1 and D19 are L-aspartate, X2, X3 and X4 are L-arginine, and X5, X6, X7, X8, X9, X11, X13, X17 and X18 are independently of each other an amino acid or a deletion, wherein at most one of X2, X3, X4, X5, X6, X7, X8, X9, X11, X13, X17 or X18 is a deletion; R14, R15 and R16 are L-arginine, Y10 is L-tyrosine, and G12 is glycine.

In a preferred embodiment, at least one of said amino acid sequence (I) has at least 73 % sequence identity, preferably at least 78 % sequence identity, more preferably at least 84 % sequence identity, even more preferably at least 89 % sequence identity, again more preferably at least 94 % sequence identity to the amino acid sequence of DRRRFNSADYKGPRRRKAD (SEQ ID NO: 52).

In another preferred embodiment, at least one of said amino acid sequence (I), preferably all of said at least one amino acid sequence (I), is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently selected from the following:
D at positions 1 and 19 are independently of each other optionally exchanged by (i) a deletion, (ii) an amino acid having a negative net charge at pH 7, wherein preferably said amino acid having a negative net charge at pH 7 is a D-amino acid or L-amino acid, and further D-amino acid or L-amino acid, preferably an L-amino acid having a negative net charge at pH 7, or (iii) glycine or a glycine derivative selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine, ethyl acetamidoacetate, alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, wherein preferably said glycine derivative is a D-stereoisomer or L-stereoisomer, further preferably an L-stereoisomer, and wherein again further preferably D at positions 1 and 19 are independently of each other optionally exchanged by glycine;
R at positions 14, 15 and 16 are independently of each other optionally exchanged by a basic amino acid, preferably by a basic D-amino acid or a basic L-amino acid, and further preferably by a basic L-amino acid;
amino acids at positions 2, 3, 4, 5, 6, 7, 8, 9, 11, 13, 17 and 18 are independently of each other optionally exchanged by any amino acid, preferably by a D-amino acid or L-amino acid, and further preferably an L-amino acid, or a deletion, wherein at most one of positions 2, 3, 4, 5, 6, 7, 8, 9, 11, 13, 17 and 18 is optionally exchanged by a deletion;
Y at position 10 is optionally exchanged by an aromatic amino acid, preferably by an aromatic D-amino acid or an aromatic L-amino acid, preferably an aromatic L-amino acid; and
G at position 12 is optionally exchanged by a glycine derivative selected from 2-aminobutyric acid, vinylglycine, benzylglycine, allylglycine, glycine C1-C3 alkylester, C1-C3 alkylglycine, C2-C3 alkenylglycine, formylglycine, N-formylglycine or ethyl acetamidoacetate, wherein preferably said glycine derivative is a D-stereoisomer or L-stereoisomer, further preferably an L-stereoisomer, and wherein said peptide has a length of at most 160 amino acids, preferably of at most 100 amino acids, further preferably of at most 50 amino acids and most preferably of at most 30 amino acids.

At positions 1 and 19, said amino acid having a negative net charge at pH 7, preferably said D-amino acid or L-amino acid having a negative net charge at pH 7, and further preferably said L-amino acid having a negative net charge at pH 7, is independently of each other preferably D-aspartate or an aspartate derivative, wherein the aspartate derivative is selected from 2-amino-6-methylene-pimelic acid, 4-fluoro-glutamic acid, carboxymethylated cysteine, 2-amino-6-oxopimelic acid, 3,3-dimethyl aspartic acid, 4-methylglutamate, 3-methyl-glutamic acid, 2-aminoadipic acid, 5-o-methyl-glutamic acid, 3-methyl-aspartic acid, glutamate, 2-amino-propanedioic acid, 4-hydroxy-glutamic-acid, beta-hydroxyaspartic acid, or 6-carboxylysine, and wherein preferably said aspartate derivative is a D-stereoisomer or L-stereoisomer, further preferably wherein said aspartate derivative is an L-stereoisomer. More preferably, at positions 1 and 19, said amino acid having a negative net charge at pH 7 is D-aspartate or an aspartate derivative, wherein the aspartate derivative is selected from glutamate, 3-methyl-aspartic acid, beta-hydroxyaspartic acid or 3,3-dimethyl aspartic acid, and wherein preferably said aspartate derivative is a D-stereoisomer or L-stereoisomer, further preferably an L-stereoisomer. Again more preferably, at positions 1 and 19, said D- or L-amino acid having a negative net charge at pH 7 is D-aspartate.

At positions 14, 15 and 16, said basic amino acid, preferably said basic D- or L-amino acid, is independently of each other (i') a lysine derivative, wherein said lysine derivative is selected from diaminobutyric acid, 2,3-diaminopropanoic acid, 2,8-diaminooctanoic acid, ornithine, thialysine or lysine, and wherein preferably said lysine derivative is a D-stereoisomer or L-stereoisomer, further preferably an L-stereoisomer, or (ii') D-arginine or an arginine derivative, wherein the arginine derivative is selected from citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline or 4-guanidinobutryric acid, and wherein preferably said arginine derivative is a D-stereoisomer or L-stereoisomer, further preferably an L-stereoisomer. More preferably at positions 14, 15 and 16, said basic amino acid, preferably said basic D- or L-amino acid, is independently of each other D-arginine or an arginine derivative, wherein said arginine derivative is selected from a D-stereoisomer or L-stereoisomer, preferably from an L-stereoisomer, of citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline or 4-guanidinobutryric acid. Even more preferably, said basic D- or L-amino acid at positions 14, 15 and 16 is independently of each other D-lysine, L-lysine or D-arginine.

At position 10, said aromatic amino acid, preferably said aromatic D- or L-amino acid, is independently of each other (i') D-tyrosine, or a tyrosine derivative, wherein said tyrosine derivative is selected from phenylalanine, 4-methylphenylalanine or homophenylalanine, and wherein preferably said tyrosine derivative is a D-stereoisomer or L-stereoisomer, further preferably an L-stereoisomer, and wherein again further preferably said Y at position 10 is optionally exchanged by D-tyrosine; or (ii') a histidine derivative, wherein said histidine derivative is selected from 2-fluoro-histidine, 2-fluoro-histidine, 2-fluoro-histidine, (2-furyl)-alanine, histidine, 3-(1-pyrazolyl)-alanine, 3-(2-tetrazolyl)-alanine, 3-(3-thienyl)-alanine, 3-(2-thienyl)-alanine, 3-(1,2,4-triazol-1-yl)-alanine or (4-thiazolyl)-alanine, and wherein preferably said histidine derivative is a D-stereoisomer or L-stereoisomer, further preferably an L-stereoisomer, and wherein again further preferably said Y at position 10 is optionally exchanged by D- or L-histidine, preferably by L-histidine; or (iii') a tryptophan derivative, wherein said tryptophan derivative is selected from 7-hydroxy-tryptophan, 3-(4H-thieno[3,2-b]pyrrol-6-yl)-alanine, 4-fluoro-tryptophan, 4-hydroxy-tryptophan, 4-amino-tryptophan, 6-chloro-tryptophan, 3-(3-benzothienyl)-alanine, 6-bromo-tryptophan, 7-chloro-tryptophan, 6-fluoro-tryptophan, 5-fluoro-tryptophan, 5-hydroxy-tryptophan, beta-hydroxy-tryptophan, 5-methoxy-tryptophan, 5-methyl-tryptophan, 6-methyl-tryptophan, 2-hydroxy-tryptophan, tryptophan, 6-hydroxy-tryptophan or 6-amino-7-hydroxy-tryptophan, and wherein preferably said tryptophan derivative is a D-stereoisomer or L-stereoisomer, further preferably an L-stereoisomer, and wherein again further preferably said Y at position 10 is optionally exchanged by D- or L-tryptophan, preferably by L-tryptophan; and wherein further preferably said aromatic amino acid, preferably said D- or L-amino acid, at position 10 is independently of each other D-tyrosine, D-phenylalanine, L-phenylalanine, D-histidine, L-histidine, D-tryptophan or L-tryptophan, and again further preferably said aromatic amino acid at position 10 is D-tyrosine.

In a preferred embodiment, the at least one of said amino acid sequence (I) is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently of each other selected from the following:
D at positions 1 and 19 are independently of each other optionally exchanged by D-aspartate, glycine, an aspartate derivative or glycine derivative, wherein the aspartate derivative or glycine derivative is selected from vinylglycine, 2-allyl-glycine, alanine, 2-aminobutyric acid, diethylalanine, leucine, allo-isoleucine, isoleucine, norleucine, leucine, valine, norvaline, 3-methyl-aspartic acid, beta-hydroxyaspartic acid, or 3,3-dimethyl aspartic acid, wherein the aspartate derivative or glycine derivative is a D- or L-stereoisomer, more preferably an L-stereoisomer; and wherein D-aspartate is preferred; and
R at positions 14, 15 and 16 is independently of each other optionally exchanged by D-arginine or an arginine derivative selected from 5-methyl-arginine, c-gamma-hydroxy arginine, homoarginine, citrulline, 2-amino-4-guanidinobutryric acid, 2-amino-3-guanidinopropionic acid, lysine, ornithine, or 2,8-diaminooctanoic acid, wherein the arginine derivative is a D- or L-stereoisomer, more preferably an L-stereoisomer; and wherein D-arginine, L-lysine or D-lysine are preferred;
amino acids at positions 2, 3, 4, 5, 6, 7, 8, 9, 11, 13, 17 and 18 are independently of each other optionally exchanged by any other D-amino acid or L-amino acid (mentioned herein as X2, X3, X4, X5, X6, X7, X8, X9, X11, X13, X17 and X18), preferably D- or L-alanine or D- or L-arginine, or a deletion, wherein at most one of positions 2, 3, 4, 5, 6, 7, 8, 9, 11, 13, 17 and 18 is optionally a deletion;
Y at position 10 is optionally exchanged by D-tyrosine or a tyrosine derivative, wherein the tyrosine derivative is selected from phenylalanine, histidine or tryptophan, wherein the tyrosine derivative is a D- or L-stereoisomer, more preferably an L-stereoisomer; and wherein D-tyrosine, L-phenylalanine and D-phenylalanine are preferred; and
G at position 12 is optionally exchanged by a glycine derivative selected from 2-aminobutyric acid, glycine C1-C3 alkylester, C1-C3 alkylglycine, C2-C3 alkenylglycine, formylglycine, N-formylglycine or ethyl acetamidoacetate, wherein said glycine derivative is a D-stereoisomer or L-stereoisomer, preferably an L-stereoisomer;
and wherein said peptide has a length of at most 160 amino acids, preferably of at most 100 amino acids, further preferably of at most 80 amino acids, further preferably of at most 70 amino acids, further preferably of at most 50 amino acids and most preferably of at most 30 amino acids.

In a preferred embodiment, the at least one of said amino acid sequence (I) is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently of each other selected from the following:
D at positions 1 and 19 are independently of each other optionally exchanged by D-aspartate, glycine, an aspartate derivative or glycine derivative, wherein the aspartate derivative or glycine derivative is selected from vinylglycine, 2-allyl-glycine, alanine, 2-aminobutyric acid, diethylalanine, leucine, allo-isoleucine, isoleucine, norleucine, leucine, valine, norvaline, 3-methyl-aspartic acid, beta-hydroxyaspartic acid, or 3,3-dimethyl aspartic acid, wherein the aspartate derivative or glycine derivative is a D- or L-stereoisomer, more preferably an L-stereoisomer; and wherein D-aspartate is preferred; and
R at positions 14, 15 and 16 is independently of each other optionally exchanged by D-arginine or an arginine derivative selected from 5-methyl-arginine, c-gamma-hydroxy arginine, homoarginine, citrulline, 2-amino-4-guanidinobutryric acid, 2-amino-3-guanidinopropionic acid or lysine, wherein the arginine derivative is a D- or L-stereoisomer, more preferably an L-stereoisomer; and wherein D-arginine, L-lysine or D-lysine are preferred;
amino acids at positions 2, 3, 4, 5, 6, 7, 8, 9, 11, 13, 17 and 18 are independently of each other optionally exchanged by any other D-amino acid or L-amino acid (mentioned herein as X2, X3, X4, X5, X6, X7, X8, X9, X11, X13, X17 and X18), preferably D- or L-alanine or D- or L-arginine, or a deletion, wherein at most one of positions 2, 3, 4, 5, 6, 7, 8, 9, 11, 13, 17 and 18 is optionally a deletion;
Y at position 10 is optionally exchanged by D-tyrosine or a tyrosine derivative, wherein the tyrosine derivative is selected from phenylalanine, histidine or tryptophan, wherein the tyrosine derivative is a D- or L-stereoisomer, more preferably an L-stereoisomer; and wherein D-tyrosine, L-phenylalanine and D-phenylalanine are preferred; and
G at position 12 is optionally exchanged by a glycine derivative selected from 2-aminobutyric acid, glycine C1-C3 alkylester, C1-C3 alkylglycine, C2-C3 alkenylglycine, formylglycine, wherein said glycine derivative is a D-stereoisomer or L-stereoisomer, preferably an L-stereoisomer;
and wherein said peptide has a length of at most 160 amino acids, preferably of at most 100 amino acids, further preferably of at most 80 amino acids, further preferably of at most 70 amino acids, further preferably of at most 50 amino acids and most preferably of at most 30 amino acids.

In a preferred embodiment, the at least one of said amino acid sequence (I) is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently of each other selected from the following:
D at positions 1 and 19 are independently of each other optionally exchanged by D-aspartate, glycine or an aspartate derivative, wherein the aspartate derivative is selected from 3-methyl-aspartic acid, beta-hydroxyaspartic acid, or 3,3-dimethyl aspartic acid, wherein the aspartate derivative is a D- or L-stereoisomer, more preferably an L-stereoisomer; and wherein D-aspartate is preferred; and
R at positions 14, 15 and 16 is independently of each other optionally exchanged by D-arginine or an arginine derivative selected from 5-methyl-arginine, c-gamma-hydroxy arginine and homoarginine, citrulline, 2-amino-4-guanidinobutryric acid, 2-amino-3-guanidinopropionic acid, lysine, ornithine, or 2,8-diaminooctanoic acid, wherein the arginine derivative is a D- or L-stereoisomer, more preferably an L-stereoisomer; and wherein D-arginine is preferred;
amino acids at positions 2, 3, 4, 5, 6, 7, 8, 9, 11, 13, 17 and 18 are independently of each other optionally exchanged by any other D-amino acid or L-amino acid (mentioned herein as X2, X3, X4, X5, X6, X7, X8, X9, X11, X13, X17 and X18), preferably D- or L-alanine or D- or L-arginine, or a deletion, wherein at most one of positions 2, 3, 4, 5, 6, 7, 8, 9, 11, 13, 17 and 18 is optionally a deletion;
Y at position 10 is optionally exchanged by D-tyrosine or a tyrosine derivative, wherein the tyrosine derivative is selected from phenylalanine, histidine or tryptophan, wherein the tyrosine derivative is a D- or L-stereoisomer, more preferably an L-stereoisomer; and wherein D-tyrosine is preferred; and
G at position 12 is optionally exchanged by a glycine derivative selected from 2-aminobutyric acid, C1-C3 alkylglycine, C2-C3 alkenylglycine, formylglycine, wherein said glycine derivative is a D-stereoisomer or L-stereoisomer, preferably an L-stereoisomer;
and wherein said peptide has a length of at most 160 amino acids, preferably of at most 100 amino acids, further preferably of at most 80 amino acids, further preferably of at most 70 amino acids, further preferably of at most 50 amino acids and most preferably of at most 30 amino acids.

In a preferred embodiment, the at least one of said amino acid sequence (I) is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently of each other selected from the following:
D at positions 1 and 19 are independently of each other optionally exchanged by D-aspartate, glycine or a glycine derivative, wherein the glycine derivative is selected from vinylglycine, 2-allyl-glycine, alanine, 2-aminobutyric acid, diethylalanine, leucine, allo-isoleucine, isoleucine, norleucine, leucine, valine, norvaline, or 3-methyl-aspartic acid, wherein the glycine derivative is a D- or L-stereoisomer, more preferably an L-stereoisomer; and wherein D-glycine is preferred; and
R at positions 14, 15 and 16 is independently of each other optionally exchanged by D-arginine or an arginine derivative selected from 5-methyl-arginine, c-gamma-hydroxy arginine and homoarginine, citrulline, 2-amino-4-guanidinobutryric acid, 2-amino-3-guanidinopropionic acid, lysine, ornithine, or 2,8-diaminooctanoic acid, wherein the arginine derivative is a D- or L-stereoisomer, more preferably an L-stereoisomer; and wherein D-arginine is preferred;
amino acids at positions 2, 3, 4, 5, 6, 7, 8, 9, 11, 13, 17 and 18 are independently of each other optionally exchanged by any other D-amino acid or L-amino acid (mentioned herein as X2, X3, X4, X5, X6, X7, X8, X9, X11, X13, X17 and X18), preferably D- or L-alanine or D- or L-arginine, or a deletion, wherein at most one of positions 2, 3, 4, 5, 6, 7, 8, 9, 11, 13, 17 and 18 is optionally a deletion;
Y at position 10 is optionally exchanged by a D-tyrosine or tyrosine derivative, wherein the tyrosine derivative is selected from phenylalanine, histidine or tryptophan, wherein the tyrosine derivative is a D- or L-stereoisomer, more preferably an L-stereoisomer; and wherein D-tyrosine is preferred; and
G at position 12 is optionally exchanged by a glycine derivative selected from 2-aminobutyric acid, C1-C3 alkylglycine, C2-C3 alkenylglycine, formylglycine, wherein said glycine derivative is a D-stereoisomer or L-stereoisomer, preferably an L-stereoisomer;
and wherein said peptide has a length of at most 160 amino acids, preferably of at most 100 amino acids, further preferably of at most 80 amino acids, further preferably of at most 70 amino acids, further preferably of at most 50 amino acids and most preferably of at most 30 amino acids.

In a preferred embodiment, the at least one of said amino acid sequence (I) is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently of each other selected from the following:
D at positions 1 and 19 are independently of each other optionally exchanged by D-aspartate; R at positions 14, 15 and 16 is independently of each other optionally exchanged by D-arginine; amino acids at positions 2, 3, 4, 5, 6, 7, 8, 9, 11, 13, 17 and 18 are independently of each other optionally exchanged by any other D-amino acid or L-amino acid, preferably D- or L-alanine or D- or L-arginine, or a deletion, wherein at most one of positions 2, 3, 4, 5, 6, 7, 8, 9, 11, 13, 17 and 18 is optionally a deletion; and Y at position 10 is optionally exchanged by D-tyrosine; and wherein said peptide has a length of at most 160 amino acids, preferably of at most 100 amino acids, further preferably of at most 80 amino acids, further preferably of at most 70 amino acids, further preferably of at most 50 amino acids and most preferably of at most 30 amino acids.

In a preferred embodiment, the at least one of said amino acid sequence (I) is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently of each other selected from the following:
D at positions 1 and 19 are independently of each other optionally exchanged by D-aspartate; R at positions 14, 15 and 16 is independently of each other optionally exchanged by D-arginine;
amino acids at positions 2, 3 and 4 are independently of each other optionally exchanged by D-arginine or an arginine derivative, wherein the arginine derivative is selected from citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline or 4-guanidinobutryric acid, and wherein preferably said arginine derivative is a D-stereoisomer or L-stereoisomer, further preferably an L-stereoisomer;
amino acids at positions 5, 6, 7, 8, 9, 11, 13, 17 and 18 are independently of each other optionally exchanged by a D-amino acid or L-amino acid, preferably D- or L-alanine or D- or L-arginine, or a deletion, wherein at most one of positions 5, 6, 7, 8, 9, 11, 13, 17 and 18 is optionally a deletion;
and Y at position 10 is optionally exchanged by D-tyrosine; and wherein said peptide has a length of at most 160 amino acids, preferably of at most 100 amino acids, further preferably of at most 80 amino acids, further preferably of at most 70 amino acids, further preferably of at most 50 amino acids and most preferably of at most 30 amino acids.

Thus, in a further preferred embodiment of the present invention, the at least one of said amino acid sequence (I) is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently of each other selected from:
D at positions 1 and 19 are independently of each other optionally exchanged by D-aspartate;
amino acids at positions 5, 6, 7, 8, 9, 11, 13, 17 and 18 are independently of each other optionally exchanged by any other D- or L-amino acid (mentioned herein as X5, X6, X7, X8, X9, X11, X13, X17 and X18), preferably D- or L-alanine or D- or L-arginine, or a deletion, wherein at most one of positions 5, 6, 7, 8, 9, 11, 13, 17 and 18 is optionally a deletion;
and wherein said peptide has a length of at most 160 amino acids, preferably of at most 100 amino acids, further preferably of at most 80 amino acids, further preferably of at most 70 amino acids, further preferably of at most 50 amino acids and most preferably of at most 30 amino acids.

Thus, in again a further preferred embodiment of the present invention, the at least one of said amino acid sequence (I) is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently of each other selected from:
amino acids at positions 5, 6, 7, 8, 9, 11, 13, 17 and 18 are independently of each other optionally exchanged by any other D- or L-amino acid (mentioned herein as X5, X6, X7, X8, X9, X11, X13, X17 and X18), preferably D- or L-alanine or D- or L-arginine, or a deletion, wherein at most one of positions 5, 6, 7, 8, 9, 11, 13, 17 and 18 is optionally a deletion;
and wherein said peptide has a length of at most 160 amino acids, preferably of at most 100 amino acids, further preferably of at most 80 amino acids, further preferably of at most 70 amino acids, further preferably of at most 50 amino acids and most preferably of at most 30 amino acids.

Thus, in again a further preferred embodiment of the present invention, the at least one of said amino acid sequence (I) is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently of each other selected from:
amino acids at positions 5, 6, 7, 8, 9, 11, 13, 17 and 18 are independently of each other optionally exchanged by any other D- or L-amino acid (mentioned herein as X5, X6, X7, X8, X9, X11, X13, X17 and X18), preferably D- or L-alanine or D- or L-arginine, or a deletion, wherein at most one of positions 5, 6, 7, 8, 9, 11, 13, 17 and 18 is optionally a deletion;
and wherein said peptide has a length of at most 100 amino acids, further preferably of at most 80 amino acids, further preferably of at most 70 amino acids, further preferably of at most 50 amino acids and most preferably of at most 30 amino acids.

In again a further preferred embodiment of the present invention, the at least one of said amino acid sequence (I) is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently of each other selected from:
amino acids at positions 5, 6, 7, 8, 9, 11, 13, 17 and 18 are independently of each other optionally exchanged by any other D- or L-amino acid (mentioned herein as X5, X6, X7, X8, X9, X11, X13, X17 and X18), preferably D- or L-alanine or D- or L-arginine, or a deletion, wherein at most one of positions 5, 6, 7, 8, 9, 11, 13, 17 and 18 is optionally a deletion;
and wherein said peptide has a length of 80 amino acids, further preferably of at most 70 amino acids, further preferably of at most 50 amino acids and most preferably of at most 30 amino acids.

In again a further preferred embodiment of the present invention, the at least one of said amino acid sequence (I) is the amino acid sequence of DRRRFNSADYKGPRRRKAD (SEQ ID NO: 52) or an amino acid sequence, wherein up to 4 amino acids, again further preferably up to 3, again further preferably up to 2, and again further preferably one amino acid of SEQ ID NO: 52 is/are exchanged, and wherein the up to 6 amino acid exchanges are independently of each other selected from:
amino acids at positions 5, 6, 7, 8, 9, 11, 13, 17 and 18 are independently of each other optionally exchanged by any other D- or L-amino acid (mentioned herein as X5, X6, X7, X8, X9, X11, X13, X17 and X18), preferably D- or L-alanine or D- or L-arginine, or a deletion, wherein at most one of positions 5, 6, 7, 8, 9, 11, 13, 17 and 18 is optionally a deletion;
and wherein said peptide has a length of 80 amino acids, further preferably of at most 70 amino acids, further preferably of at most 50 amino acids and most preferably of at most 30 amino acids.

In a further preferred embodiment, said amino acid sequence (I) comprises at most 14, preferably at most 12, more preferably at most 2 to 7, more preferably at most 4, and again more preferably at most 2 amino acids, which are D- or L-alanine, more preferably L-alanine.

In another preferred embodiment, at least one of X2, X3, X4, X5, X6, X7, X8, X9, X11, X13, X17 or X18 is independently of each other D- or L-alanine, preferably L-alanine. In another preferred embodiment, at least one of X4 to X9, X11, X13, X17 or X18 is independently of each other D- or L-alanine, preferably L-alanine. More preferably at least one of X4, X5, X7, X8, X11, X13 or X18 is independently of each other D- or L-alanine, preferably L-alanine. Peptides having alanine at these positions are particularly stable against proteolysis. Even more preferably, at least one of X5 to X9, X11, X13, X17 or X18 is independently of each other D- or L-alanine, preferably L-alanine; and again more preferably at least one of X5, X8, X9 or X17 is independently of each other D- or L-alanine, preferably L-alanine. Peptides having alanine at these positions bind with a very high affinity to c-di-GMP.

In a preferred embodiment, said amino acid sequence (I) comprises at most 15, more preferably at most 12, more preferably at most 10, more preferably at most 8, and even more preferably at most six amino acids, which are D- or L-arginine, most preferably L-arginine.

In a preferred embodiment, X2 and X3 are independently of each other D- or L-arginine, preferably L-arginine; and preferably X2, X3 and X4 are independently of each other D- or L-arginine, preferably L-arginine.

In a more preferred embodiment, X2, X3, X4, R14, R15 and R16 are independently of each other D- or L-arginine, preferably L-arginine. In an even more preferred embodiment, X2, X3, X4, R14, R15 and R16 are independently of each other D- or L-arginine, preferably L-arginine, and X8 and X18, again more preferably X5, X8 and X18 are independently of each other D- or L-alanine, preferably L-alanine. In an again more preferred embodiment, X2, X3, X4, R14, R15 and R16 are independently of each other D- or L-arginine, preferably L-arginine, and X8, X9 and X18 are independently of each other D- or L-alanine, preferably L-alanine. In another preferred embodiment, X2, X3, X4, R14, R15 and R16 are independently of each other D- or L-arginine, preferably L-arginine, X8 is D- or L-lysine, preferably L-lysine, and X18 is D- or L-alanine, preferably L-alanine.

The peptide of the invention can include one or more deletions. In the embodiments according to the invention, X2, X3, X4, X5, X6, X7, X8, X9, X11, X13, X17 and X18 are independently of each other an amino acid or a deletion, wherein at most one of X2, X3, X4, X5, X6, X7, X8, X9, X11, X13, X17 or X18 is a deletion.

In one embodiment, D1 and D19 are independently of each other an amino acid as defined herein, and X2, X3, X4, X5, X6, X7, X8, X9, X11, X13, X17 and X18 of the peptide of the invention are independently of each other a deletion, wherein at most one of X2, X3, X4, X5, X6, X7, X8, X9, X11, X13, X17 or X18 is a deletion. Preferably D1, D19, X2, X3, X11, X13 and X17 are independently of each other an amino acid as defined herein, and X4, X5, X6, X7, X8, X9 and X18 are independently of each other a deletion, wherein at most one of X4, X5, X6, X7, X8, X9 or X18 is a deletion. In a more preferred embodiment, D1 and D19 are independently of each other an amino acid as defined herein, and X2, X3, X4, X5, X6 X11, X13, X17 and X18 of the peptide of the invention are independently of each other an amino acid as defined herein, and X7 or X8 or X9 is independently of each other a deletion, wherein at most one of X7 or X8 or X9 is independently of each other a deletion. A deletion at position X7 or X8 or X9 has been shown to increase binding affinity of the peptide of the invention to the target c-di-GMP.

In another embodiment, D1 is an amino acid as defined herein, and D19, X2, X3, X4, X5, X6, X7, X8, X9, X11, X13, X17 and X18 of the peptide of the invention are independently of each other a deletion, wherein at most one of X2, X3, X4, X5, X6, X7, X8, X9, X11, X13, X17 or X18 is a deletion. Preferably D1, X2, X3, X11, X13 and X17 are independently of each other an amino acid as defined herein, and D19, X4, X5, X6, X7, X8, X9 and X18 are independently of each other a deletion, wherein at most one of X4, X5, X6, X7, X8, X9 or X18 is a deletion. In a more preferred embodiment, D1, X2, X3, X4, X5, X6 X11, X13, X17 and X18 of the peptide of the invention are independently of each other an amino acid as defined herein, and D19 and X7 or X8 or X9 are independently of each other a deletion, wherein at most one of X7 or X8 or X9 is a deletion.

In another preferred embodiment, D19 is an amino acid as defined herein, and D1, X2, X3, X4, X5, X6, X7, X8, X9, X11, X13, X17 and X18 of the peptide of the invention are independently of each other a deletion, wherein at most one of X2, X3, X4, X5, X6, X7, X8, X9, X11, X13, X17 or X18 is a deletion. Preferably D19, X2, X3, X11, X13 and X17 are independently of each other an amino acid as defined herein, and D1, X4, X5, X6, X7, X8, X9 and X18 are independently of each other a deletion, wherein at most one of X4, X5, X6, X7, X8, X9 or X18 is a deletion. In a more preferred embodiment, D19, X2, X3, X4, X5, X6 X11, X13, X17 and X18 of the peptide of the invention are independently of each other an amino acid as defined herein, and D1 and X7 or X8 or X9 are independently of each other a deletion, wherein at most one of X7 or X8 or X9 is a deletion.

In another preferred embodiment, D1, D19, X2, X3, X4, X5, X6, X7, X8, X9, X11, X13, X17 and X18 of the peptide of the invention are independently of each other a deletion, wherein at most one of X2, X3, X4, X5, X6, X7, X8, X9, X11, X13, X17 or X18 is a deletion. Preferably X2, X3, X11, X13 and X17 are independently of each other an amino acid as defined herein, and D1, D19, X4, X5, X6, X7, X8, X9 and X18 are independently of each other a deletion, wherein at most one of X4, X5, X6, X7, X8, X9 or X18 is a deletion. In a more preferred embodiment, X2, X3, X4, X5, X6 X11, X13, X17 and X18 of the peptide of the invention are independently of each other an amino acid as defined herein, and D1, D19 and X7 or X8 or X9 are independently of each other a deletion, wherein at most one of X7 or X8 or X9 is a deletion.

In a preferred embodiment, at least one of said amino acid sequence (I) is an amino acid sequence selected from SEQ ID NO: 1 to 7, 9, 10, 12 to 21, 23, 25, 29 to 37, 39 to 43, 46 to 52 or 60 to 87. In a further preferred embodiment, at least one of said amino acid sequence (I) is an amino acid sequence selected from SEQ ID NO: 1 to 7, 12, 13, 17 to 21, 23, 29, 30, 43, 48, 50 to 52, 60, 64 to 68, 69, 77, 78, 84, 86 or 87.

In a further preferred embodiment, said amino acid sequence (I) is an amino acid sequence selected from SEQ ID NO: 1 to 7, 9, 10, 12 to 21, 23, 25, 29 to 37, 39 to 43, 46 to 52 or 60 to 87. In again a further preferred embodiment, said amino acid sequence (I) is an amino acid sequence selected from SEQ ID NO: 1 to 7, 12, 13, 17 to 21, 23, 29, 30, 43, 48, 50 to 52, 60, 64 to 68, 69, 77, 78, 84, 86 or 87.

In another embodiment, the peptide of the invention comprises N amino acid sequences (I), wherein N is an integer of ≥ 1. The N amino acid sequences (I) form an N-mer, i.e. two, three or more amino acid sequences (I) included in the peptide of the invention form a dimer, trimer, oligomer or polymer. The amino acid sequences (I) can be coupled directly to each other to form an N-mer, or the amino acid sequences (I) can be coupled to at least one intermediate amino acid, at least one intermediate amino acid sequence or at least one linker, wherein preferably the linker comprises or more preferably consist of at least one amino acid.

In another embodiment, said peptide comprises at least two of said amino acid sequence (I), and wherein preferably said amino acid sequence (I) is an amino acid sequence selected from SEQ ID NO: 1 to 7, 9, 10, 12 to 21, 23, 25, 29 to 37, 39 to 43, 46 to 52 or 60 to 87 and wherein further preferably said amino acid sequence (I) is an amino acid sequence selected from SEQ ID NO: 1 to 7, 12, 13, 17 to 21, 23, 29, 30, 43, 48, 50 to 52, 60, 64 to 68, 69, 77, 78, 84, 86 or 87.

Thus, the present invention provides a peptide comprising at least one, preferably exactly one of an amino acid sequence selected from SEQ ID NO: 1 to 7, 9, 10, 12 to 21, 23, 25, 29 to 37, 39 to 43, 46 to 52 or 60 to 87, wherein said peptide has a length of at most 160 amino acids, preferably of at most 100 amino acids, further preferably of at most 80 amino acids, further preferably of at most 70 amino acids, further preferably of at most 50 amino acids and most preferably of at most 30 amino acids. In a preferred embodiment, the peptide of the invention consists of an amino acid sequence selected from SEQ ID NO: 1 to 7, 9, 10, 12 to 21, 23, 25, 29 to 37, 39 to 43, 46 to 52 or 60 to 87.

In a certain embodiment, the peptide according to the invention comprises (i) an N-terminus which is selected from a free amine group or a modified N-terminus; wherein said modified N-terminus is preferred; said modified N-terminus includes a modification selected from (a) acetylation of said N-terminus of the peptide; (b) addition of at least one, preferably exactly one N-terminal compound to said N-terminus of the peptide; (c) addition of at least one, preferably exactly one N-terminal compound to said N-terminus of the peptide and acetylation of an N-terminus of said N-terminal compound; (d) addition of at least one, preferably exactly one N-terminal D-amino acid to said N-terminus of the peptide; or (e) addition of at least one, preferably exactly one N-terminal D-amino acid to said N-terminus of the peptide and acetylation of an N-terminus of said D-amino acid; wherein option (d) is preferred; and (ii) a C-terminus which is selected from a free carboxyl group or a modified C-terminus, wherein said modified C-terminus is preferred; said modified C-terminus includes a modification selected from (a') amidation of said C-terminus of the peptide; (b') addition of at least one, preferably exactly one C-terminal compound to said C-terminus of the peptide; (c') addition of at least one, preferably exactly one C-terminal compound to said C-terminus of the peptide and amidation of the C-terminus of said C-terminal compound; (d') addition of at least one, preferably exactly one C-terminal D-amino acid to said C-terminus of the peptide; or (e') addition of at least one, preferably exactly one C-terminal D-amino acid to said C-terminus of the peptide and amidation of the C-terminus of said D-amino acid, wherein option (e') is preferred.

In a certain embodiment, the peptide of the invention comprises an amidated C-terminus and/or an acetylated N-terminus, preferably an amidated C-terminus and an acetylated N-terminus. In another embodiment, the C-terminus is a D-amino acid and/or the N-terminus is a D-amino acid, preferably the C-terminus and N-terminus are independently of each other a D-amino acid. In a further embodiment, the C-terminus of the peptide is a D-amino acid with an amidated C-terminus. In another embodiment, the N-terminus is a D-amino acid with an acetylated N-terminus. Preferably the C-terminus of the peptide is a D-amino acid with an amidated C-terminus, and the N-terminus is a D-amino acid with an acetylated N-terminus. In another embodiment, the C-terminus and N-terminus are independently of each other a D-amino acid, and the N-terminal D-amino acid is acetylated. In a most preferred embodiment, the C-terminus and N-terminus are independently of each other a D-amino acid, and the C-terminal D-amino acid is amidated. N- and C-terminal modifications of latter embodiment specifically increase stability of the peptide of the invention to a significant extend.

In a preferred embodiment, at least one additional D- or L-amino acid, more preferably at least one additional D-amino acid is independently of each other attached to D1 and/or D19, more preferably to D1 and D19 of the peptide of the invention. In another preferred embodiment, exactly one additional D- or L-amino acid, more preferably exactly one additional D-amino acid is independently of each other attached to D1 and/or D19, more preferably D1 and D19. In another preferred embodiment, the N-terminal of D1 is acetylated and/or the C-terminal of D 19 is amidated, more preferably the N-terminal of D1 is acetylated and the C-terminal of D19 is amidated. In a more preferred embodiment, at least one, preferably exactly one additional D-amino acid with an acetylated N-terminus is attached to D1, or at least one, preferably exactly one additional D-amino acid with an amidated C-terminus is attached to D 19. In an again more preferred embodiment, at least one, preferably exactly one additional D-amino acid with an acetylated N-terminus is attached to D1, and at least one, preferably exactly one additional D-amino acid with an amidated C-terminus is attached to D19. In another embodiment, at least one, preferably exactly one additional D-amino acid with an acetylated N-terminus is attached to D1, and at least one, preferably exactly one additional D-amino acid is attached to D19. In a most preferred embodiment, at least one, preferably exactly one additional D-amino acid is attached to D1, and at least one, preferably exactly one additional D-amino acid with an amidated terminus is attached to D 19.

In a certain embodiment of the invention, the peptide according to the invention comprises at least one, preferably exactly one, cell penetrating peptide. The cell penetrating peptide can be added to any of the peptide termini or internal amino acid residues of the peptide of the invention. Preferably, the at least one, preferably exactly one cell penetrating peptide is added to the N- and/or C-terminus of the peptide of the invention. In a preferred embodiment, at least one, preferably exactly one N-terminal compound is added to the N-terminus of the peptide of the invention, wherein said N-terminal compound is a cell penetrating peptide. In another embodiment, at least one, preferably exactly one C-terminal compound is added to the C-terminus of the peptide of the invention, wherein said C-terminal compound is a cell penetrating peptide. In another embodiment, the peptide of the invention comprises an N-terminal compound and a C-terminal compound which are both independently of each other a cell penetrating peptide. In a most preferred embodiment, the C-terminal compound or N-terminal compound, preferably the N-terminal compound of the peptide of the invention is a cell penetrating peptide, wherein said cell penetrating peptide is preferably polyarginine.

Preferred cell penetrating peptides are trans-activating transcriptional activator (TAT) peptides, such as TAT HIV peptides, or more preferably polyarginine, such as hexa-, hepta-, octa- or nona-arginine. In a most preferred embodiment, the cell penetrating peptide is octa-arginine.

In another preferred embodiment, said C-terminal compound or said N-terminal compound, preferably said N-terminal compound, is a cell penetrating peptide, wherein said cell penetrating peptide is selected from a polyarginine or a trans-activating transcriptional activator peptide (TAT).

In another preferred embodiment, the cell penetrating peptide has the amino acid sequence YGRKKRRQRRR (SEQ ID NO: 53).

In a particularly preferred embodiment, the peptide of the invention comprises at least one, preferably exactly one cell penetrating peptide, which is preferably polyarginine, said cell penetrating peptide is attached to the N-terminus of the peptide of the invention, wherein the C-terminus of the peptide of the invention is amidated. In a further embodiment, the peptide of the invention comprises at least one, preferably exactly one cell penetrating peptide, which is preferably polyarginine, said cell penetrating peptide is attached to the C-terminus or N-terminus or to the C- and N-terminus of the peptides of SEQ ID NO: 4, 12 and 39 to 42 in order to facilitate cellular internalization of these peptides.

In a certain embodiment, the peptide according to the invention comprises a linker for attaching at least one additional compound or sequence to the peptide of the invention, wherein preferably the linker is preferably connected to the N- or C-terminal of the peptide. A preferred linker comprises at least one or more amino acids. A preferred additional compound added to the peptide of the invention via the linker is the at least one cell penetrating peptide, preferably polyarginine, preferably added to the N- and/or C-terminal of the peptide of the invention.

In a further embodiment, the peptide of the invention binds c-di-GMP with a dissociation constant of 5 µM or lower, preferably 4 µM or lower, more preferably 3 µM or lower, even more preferably 2 µM or lower, again more preferably 1.5 µM or lower, again more preferably 1.4 µM or lower, again more preferably 1.3 µM or lower, again more preferably 1.2 µM or lower, again more preferably 1.1 µM or lower, again more preferably 1.0 µM or lower, again more preferably 0.5 µM or lower, or most preferably 0.2 µM or lower.

In another embodiment, the peptide of the invention binds c-di-GMP with a dissociation constant of 1.3 or lower, preferably 1.25 µM or lower, more preferably 1.2 µM or lower, or most preferably 1.15 µM or lower. In a preferred embodiment, the dissociation constant of the peptide for c-di-GMP is 1.1 µM or lower, preferably 1.05 µM or lower or more preferably 1.04 µM or lower. In a further preferred embodiment, the dissociation constant of the peptide for c-di-GMP is 1.0 µM or lower, preferably 0.95 µM or lower, more preferably 0.9 µM or lower, 0.85 µM or lower, 0.8 µM or lower, 0.75 µM or lower, most preferably 0.7 µM or lower, 0.65 µM or lower, 0.6 µM or lower, or 0.55 µM or lower. In a more preferred embodiment, the dissociation constant is 0.5 µM or lower, 0.45 µM or lower, preferably 0.4 µM or lower, 0.35 µM or lower, more preferably 0.3 µM or lower, most preferably 0.25 µM or lower. In a most preferred embodiment, the dissociation constant is 0.2 µM or lower, 0.15 µM or lower, 0.14 µM or lower, preferably 0.13 µM or lower, 0.12 µM or lower, more preferably 0.11 µM or lower, most preferably 0.10 µM or lower.

In a further embodiment, the concentration of the peptide of the invention leading to 50% biofilm reduction (MBIC50) is from 0.1 mg/L to 0.9 mg/L, preferably from 0.25 mg/L to 0.5 mg/L, more preferably less than 0.25 mg/L.

In a another embodiment of the invention, the concentration of the peptide of the invention leading to 50% biofilm reduction (MBIC50) is from 0.1 mg/L to 0.5 mg/L, preferably from 0.25 mg/L or less to 0.5 mg/L, more preferably from 0.25 mg/L or less to 0.4 mg/L, and again more preferably from 0.25 mg/L or less to 0.3 mg/L. Even more preferably the MBIC50 is 0.2 mg/L or less, again more preferably 0.15 mg/L, or most preferably 0.1 mg/L or less.

MBIC50 in accordance with the invention is quantified by a standard biofilm assay. In a certain embodiment, the medium used for detecting the MBIC50 value is Mueller-Hinton broth (MHB), preferably Mueller-Hinton broth type II. In another embodiment, the MBIC50 values were obtained by growing *P. aeruginosa.* According to a further embodiment, *P*. *aeruginosa* is grown in Mueller-Hinton broth (MHB), more preferably, in Mueller-Hinton broth type II.

Another aspect of the invention relates to a composition comprising or preferably consisting of:
(1) at least one peptide according to the invention; and
(2) at least one antimicrobial agent or at least one anti-infective or a combination of at least one antimicrobial agent and at least one anti-infective.

The antimicrobial agent may be selected from an antiseptic, disinfectant or a combination of both. In a preferred embodiment, the antimicrobial agent is a disinfectant, preferably alcohol, hydrochloric acid, oxidizing agent, silver dihydrogen citrate, silver sulphadiazine or copper alloys or combinations thereof.

The anti-infective may be selected from an antiviral agent, antimycotic, antiprotozoal agent and antibiotic. In a more preferred embodiment, the anti-infective is an antimycotic or antibiotic. The antibiotic is the most preferred anti-infective. The composition including at least one peptide of the invention and an antibiotic or antimycotic or both is used for example for laboratory purposes in culture mediums, in animal husbandry, bee-keeping, fish farming and other forms of aquaculture, ethanol production, horticulture, antifouling paints, food preservation and domestically.

The compositions of the invention may contain 0.1-99 %, preferably 50 %, more preferably 10 % by weight of the peptide of the invention.

In another aspect, the invention relates to a pharmaceutical composition comprising of at least one peptide according to the invention.

In a preferred embodiment, the pharmaceutical composition includes one or more pharmaceutically active agents, such as an immunostimulating agent, antibody or vaccine, and optionally an adjuvant.

In a preferred embodiment, the pharmaceutical composition of the invention comprises one or more pharmaceutically active agents selected from an anti-infective, an antiseptic or a combination of at least one anti-infective and at least one antiseptic; wherein preferably said one or more pharmaceutically active agent is the anti-infective, wherein the anti-infective is an antibiotic; and wherein more preferably said antibiotic is effective in reducing *P*. *aeruginosa*.

In a preferred embodiment, the anti-infective is selected from an antibiotic, antiviral agent, antiprotozoal agent and antimycotic. The more preferred anti-infective is at least one antibiotic or at least one antimycotic or a combination of at least one antibiotic and at least one antimycotic. Most preferably, the anti-infective is at least one antibiotic.

In another preferred embodiment, the pharmaceutical composition comprises the peptide of the invention, an antibiotic and a further pharmaceutically active agent, preferably an immunostimulating agent.

Preferred antibiotics are at least effective against *P. aeruginosa* and *Enterobacteriaceae* including *E. coli, K. pneumonia and S. typhimurium*. Antibiotics are selected based on the Minimal Inhibitory Concentration (MIC) values. Preferred antibiotics have a MIC value of 4 mg/L or less, such as the class of cephalosporins (e.g. ceftolozane-tazobactam, ceftaroline, ceftobiprole), carbapenams (e.g. doripenam, doripenem, ertapenem), monobactams (e.g. aztreonam), fluoroquinolones (e.g. ciprofloxacin and levofloxacin), aminoglycosides (e.g. gentamycin, netilmicin and tobramycin) or tetracyclins (e.g. tigecycline). Less preferred are antibiotics having a MIC value of more than 4 mg/L, such as penicillins, colistin, chloramphenicol, fosfomycin or nifurantoin. More preferred antibiotics are meropenem, colistin and azithromycin, since they maintain their activity under conditions of reduced oxygen tension and low metabolic activity as found in deeper layers of biofilms. Preferably, different antibiotics are combined in the pharmaceutical composition of the invention. More preferably, the different antibiotics belong to different classes of antibiotics.

Moreover, the pharmaceutical composition of the invention may include enzymes that degrade the biofilm matrix. Preferred enzymes are DNases (e.g. dornase alfa), preferably used for treating chronic biofilm infections in patients with cystic fibrosis; glycoside hydrolase (e.g. dispersin B), since it hydrolyses the extracellular polysaccharide poly-N-acetylglucosamine, preferably of bacterial species, such as *E. coli, S. epidermidis* and *Pseudomonas fluorescens*; and alginate lyase, since it degrades alginate in mucoid biofilms. Moreover, the pharmaceutical composition of the invention may include bacteriophages, i.e. viruses that infect bacteria.

Furthermore, in a certain embodiment, inhibitors of bacterial efflux pump, such as thioridazine, 1-(1-naphthylmethyl)-piperazine and Phe-Arg-naphthylamide, or antioxidants, such as 1-proline, N-acetylcysteine, β-carotene or l-cysteine, or combinations thereof can be included in the pharmaceutical composition of the invention in order to enhance the anti-biofilm activity of the peptide of the invention.

The pharmaceutical composition of the invention can comprise at least one suitable pharmaceutically acceptable carrier. The pharmaceutically acceptable carrier includes but is not limited to as an excipient, vehicle, diluent, solvent, wetting, dispersion or suspension agent, surface active agent, isotonic agent, thickening or emulsifying agent, preservative, sweetening agents, favoring agents, coloring agents, encapsulating agent, solid binder or lubricant. The diluent or solvent includes one or more sterile aqueous (e.g., physiological saline) or non-aqueous solutions, emulsions and suspensions. Examples of non-aqueous diluents and solvents are propylene glycol, polyethylene glycol, vegetable oils, such as olive oil, and injectable organic esters, such as ethyl oleate.

The pharmaceutical composition of the invention may be in a form which contains salts, buffers, adjuvants or other substances which are desirable for improving the efficacy of the peptide of the invention. Examples of materials suitable for use in preparation of pharmaceutical compositions are provided in numerous sources including Remington's Pharmaceutical Sciences (Osol, ed. Mack Publishing Co., 1990).

In therapeutic use, the active compound may be administered, e.g., orally, rectally, intravaginally, parenterally, subcutaneously, intradermally, transdermally, sublingually, bucally, pulmonarily, by inhalation, topically, ocularly, intranasally, intralesionally, intraperitoneally, intranodally, intravenously, intramuscularly or to the interstitial space of a tissue.

The pharmaceutical composition may be formulated, e.g., in a tablet, capsule, lozenge, dragee, pill, droplet, granules, syrups, suppository, powder, spray, vaccine, ointment, paste, cream, gel, inhalant, patch, nano-particle, aerosol, aqueous or oily suspension, oil-in-water emulsions or a sustained release form. The patch may include an occlusive dressings which may increase skin permeability and enhance transfer or absorption of the peptide of the invention or other compounds included in the pharmaceutical composition of the invention into or through the skin.

The compositions of the invention may contain 0.1-99 %, preferably 50 %, more preferably 10 % by weight of the peptide of the invention. In one embodiment said pharmaceutical composition comprises an effective dose or amount of the peptide of the invention. An "effective amount" refers to an amount which needs to be administered to a subject in order to achieve a detectable physiological effect. For purposes of the present invention, an effective dose will be preferably from about 0.01 µg/kg to 50 mg/kg, more preferably 0.5 pg/kg to about 10 mg/kg of the peptide of the invention.

The composition of the invention is generally prepared in unit dosage form. Preferably, a unit dose comprises the peptide of the invention in an amount of 1-1.000 mg, more preferably, 1-500 mg, even more preferably, 1-100 mg. The administered amount of the peptide of the invention is selected with regard to the used formulation, administration route, pathogen causing the infection, severity of infection, weight, sex, general physical condition and age of the patient.

Another aspect of the invention relates to the peptide of the invention for use as a medicament in the treatment or prevention of a disease, wherein preferably said disease is a microbial, bacterial, fungal or protozoan infection.

In a particularly preferred embodiment said disease is a microbial infection, wherein said microbial infection is preferably associated with or caused by *Pseudomonas aeruginosa* in a patient having more preferably cystic fibrosis.

The peptide or pharmaceutical composition of the invention can be used in the medical, veterinary or dental field. Preferred infections to be treated or prevented by the peptide of the invention are caused by or associated with *Pseudomonas aeruginosa, Burkholderia cenocepacia, Mycobacterium tuberculosis, Klebsiella pneumonia. Vibrio cholerae, Salmonella Typhimurium, Salmonella typhi, uropathogenic* and *enterohemorrhagic Escherichia coli, Shigella dysenteriae, Yersinia pestis, Yersinia enterocolitica, Bacillus anthracis, Clostridium difficile, Enterobacter cloacae, Erwinia amylovora, Morganella morgami, Acinetobacter calcoaceticus, Agrobacterium tumefaciens, Francisella tularensis, Legionella pneumophila, Pseudomonas syringae, Rhizobium meliloti* and *Haemophilus influenzae*.

In a more preferred embodiment, an infection involving or caused by *Pseudomonas aeruginosa* is treated or prevented in a patient suffering from cystic fibrosis. The peptide or pharmaceutical composition of the invention is preferably used in chronic infections or clinical complications. Preferably, the peptide and pharmaceutical composition are used to treat infections related to biofilms, such as biofilm growth on medical implants, devices and catheters, chronic wound infections, ventilator-associated pneumonia and tuberculosis.

Patients to be treated by the peptide or pharmaceutical composition of the invention are humans and animals. Preferred animals include mammals; domestic and farm animals, including but not limited to guinea pig, rabbit, horse, donkey, camel, cow, sheep, goat, pig, chicken, duck, turkey, goose, parrot, canary bird, cat, dog, goldfish, farm fish, such as trout, pangasius, carp, koi, perch, catfish, salmon, turtle, tortoise, snake, or lizard; laboratory animals, including but not limited to rodents, such as mouse, rat and rabbit; and other vertebra, such as reptiles or preferably fish and birds. Preferred patients are humans and mammals.

The peptide and pharmaceutical composition of the invention can be used alone (i.e. as monotherapy or stand-alone therapy) or preferably in combination with one or more pharmaceutically active agents or pharmaceutical compositions. The pharmaceutically active agent is preferably a vaccine, optionally combined with an adjuvant. More preferably, the pharmaceutically active agent is an anti-infective, preferably an antibiotic or antimycotic or a combination of both. The pharmaceutically active agent, preferably the anti-infective, more preferably the antibiotic can be administered simultaneously (included in one common or more than one, preferably two separate formulations) or subsequent to the administration of the peptide of the invention.

In another aspect, the invention relates to the use of the peptide of the invention for preventing or reducing biofilm formation or for eradicating or reducing an existing biofilm.

In a preferred embodiment, the peptide of the invention is used as surface cleaning agent; as coating of (i) a medical device, (ii) a packing material or (iii) a storing material; as preservative; as additive of cosmetic; in drinking water treatment; for treating crop plants and in a competitive screening assay for identifying at least one c-di-GMP binding compound or at least one compound having anti-biofilm activity.

The peptide can be used alone or together with an additive. In a preferred embodiment, the additive is an antimicrobial agent, preferably a disinfectant for cleaning surfaces and interfaces. In another preferred embodiment, the peptide is used in combination with a preserving agent, preferably an antimicrobial preservative, such as lactic acid, or an antioxidant, such as ascorbic acid or tocopherol.

When used as coating of an implant, medical device, packing material, storing material or the like, the peptide of the invention can be combined with silver or silver alloys. Preferably the peptide of the invention can be combined with silver sulphadiazine, for example in wound dressings.

When used in a competitive screening assay for identifying at least one c-di-GMP binding compound, c-di-GMP is exposed to the peptide of the invention and subsequently or simultaneously to a compound to be tested. Then binding affinity of the compound to be tested is measured by detecting competitive displacement of the peptide of the invention, if present.

When used in a competitive screening assay for identifying a compound having anti-biofilm activity, MBIC50 values of the compound to be tested and the peptide of the invention are detected and compared.

In another aspect, the invention relates to a nucleic acid encoding a peptide of the invention. In a preferred embodiment, the nucleic acid of the invention comprises or preferably consists of at least one nucleic acid sequence selected from SEQ ID NO: 54 to 58. These are nonlimiting examples of nucleic acids, which encode peptides of SEQ ID NO: 1, 2, 4, 48 and 51 (cf. Table 1B). The nucleic acids encoding a peptide of the invention can also include degenerate codons.

In a preferred embodiment, the nucleic acid according to the invention is included in a host cell or a vector.

In a further aspect, the invention provides a peptide comprising or consisting of at least one amino acid sequence, wherein said amino acid sequence is selected from SEQ ID NO: 8, 11, 22, 24, 26 to 28 or 38.

### EXAMPLES

### MATERIALS AND METHODS

### Peptides

Lyophilized peptides with at least 95% purity, except for FITC-labeled peptides with at least 70% purity, were purchased from a company (ProteoGenix SAS, France). Peptides were dissolved in ultrapure water and the concentrations were determined using a NanoDrop (Thermo Scientific).

**Table 1A. Peptides utilized in this study. Modifications to peptides are indicated with abbreviations: "AC-" for acetylation, "-NH2" for amidation, small letters "d", "r", "a" for D-configurations and "FITC-" for fluorescein isothiocynate.**

| **SEQ ID NO:** | **Amino Acid Sequence** |
|---|---|
| 1 | SKPREWVEAVAYVGPDRRRFNSADYKGPRKRKADAS |
| 2 | SKPREWVEAVAYVGPDRRRFNSADYKGPRRRKADAS |
| 3 | WVEAVAYVGPDRRRFNSADYKGPRRRKAD |
| 4 | DRRRFNSADYKGPRRRKADW |
| 5 | WDRRRFNSADYKGPRRRKAD |
| 6 | YVGPDRRRFNSADYKGPRRRKAD |
| 7 | WVGPDRRRFNSADYKGPRRRKAD |
| 8 | RRRFNSADYKGPRRR |
| 9 | RRRFNSADYKGPRRRKAD |
| 10 | DRRRFNSADYKGPRRRK |
| 11 | DRRRFNSADYKGPRRR |
| 12 | AC-DRRRFNSADYKGPRRRKAD-NH₂ |
| 13 | AC-ARRRFNSADYKGPRRRKAD-NH₂ |
| 14 | AC-DARRFNSADYKGPRRRKAD-NH₂ |
| 15 | AC-DRARFNSADYKGPRRRKAD-NH₂ |
| 16 | AC-DRRAFNSADYKGPRRRKAD-NH₂ |
| 17 | AC-DRRRANSADYKGPRRRKAD-NH₂ |
| 18 | AC-DRRRFASADYKGPRRRKAD-NH₂ |
| 19 | AC-DRRRFNAADYKGPRRRKAD-NH₂ |
| 20 | AC-DRRRFNSLDYKGPRRRKAD-NH₂ |
| 21 | AC-DRRRFNSAAYKGPRRRKAD-NH₂ |
| 22 | AC-DRRRFNSADAKGPRRRKAD-NH₂ |
| 23 | AC-DRRRFNSADYAGPRRRKAD-NH₂ |
| 24 | AC-DRRRFNSADYKAPRRRKAD-NH₂ |
| 25 | AC-DRRRFNSADYKGARRRKAD-NH₂ |
| 26 | AC-DRRRFNSADYKGPARRKAD-NH₂ |
| 27 | AC-DRRRFNSADYKGPRARKAD-NH₂ |
| 28 | AC-DRRRFNSADYKGPRRAKAD-NH₂ |
| 29 | AC-DRRRFNSADYKGPRRRAAD-NH₂ |
| 30 | AC-DRRRFNSADYKGPRRRKLD-NH₂ |
| 31 | AC-DRRRFNSADYKGPRRRKAA-NH₂ |
| 32 | AC-DRRFNSADYKGPRRRKAD-NH₂ |
| 33 | AC-DRRRNSADYKGPRRRKAD-NH₂ |
| 34 | AC-DRRRFSADYKGPRRRKAD-NH₂ |
| 35 | AC-DRRRFNADYKGPRRRKAD-NH₂ |
| 36 | AC-DRRRFNSDYKGPRRRKAD-NH₂ |
| 37 | AC-DRRRFNSAYKGPRRRKAD-NH₂ |
| 38 | AC-DRRRFNSADYKPRRRKAD-NH₂ |
| 39 | dRRRFNSADYKGPRRRKAd |
| 40 | rDRRRFNSADYKGPRRRKADa |
| 41 | AC-rDRRRFNSADYKGPRRRKADa-NH₂ |
| 42 | rDRRRFNSADYKGPRRRKADa-NH₂ |
| 43 | FITC-DRRRFNSADYKGPRRRKAD-NH₂ |
| 44 | FITC-RRRRRRRRRFNSADYKGPRRRKAD-NH₂ |
| 45 | FITC-RRRRRRRRR-NH₂ |
| 46 | FITC-RRRRRRDRRRFNSADYKGPRRRKAD-NH₂ |
| 47 | FITC-RRRRRRRDRRRFNSADYKGPRRRKAD-NH₂ |
| 48 | FITC-RRRRRRRRDRRRFNSADYKGPRRRKAD-NH₂ |
| 49 | FITC-RRRRRRRRRDRRRFNSADYKGPRRRKAD-NH₂ |
| 50 | FITC-YGRKKRRQRRRGRRRFNSADYKGPRRRKAD-NH₂ |
| 51 | FITC-YGRKKRRQRRRDGRRRFNSADYKGPRRRKAD-NH₂ |
| 52 | DRRRFNSADYKGPRRRKAD |
| 53 | YGRKKRRQRRR |
| 60 | ARRRFNSADYKGPRRRKAD |
| 61 | DARRFNSADYKGPRRRKAD |
| 62 | DRARFNSADYKGPRRRKAD |
| 63 | DRRAFNSADYKGPRRRKAD |
| 64 | DRRRANSADYKGPRRRKAD |
| 65 | DRRRFASADYKGPRRRKAD |
| 66 | DRRRFNAADYKGPRRRKAD |
| 67 | DRRRFNSLDYKGPRRRKAD |
| 68 | DRRRFNSAAYKGPRRRKAD |
| 69 | DRRRFNSADYAGPRRRKAD |
| 70 | DRRRFNSADYKGARRRKAD |
| 71 | DRRFNSADYKGPRRRKAD |
| 72 | DRRRNSADYKGPRRRKAD |
| 73 | DRRRFSADYKGPRRRKAD |
| 74 | DRRRFNADYKGPRRRKAD |
| 75 | DRRRFNSDYKGPRRRKAD |
| 76 | DRRRFNSAYKGPRRRKAD |
| 77 | DRRRFNSADYKGPRRRAAD |
| 78 | DRRRFNSADYKGPRRRKLD |
| 79 | DRRRFNSADYKGPRRRKAA |
| 80 | RDRRRFNSADYKGPRRRKADA |
| 81 | RRRRRRRRRFNSADYKGPRRRKAD |
| 82 | RRRRRRDRRRFNSADYKGPRRRKAD |
| 83 | RRRRRRRDRRRFNSADYKGPRRRKAD |
| 84 | RRRRRRRRDRRRFNSADYKGPRRRKAD |
| 85 | RRRRRRRRRDRRRFNSADYKGPRRRKAD |
| 86 | YGRKKRRQRRRGRRRFNSADYKGPRRRKAD |
| 87 | YGRKKRRQRRRDGRRRFNSADYKGPRRRKAD |

**Table 1B. Exemplary nucleic acids of the invention**

| **SEQ ID NO:** | **SEQ ID NO of the encoded peptide** | **Encoding nucleic acid sequence** |
|---|---|---|
| 54 | 1 | AGTAAGCCGCGCGAATGGGTCGAGGCCGTCGCCTATGT CGGGCCTGATCGCCGTCGTTTCAACTCGGCGGACTATA AGGGGCCGCGCAAGCGCAAGGCGGACGCCAGC |
| 55 | 2 | AGTAAGCCGCGCGAATGGGTCGAGGCCGTCGCCTATGT CGGGCCTGATCGCCGTCGTTTCAACTCGGCGGACTATA AGGGGCCGCGCAGGCGCAAGGCGGACGCCAGC |
| 56 | 4 | GATCGCCGTCGTTTCAACTCGGCGGACTATAAGGGGCC GCGCAGGCGCAAGGCGGAC |
| 57 | 48 | CGTAGACGACGCCGTCGGCGTAGGGATCGCCGTCGTTT CAACTCGGCGGACTATAAGGGGCCGCGCAGGCGCAAG GCGGAC |
| 58 | 51 | TATGGCAGGAAGAAGCGGAGACAGCGACGAAGAGATG GTCGCCGTCGTTTCAACTCGGCGGACTATAAGGGGCCG CGCAGGCGCAAGGCGGAC |

C-di-GMP (also called cyclic di-GMP, cyclic diguanylate), c-di-AMP, pGpG, 2'3'-cGAMP and 3'3'-cGAMP were purchased from BIOLOG (Germany). GTP and GMP were bought from Sigma-Aldrich (Switzerland).

### Bacteria

In this invention, bacteria of the strain *P. aeruginosa* PAO1 were used. They originated from the study of Holloway B.W., 1955 (Genetic recombination in Pseudomonas aeruginosa. J Gen Microbiol. 13: 572-581).

### Binding Affinity Determination

Binding affinities of peptides to c-di-GMP were measured using isothermal titration calorimetry (ITC) on a VP-ITC MicroCalorimeter (MicroCal, Northampton, MA, USA). Peptides, c-di-GMP and buffer (50 mM Tris-HCl, pH 8.0, 100 mM NaCl) were degassed for 15 min prior to filling into sample cell and syringe. All experiments were carried out at a temperature of 15°C. The data were evaluated using ITC Data Analysis in ORIGIN provided by the manufacturer.

To determine if the peptide is specific to c-di-GMP, the binding of fluorescein isothiocyante (FITC)-labeled peptide (SEQ ID NO: 43) to related nucleotides like GMP, GTP, c-di-AMP, pGpG, 2'3'-cGAMP and 3'3'-cGAMP was measured. Experiments were carried out using microscale thermophoresis (MST; NanoTemper Technologies, Germany) in a Monolith NT.115 instrument with standard-treated capillaries. Fluorescence changes were recorded using blue channel optics of the instrument for a 30 s period of infrared laser heating at 50% of maximum laser power followed by a cooling period of 5 s. Measurements were performed in a buffer containing 50 mM Tris-HCl, pH 8.0, 100 mM NaCl, and 0.1% Tween 20. Data were analyzed with the program provided by the manufacturer.

### Stability against bacterial proteases

*P. aeruginosa* PAO1 lysate, which was used to test peptide stability, was prepared by the following procedure. 500 ml of cells grown overnight at 37°C were harvested by centrifugation at 5000 rpm, 10 min at 4°C. Pellets were resuspended in 60 ml PBS (Gibco) and centrifuged at 5000 rpm, 10 min at 4°C. Pellets were resuspended again in 20 ml PBS and centrifuged at 5000 rpm, 10 min at 4°C. Cells were disrupted using high-pressure cell disrupter (Stansted Fluid Power). Cell lysate was separated from cell debris by centrifugation at 14,000 rpm for 20 min at 4°C. Lysate was diluted 10 fold to yield a protein content of about 1 mg/ml. The lysate was used for all experiments described below.

The stability of peptides was tested against *P. aeruginosa* PAO1 cell lysate. About 1 to 3 µg of peptides were incubated with 5 µl *P. aeruginosa* PAO1 cell lysate or PBS (untreated controls) at 37°C. After 2 hours, 6 µl of 2x SDS sample buffer was added to the samples and heated to 95°C for 5 min. Samples were analyzed on 10-20% Mini-PROTEAN tris-tricine gels (BIO-RAD #4563115) according to manufacturer's recommended protocol. Band intensities were quantified using the program ImageJ (Schneider, C. A., Rasband, W. S. and Eliceiri, K. W. 2012. NIH Image to ImageJ: 25 years of image analysis. Nature methods. 9: 671-675).

The protocol mentioned above was also used to compare stability of peptides in the presence of c-di-GMP. Peptides SEQ ID NO: 12 and 28 are high and low c-di-GMP binders, respectively. 12 µl of 1 mM peptide solution was pre-mixed with an equimolar solution of c-di-GMP and then added to 60 µl *P. aeruginosa* cell lysate. 14 µl samples were removed at 2, 4, 6 and 8 hours after incubation at 37°C and analyzed on tris-tricine gels as described above.

### Cell penetration - FACS based assay

Flow cytometry was applied to identify peptides with improved ability to penetrate into *P*. *aeruginosa* PAO1. *P. aeruginosa* PAO1 cultures were treated with 10 mg/L fluorescein isothiocynate (FITC)-labeled peptides for 30 min at 37°C on a shaker (170 rpm) in the dark. Afterwards, cells were washed with PBS to remove non-penetrated or excess peptides. Samples were analyzed with a Canto II (BD Biosciences), excitation 488nm, filters 530/30 and 502LP. For each condition, the median FITC intensity value of a population of approximately 5x10⁴ cells was used for quantification.

### Fluorescence microscopy

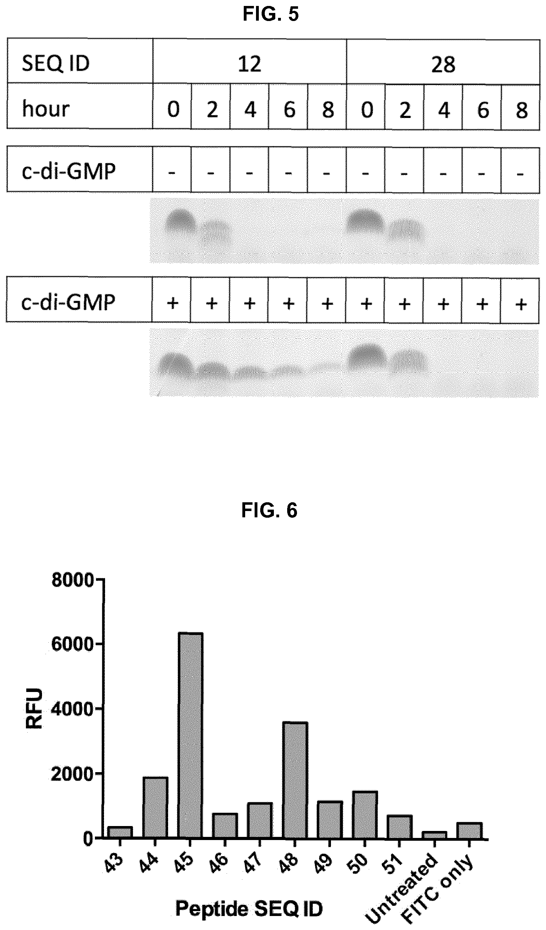

Fluorescence microscopy was applied to analyze the localization of FITC-labeled peptides that have been shown to associate with *P. aeruginosa* PAO1 by FACS. *P. aeruginosa* PAO1 cultures were treated with 10 mg/L fluorescein isothiocynate (FITC)-labeled peptides for 30 min at 37°C, on a shaker (170 rpm) in the dark. Afterwards, cells were washed with Mueller-Hinton II broth to remove non-penetrated or excess peptides. Samples were spotted on predried LB 1% agar plates and covered with a glass cover slip. Images were acquired on an inverted motorized Nikon Eclipse Ti-E microscope with at 100x objective lens using the settings as previously described (Kudryashev M, Wang RY, Brackmann M, Scherer S, Maier T, Baker D, DiMaio F, Stahlberg H, Egelman EH, Basler M. 2015. Structure of the type VI secretion system contractile sheath. Cell. 160:952-62.) VisiView software (Visin Systems, Germany) was used to record images and program Fiji (Schindelin J, Arganda-Carreras I, Frise E, Kaynig V, Longair M, Pietzsch T, Preibisch S, Rueden C, Saalfeld S, Schmid B, Tinevez JY, White DJ, Hartenstein V, Eliceiri K, Tomancak P, Cardona A. 2012. Fiji: an open-source platform for biological-image analysis. Nat. Methods. 9:676-82) was used for image analysis and manipulations. To increase visibility, contrast settings were adjusted for each image individually. To compare signal intensities among different conditions, reference is made to the FACS data (Figure 6).

### P. aeruginosa membrane and cytoplasmic fractionation

Overnight culture of *P. aeruginosa* PAO1 was 10-fold diluted with Mueller-Hinton broth. FITC-labeled peptides were added to cells to a final concentration of 10 mg/L. Samples were incubated for 30 min at 37°C, on a shaker (170 rpm) in the dark. Cells were collected by centrifugation at 8000 x g for 1 min. Cell pellets were washed with 10 ml PBS and centrifuged again like before. Cell pellets were reconstituted in 5 ml PBS containing protease inhibitor cocktail (Roche) and DNase I (Roche). Cells were disrupted using high-pressure cell disrupter (Stansted Fluid Power) and subjected to centrifugation at 9000 x g for 10 min at 4°C to remove intact cells and cell debris. To separate cytoplasmic content from membrane, supernatant (input fraction) from previous step was centrifuged at 100,000 x g for 60 min at 4°C. After centrifugation, supernatant (cytoplasmic fraction) was carefully removed and the pellet (membrane fraction) was resuspended in an equal amount PBS. Fluorescence signals were measured using a microtiter plate reader (Synergy H4, BIOTEK) with an excitation wavelength of 490 nm and an emission wavelength 520 nm.

### Minimal Biofilm Inhibitory Concentration (MBIC) Determination.

The standard biofilm assay (O'Toole G.A., Pratt L.A., Watnick P.I., Newman D.K., Weaver V.B., and Kolter R. 1999. Genetic approaches to study of biofilms. Methods Enzymol. 310: 91-109) was followed with minor modification, i.e. Mueller-Hinton II broth (MHB) (Becton Dickinson #212322) was used instead of lysogenic broth (LB) or minimal medium. MHB is preferred by The European Committee on Antimicrobial Susceptibility Testing (EUCAST) as the medium for MIC determination because most bacteria can be grown in this medium, which allows a more reliable MIC values comparisons. The MBIC₅₀ was defined as the peptide concentration leading to 50% biofilm reduction (de la Fuente-Nunez C., Korolik V., Bains M., Nguyen U., Breidenstein E. B. M., Horsman S., Lewenza S., Burrows L., Hancock, R. E. W. (2012) Inhibition of bacterial biofilm formation and swarming motility by a small synthetic cationic peptide. 56: 2696-2704). MBIC₅₀ values were obtained by growing *P*. *aeruginosa* (PAO1) biofilms in 96-well plates (Falcon #353072) in presence of peptides at 37°C without agitation in MHB. After 24 hours, bacterial growth was evaluated by measuring the OD600 in a microtiter plate reader (EL800, BioTek). Afterwards, the plates were rinsed three times with demineralized water to remove non-attached bacteria. The adherent biofilms were stained with 0.1% Crystal Violet and subsequently dissolved in 20% acetic acid. Dissolved biofilms were quantified by measuring the OD600 in a microtiter plate reader.

### P. aeruginosa growth in presence of peptides

Growth curves were obtained by growing *P. aeruginosa* (PAO1) in 96-well plates (Falcon #353072) in presence of 64 mg/L peptides at 37°C with agitation in Mueller-Hinton II Broth (MHB) (BD #212322) using an Epoch 2 microplate reader (BioTek).

### Cytotoxicity assays

### Resazurin reduction assay

The cytotoxicity of the peptides was determined with a Resazurin cell viability assay (Sigma #R7017). In a 96-well plate (Corning #3904), monolayers of epithelial HeLa ATCC CCL-2 cells were grown in 100 µl growth medium (DMEM (Sigma #D5796) containing 10% FCS). Afterwards, the medium was removed and either replaced by DMEM/10% FCS containing 2-fold serial diluted peptides with a highest concentration of 256 mg/L. After 24 hours, the medium was removed and replaced by 100 µl fresh growth medium. 1% Triton X-100 (Sigma) was used as positive lysis control. 20 µl of resazurin solution (0.1 mg/ml) was added. After 2 hours, the fluorescence signal was recorded using a microplate reader (Synergy H4 BioTek) with an excitation wavelength of 560 nm and emission wavelength 590 nm.

### LDH release assay

Cells were prepared and treated with peptides as described for the resazurin reduction assay. 24 hours after peptide treatment, control wells were treated with 1% Triton X-100 (Sigma) as lysis control. LDH release was measured using the LDH Cytotoxicity Detection Kit (Clontech). Absorbance was measured at 490 nm using a microplate reader (Synergy H4 BioTek). The percentage of cell death was calculated as follows: (LDH peptide treated - LDH untreated)/(LDH total lysis - LDH untreated)* 100.

### RESULTS

### Core binding sequence

To identify the minimal c-di-GMP binding sequence, peptide derivatives with various lengths were designed and their affinities to c-di-GMP were measured using ITC (Table 2). A sequence comprising 19 residues (DRRRFNSADYKGPRRRKAD) (SEQ ID NO: 52) that binds c-di-GMP with high affinity was identified. Shortening in the core binding sequence resulted in decreased binding affinities (SEQ ID NO: 8-11).

**Table 2. Dissociation constant (K_{d}) of peptides with different length towards c-di-GMP. Tryptophan (W) was added to peptides SEQ ID NO: 4, 5 and 7 for the ease of peptide amount quantification.**

| SEQ ID NO: | Sequence | Dissociation constant (µM) |
|---|---|---|
| 1 | SKPREWVEAVAYVGPDRRRFNSADYKGPRKRKADAS | 0.11 |
| 2 | SKPREWVEAVAYVGPDRRRFNSADYKGPRRRKADAS | 0.10 |
| 3 | WVEAVAYVGPDRRRFNSADYKGPRRRKAD | 0.15 |
| 4 | DRRRFNSADYKGPRRRKADW | 0.12 |
| 5 | WDRRRFNSADYKGPRRRKAD | 0.16 |
| 6 | YVGPDRRRFNSADYKGPRRRKAD | 0.13 |
| 7 | WVGPDRRRFNSADYKGPRRRKAD | 0.12 |
| 8 | RRRFNSADYKGPRRR | no binding |
| 9 | RRRFNSADYKGPRRRKAD | 1.41 |
| 10 | DRRRFNSADYKGPRRRK | 1.71 |
| 11 | DRRRFNSADYKGPRRR | no binding |

### Amino acid substitution analysis

Next, the binding motif was characterized by single amino acid substitution analysis. All positions were individually substituted by alanine, except for alanine residues A₈ and A₁₈, which were replaced by leucine (SEQ ID NO: 20 and 30, respectively). Binding affinities were determined using ITC, and the results are shown in Table 3 and Figure 2. Substitution at positions D₁ (SEQ ID NO: 13), R₂ (SEQ ID NO: 14), R₃ (SEQ ID NO: 15), Y₁₀ (SEQ ID NO: 22), G₁₂ (SEQ ID NO: 24), R₁₄ (SEQ ID NO: 26), R₁₅ (SEQ ID NO: 27), R₁₆ (SEQ ID NO: 28) and D₁₉ (SEQ ID NO: 31) significantly reduced binding affinities. In contrast, substitution at position X5 (SEQ ID NO: 17), X6 (SEQ ID NO: 18), X7 (SEQ ID NO: 19), X8 (SEQ ID NO: 20), X9 (SEQ ID NO: 21), X11 (SEQ ID NO: 23), X17 (SEQ ID NO: 29) and X18 (SEQ ID NO: 30) does not significantly reduce binding affinity, and despite having lower affinity than for example peptide of SEQ ID NO: 12, peptides of X4 (SEQ ID NO: 16) and X13 (SEQ ID NO: 25) still have affinities, which are sufficiently high to bind c-di-GMP and compete with c-di-GMP effector proteins having lower affinities (see also Figure 1).

**Table 3. Dissociation constant (K_{d}) of peptides with amino acid substitution.**

| SEQ ID NO: | Sequence | Dissociation constant (µM) |
|---|---|---|
| 12 | AC-DRRRFNSADYKGPRRRKAD-NH₂ | 0.17 |
| 13 | AC-ARRRFNSADYKGPRRRKAD-NH₂ | 1.53 |
| 14 | AC-DARRFNSADYKGPRRRKAD-NH₂ | 1.45 |
| 15 | AC-DRARFNSADYKGPRRRKAD-NH₂ | 1.51 |
| 16 | AC-DRRAFNSADYKGPRRRKAD-NH₂ | 0.78 |
| 17 | AC-DRRRANSADYKGPRRRKAD-NH₂ | 0.14 |
| 18 | AC-DRRRFASADYKGPRRRKAD-NH₂ | 0.22 |
| 19 | AC-DRRRFNAADYKGPRRRKAD-NH₂ | 0.21 |
| 20 | AC-DRRRFNSLDYKGPRRRKAD-NH₂ | 0.10 |
| 21 | AC-DRRRFNSAAYKGPRRRKAD-NH₂ | 0.15 |
| 22 | AC-DRRRFNSADAKGPRRRKAD-NH₂ | 4.36 |
| 23 | AC-DRRRFNSADYAGPRRRKAD-NH₂ | 0.26 |
| 24 | AC-DRRRFNSADYKAPRRRKAD-NH₂ | 4.07 |
| 25 | AC-DRRRFNSADYKGARRRKAD-NH₂ | 0.35 |
| 26 | AC-DRRRFNSADYKGPARRKAD-NH₂ | 1.83 |
| 27 | AC-DRRRFNSADYKGPRARKAD-NH₂ | 3.95 |
| 28 | AC-DRRRFNSADYKGPRRAKAD-NH₂ | no binding |
| 29 | AC-DRRRFNSADYKGPRRRAAD-NH₂ | 0.27 |
| 30 | AC-DRRRFNSADYKGPRRRKLD-NH₂ | 0.25 |
| 31 | AC-DRRRFNSADYKGPRRRKAA-NH₂ | 1.75 |

### Single amino acid deletion analysis

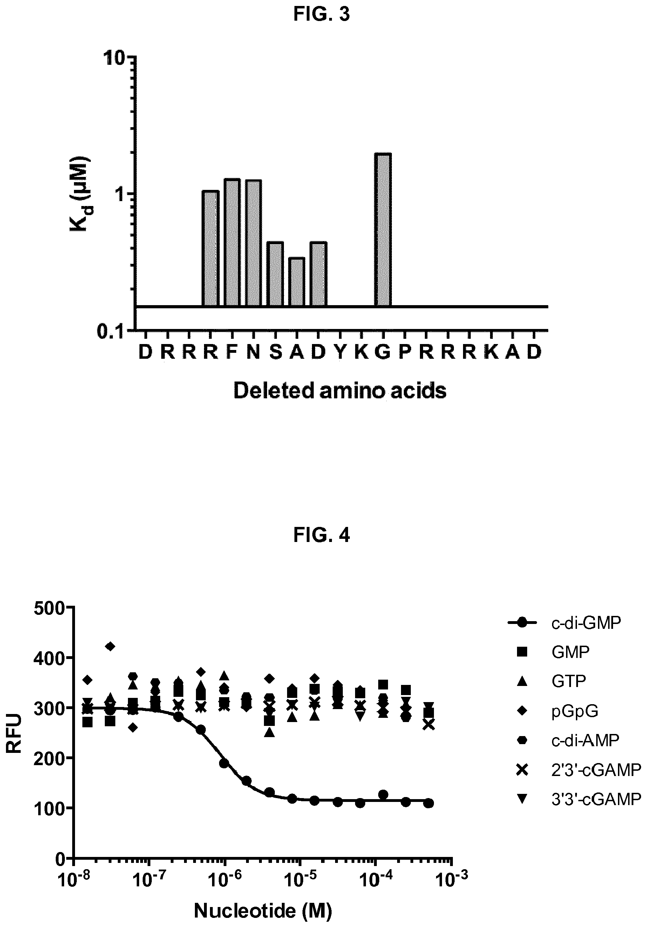

The core binding region was shortened by deletion at positions X₄ (cf. SEQ ID NO: 32), X₅ (cf. SEQ ID NO: 33), X₆ (cf. SEQ ID NO: 34), X₇ (cf. SEQ ID NO: 35), X₈ (cf. SEQ ID NO: 36), X₉ (cf. SEQ ID NO: 37) or G₁₂ (cf. SEQ ID NO: 38). Compared to the peptide of SEQ ID NO: 12, c-di-GMP binding affinities decreased only by about 2 to 3-fold when position X₇ (cf. SEQ ID NO: 35), X₈ (cf. SEQ ID NO: 36) or X₉ (cf. SEQ ID NO: 37) was deleted. Affinity decreased further when position X₄ (cf. SEQ ID NO: 32), X₅ (cf. SEQ ID NO: 33) or X₆ (cf. SEQ ID NO: 34) was deleted. A more drastic reduction in binding affinity was observed when position G₁₂ (cf. SEQ ID NO: 38) was removed from the peptide of SEQ ID NO: 12, with about 12-fold lower affinity (Table 4 and Figure 3).

**Table 4. Deletion of single amino acid.**

| SEQ ID NO: | Sequence | Dissociation constant (µM) |
|---|---|---|
| 32 | AC-DRRFNSADYKGPRRRKAD-NH₂ | 1.04 |
| 33 | AC-DRRRNSADYKGPRRRKAD-NH₂ | 1.27 |
| 34 | AC-DRRRFSADYKGPRRRKAD-NH₂ | 1.25 |
| 35 | AC-DRRRFNADYKGPRRRKAD-NH₂ | 0.44 |
| 36 | AC-DRRRFNSDYKGPRRRKAD-NH₂ | 0.34 |
| 37 | AC-DRRRFNSAYKGPRRRKAD-NH₂ | 0.44 |
| 38 | AC-DRRRFNSADYKPRRRKAD-NH₂ | 1.95 |

### Specificity

Further, it was demonstrated that the peptide of the invention binds specifically to c-di-GMP. As shown in Figure 4, Nucleotides closely related to c-di-GMP did not bind to the peptide SEQ ID NO: 43, which is identical to SEQ ID NO: 4 or 12 except for the C- or N-terminal modification. The c-di-GMP affinity could be fitted with a K_{d} of 0.79 µM (Figure 4, solid line).

### Stability

Peptide stability in *P. aeruginosa* PAO1 lysate was determined as described in the Materials and Methods section. After 2 hours, 11% of the peptide of SEQ ID NO: 12 could be detected. The same treatment was applied to all the single amino acid substituted peptides of SEQ ID NO: 13-31 (Table 5) to identify positions that are susceptible to proteolysis. The results revealed that many peptides are more stable when mutated to alanine at specific position, notably at position X₅ (cf. SEQ ID NO: 17), wherein 70% of the peptide could be detected.

To protect the peptide termini from exopeptidase activity, the first and last amino acids were substituted or extended with D-amino acids. The extension or substitution was coupled with acetylation of the N-terminal and amidation of the C-terminal (SEQ ID NO: 39-42). Only D-amino acid extension at both termini together with amidated C-terminal (SEQ ID NO: 42) showed significant improvement (Table 5).

**Table 5. Peptide stability in P. aeruginosa PAO1 cell lysate.**

| SEQ ID NO: | Sequence | Remaining peptide after 2 hours (%) |
|---|---|---|
| 12 | AC-DRRRFNSADYKGPRRRKAD-NH₂ | 11 |
| 13 | AC-ARRRFNSADYKGPRRRKAD-NH₂ | 56 |
| 14 | AC-DARRFNSADYKGPRRRKAD-NH₂ | 12 |
| 15 | AC-DRARFNSADYKGPRRRKAD-NH₂ | 11 |
| 16 | AC-DRRAFNSADYKGPRRRKAD-NH₂ | 35 |
| 17 | AC-DRRRANSADYKGPRRRKAD-NH₂ | 70 |
| 18 | AC-DRRRFASADYKGPRRRKAD-NH₂ | 1 |
| 19 | AC-DRRRFNAADYKGPRRRKAD-NH₂ | 30 |
| 20 | AC-DRRRFNSLDYKGPRRRKAD-NH₂ | 27 |
| 21 | AC-DRRRFNSAAYKGPRRRKAD-NH₂ | 2 |
| 22 | AC-DRRRFNSADAKGPRRRKAD-NH₂ | 39 |
| 23 | AC-DRRRFNSADYAGPRRRKAD-NH₂ | 42 |
| 24 | AC-DRRRFNSADYKAPRRRKAD-NH₂ | 41 |
| 25 | AC-DRRRFNSADYKGARRRKAD-NH₂ | 30 |
| 26 | AC-DRRRFNSADYKGPARRKAD-NH₂ | 27 |
| 27 | AC-DRRRFNSADYKGPRARKAD-NH₂ | 2 |
| 28 | AC-DRRRFNSADYKGPRRAKAD-NH₂ | 11 |
| 29 | AC-DRRRFNSADYKGPRRRAAD-NH₂ | 9 |
| 30 | AC-DRRRFNSADYKGPRRRKLD-NH₂ | 51 |
| 31 | AC-DRRRFNSADYKGPRRRKAA-NH₂ | 26 |
| 39 | dRRRFNSADYKGPRRRKAd | 19 |
| 40 | rDRRRFNSADYKGPRRRKADa | 15 |
| 41 | AC-rDRRRFNSADYKGPRRRKADa-NH₂ | 19 |
| 42 | rDRRRFNSADYKGPRRRKADa-NH₂ | 38 |

### Peptide stability in the presence of c-di-GMP

Next, the stability of peptides with different affinities to c-di-GMP was compared. Peptide of SEQ ID NO: 12 possessed high affinity towards c-di-GMP, while peptide SEQ ID NO: 28 did not bind c-di-GMP due to a single amino acid substitution (Table 3). As shown in Figure 5 and Table 6, peptide SEQ ID NO: 12 was much more stable in the presence of *P. aeruginosa* PAO1 lysate when it forms a complex with c-di-GMP, with 61% remaining peptide compared to 32% after 2 hour treatment. On the other hand, the stability of peptide SEQ ID NO: 28 was identical in the presence or absence of c-di-GMP.

**Table 6. Stability of selected peptides in the presence or absence of c-di-GMP.**

| SEQ ID NO: | Sequence | c-di-GMP | Remaining peptide after 2 hours (%) |
|---|---|---|---|
| 12 | AC-DRRRFNSADYKGPRRRKAD-NH₂ | - | 32 |
| 12 | AC-DRRRFNSADYKGPRRRKAD-NH₂ | + | 61 |
| 28 | AC-DRRRFNSADYKGPRRAKAD-NH₂ | - | 23 |
| 28 | AC-DRRRFNSADYKGPRRAKAD-NH₂ | + | 23 |

### Cell penetration

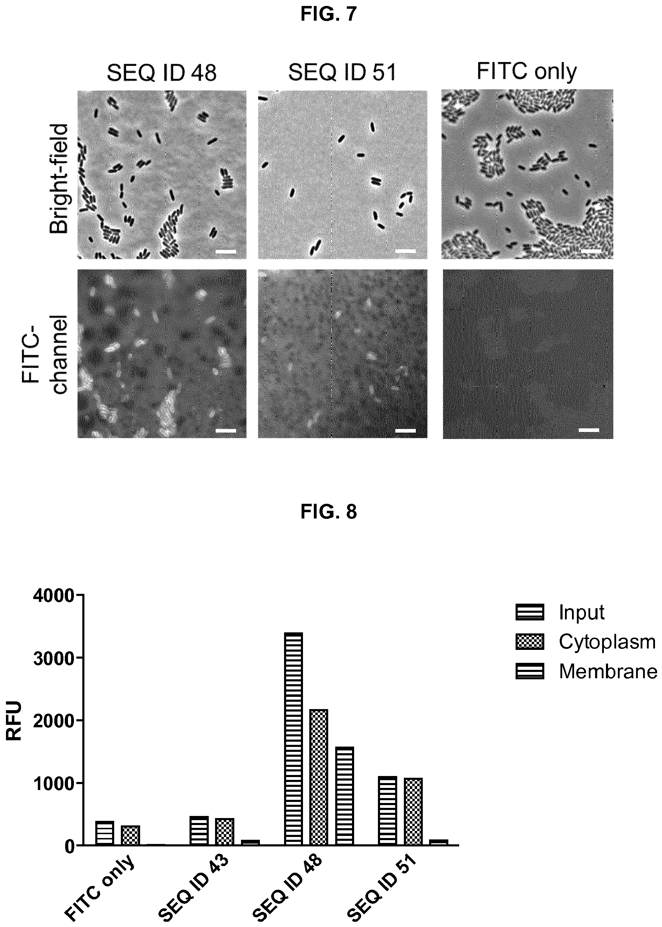

The peptide uptake by *P. aeruginosa* PAO1 cells was determined by flow cytometry as described in the Materials and Methods section. All peptides were N-terminally labeled with FITC to allow fluorescence detection (Table 7). As shown in Figure 6, the 19-residue peptide of SEQ ID NO: 43 has FITC signals comparable to the untreated control and FITC only, indicating very weak or no peptide uptake. Cell penetrating nona-arginine peptide alone (SEQ ID NO: 45) showed the most efficient uptake by *P. aeruginosa* PAO1 cells (Figure 6), therefore the peptide of invention was fused to poly-arginines peptides with 6, 7, 8 or 9 arginines (SEQ ID NO: 46-49). In addition, a shorter nona-arginine fusion was generated, by removing the first amino acid D₁ and extending the peptide by 6 arginines (SEQ ID NO: 44). The fusion peptide with octa-arginine (SEQ ID NO: 48) showed about 7-fold higher FITC signals compared to FITC alone. Further, HIV TAT peptides were fused to the peptide of invention (SEQ ID NO: 50 and 51; Table 7). Despite the difference in one residue, the signal for the peptide of SEQ ID NO: 50 was 2 fold higher than for the peptide of SEQ ID NO: 51 (Figure 7).

**Table 7. Peptides tested for P. aeruginosa cellular uptake.**

| SEQ ID NO: | Sequence |
|---|---|
| 43 | FITC-DRRRFNSADYKGPRRRKAD-NH₂ |
| 44 | FITC-RRRRRRRRRFNSADYKGPRRRKAD-NH₂ |
| 45 | FITC-RRRRRRRRR-NH₂ |
| 46 | FITC-RRRRRRDRRRFNSADYKGPRRRKAD-NH₂ |
| 47 | FITC-RRRRRRRDRRRFNSADYKGPRRRKAD-NH₂ |
| 48 | FITC-RRRRRRRRDRRRFNSADYKGPRRRKAD-NH₂ |
| 49 | FITC-RRRRRRRRRDRRRFNSADYKGPRRRKAD-NH₂ |
| 50 | FITC-YGRKKRRQRRRGRRRFNSADYKGPRRRKAD-NH₂ |
| 51 | FITC-YGRKKRRQRRRDGRRRFNSADYKGPRRRKAD-NH₂ |

Next, fluorescence microscopy was used to visualize localization of the peptides in *P. aeruginosa* PAO1. Peptides fused to either octa-arginine peptide (SEQ ID NO: 48) or TAT peptide (SEQ ID NO: 51) show a strong FITC signal on the cell membranes, although fluorescence signal could also be detected in the cytoplasm (Figure 7).

To ensure that the N-terminally attached octa-arginine or TAT peptides do not affect c-di-GMP binding, the affinities of peptide SEQ ID NO: 48 and 51 were determined using ITC as described above. The results showed that the affinities are comparable to the 19-mer peptide (SEQ ID NO: 4 or 12) despite the additional sequences, with K_{d} values of 0.25 µM and 0.2 µM for peptides SEQ ID NO: 48 and 51, respectively.

To further elucidate whether there is also FITC-labeled peptide in the cytoplasm, membranes were separated from cytoplasm using a fractionation approach (see Materials and Methods). As shown in Figure 8, signal was detected in all fractions, demonstrating that the peptides accumulated in the cytoplasm.

### Biofilm inhibition

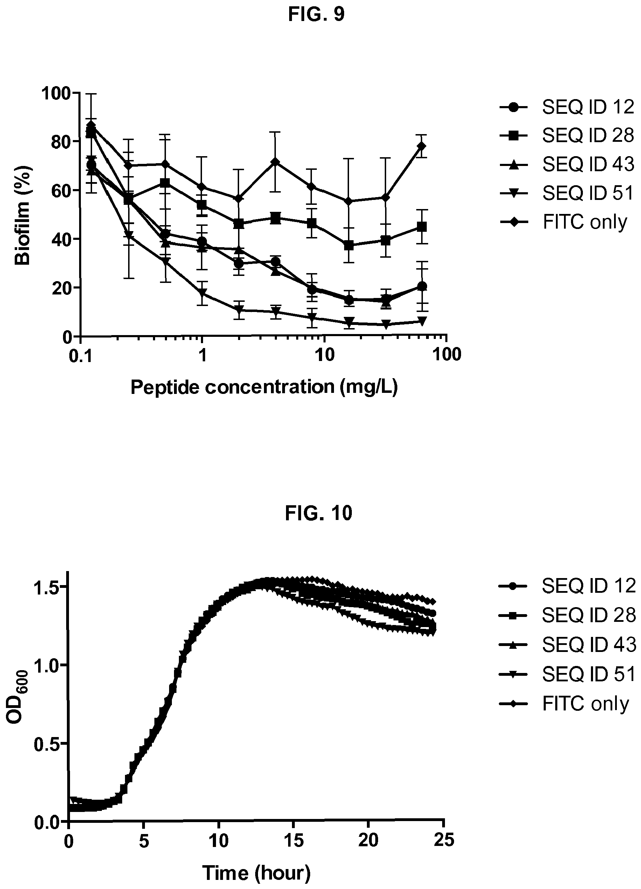

The peptide concentrations that reduce *P. aeruginosa* PAO1 biofilms by 50% (MBIC₅₀) were determined using a standard MBIC assay as described in the Materials and Methods. The MBIC₅₀ for the peptide of SEQ ID NO: 12, which binds c-di-GMP with high affinity, is between 0.25-0.5 mg/L. This value is about 4-fold lower than the MBIC₅₀ of the low affinity peptide of SEQ ID NO: 28. The sequence of peptide SEQ ID NO: 43 is identical to the peptide SEQ ID NO: 12, except for the additional FITC attached to the N-terminal. Consequently, both peptides possess identical MBIC₅₀ values (Table 8). Surprisingly, the cell penetrating fusion peptide (SEQ ID NO: 51) effectively lowers the MBIC₅₀ to below 0.25 mg/L and at the concentration of 2 mg/L this peptide reduced biofilms by 90% (Figure 9).

Notably, these peptides did not affect cell growth (Figure 10), which indicates that biofilm reduction were not due to killing of cells or delay in cellular growth phase. This property is crucial for anti-biofilm compounds, because bactericidal effects increase the risk of resistance, which is a devastating problem faced by the healthcare worldwide today. Moreover, these data show that peptides of the invention are not toxic to cells.

**Table 8. Peptides tested for MBIC₅₀.**

| SEQ ID NO: | Sequence | MBIC₅₀ (mg/L) |
|---|---|---|
| 12 | AC-DRRRFNSADYKGPRRRKAD-NH₂ | 0.25-0.5 |
| 28 | AC-DRRRFNSADYKGPRRAKAD-NH₂ | 1-2 |
| 43 | FITC-DRRRFNSADYKGPRRRKAD-NH₂ | 0.25-0.5 |
| 51 | FITC-YGRKKRRQRRRDGRRRFNSADYKGPRRRKAD-NH₂ | <0.25 |

### Cytotoxicity

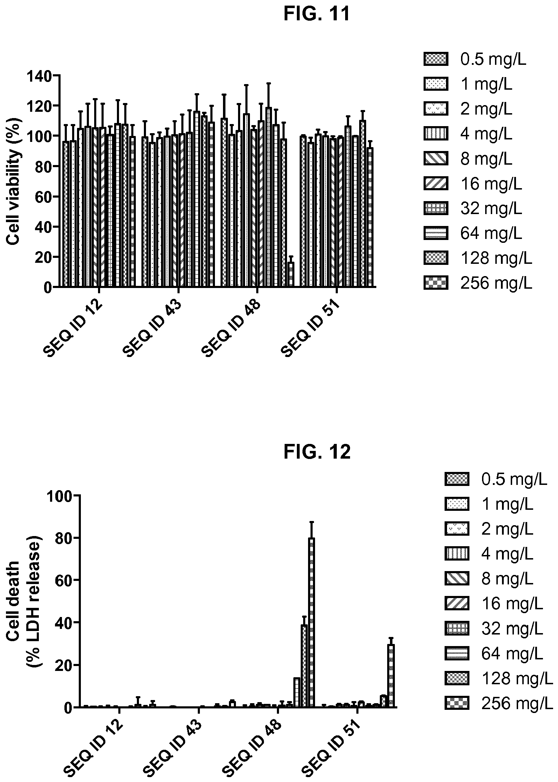

The cytotoxic effects of peptides on human cell viability were examined in epithelial HeLa cells using resazurin reduction assay (Figure 11) and LDH release assay (Figure 12). Peptides of SEQ ID NO: 12, 43 and 51 did not show a toxic effect, when a concentration of up to 100 times the MBIC50 concentration (64 mg/L) was applied, and peptide of SEQ ID NO: 48 is not toxic up to a concentration of 32 mg/L. These data demonstrate favorable safety and tolerability of the peptides of the invention.

## Claims

1. A peptide comprising at least one amino acid sequence (I), wherein said amino acid sequence (I) comprises, preferably consists of the amino sequence
D1-X2-X3-X4-X5-X6-X7-X8-X9-Y10-X11-G12-X13-R14-R15-R16-X17-X18-D19 (SEQ ID NO: 59), wherein
D1 and D19 are independently of each other (i) a deletion, (ii) an amino acid having a negative net charge at pH 7, wherein preferably said amino acid having a negative net charge at pH 7 is a D-amino acid or L-amino acid, and further preferably an L-amino acid having a negative net charge at pH 7, or (iii) glycine or a glycine derivative, wherein the glycine derivative is selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine, ethyl acetamidoacetate, alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, and wherein preferably said glycine derivative is a D-stereoisomer or L-stereoisomer, further preferably an L-stereoisomer;
X2, X3, X4, X5, X6, X7, X8, X9, X11, X13, X17 and X18 are independently of each other an amino acid, preferably a D-amino acid or L-amino acid, and further preferably an L-amino acid, or a deletion, wherein at most one of X2, X3, X4, X5, X6, X7, X8, X9, X11, X13, X17 or X18 is a deletion;
R14, R15 and R16 are independently of each other a basic amino acid, wherein preferably said basic amino acid is a D-amino acid or an L-amino acid, and further preferably a basic L-amino acid;
Y10 is an aromatic amino acid, wherein preferably said aromatic amino acid is D-amino acid or an L-amino acid, and further preferably an aromatic L-amino acid;
G12 is glycine or a glycine derivative, wherein the glycine derivative is selected from 2-aminobutyric acid, vinylglycine, benzylglycine, allylglycine, glycine C1-C3 alkylester, C1-C3 alkylglycine, C2-C3 alkenylglycine, formylglycine, N-formylglycine or ethyl acetamidoacetate, and wherein said glycine derivative is preferably a D-stereoisomer or L-stereoisomer, further preferably an L-stereoisomer;
and wherein said peptide has a length of at most 160 amino acids, preferably of at most 100 amino acids, further preferably of at most 50 amino acids and most preferably of at most 30 amino acids.

2. The peptide according to claim 1, wherein
said amino acid having a negative net charge at pH 7, preferably said D-amino acid or said L-amino acid having a negative net charge at pH 7, and further preferably said L-amino acid having a negative net charge at pH 7, is an aspartate derivative, wherein said aspartate derivative is selected from 2-amino-6-methylene-pimelic acid, 4-fluoro-glutamic acid, carboxymethylated cysteine, 2-amino-6-oxopimelic acid, 3,3-dimethyl aspartic acid, 4-methylglutamate, 3-methyl-glutamic acid, 2-aminoadipic acid, 5-o-methyl-glutamic acid, 3-methyl-aspartic acid, glutamate, 2-amino-propanedioic acid, 4-hydroxy-glutamic-acid, beta-hydroxyaspartic acid, 6-carboxylysine or aspartate, wherein preferably said aspartate derivative is aspartate, and wherein said aspartate derivative is preferably a D-stereoisomer or L-stereoisomer, further preferably an L-stereoisomer;
said basic amino acid, preferably said basic D-amino acid or said basic L-amino acid, is (i') an arginine derivative, wherein said arginine derivative is selected from arginine, citrulline, thio-citrulline, canavanine, 5-methyl-arginine, c-gamma-hydroxy arginine, homoarginine, 2-amino-3-guanidinopropionic acid, 2-amino-4-guanidinobutryric acid, homo-citrulline or 4-guanidinobutryric acid; and wherein preferably said arginine derivative is arginine; or (ii') a lysine derivative, wherein said lysine derivative is selected lysine, diaminobutyric acid, 2,3-diaminopropanoic acid, 2,8-diaminooctanoic acid, ornithine or thialysine, wherein preferably said lysine derivative is lysine, and wherein preferably said arginine derivative and said lysine derivative are independently of each other a D-stereoisomer or L-stereoisomer, further preferably an L-stereoisomer;
said aromatic amino acid, preferably said aromatic D-amino acid or said aromatic L-amino acid, is (i") a tyrosine derivative, wherein said tyrosine derivative is selected from phenylalanine, 4-methylphenylalanine, homophenylalanine or tyrosine; or (ii") a histidine derivative, wherein said histidine derivative is selected from 2-fluoro-histidine, 2-fluoro-histidine, 2-fluoro-histidine, (2-furyl)-alanine, histidine, 3-(1-pyrazolyl)-alanine, 3-(2-tetrazolyl)-alanine, 3-(3-thienyl)-alanine, 3-(2-thienyl)-alanine, 3-(1,2,4-triazol-1-yl)-alanine or (4-thiazolyl)-alanine; or (iii") a tryptophan derivative, wherein said tryptophan derivative is selected from 7-hydroxy-tryptophan, 3-(4H-thieno[3,2-b]pyrrol-6-yl)-alanine, 4-fluoro-tryptophan, 4-hydroxy-tryptophan, 4-amino-tryptophan, 6-chloro-tryptophan, 3-(3-benzothienyl)-alanine, 6-bromo-tryptophan, 7-chloro-tryptophan, 6-fluoro-tryptophan, 5-fluoro-tryptophan, 5-hydroxy-tryptophan, beta-hydroxy-tryptophan, 5-methoxy-tryptophan, 5-methyl-tryptophan, 6-methyl-tryptophan, 2-hydroxy-tryptophan, tryptophan, 6-hydroxy-tryptophan or 6-amino-7-hydroxy-tryptophan; wherein preferably said aromatic acid is selected from tyrosine, phenylalanine, histidine or tryptophan, and wherein preferably said tyrosine derivative, said histidine derivative and said tryptophan derivative are independently of each other a D-stereoisomer or L-stereoisomer, further preferably an L-stereoisomer.

3. The peptide according to claim 1 or 2, wherein
D1 and D 19 are independently of each other glycine or a D-amino acid or L-amino acid, preferably an L-amino acid, wherein said D-amino acid or L-amino acid is selected from a D- or L-stereoisomer of vinylglycine, 2-allyl-glycine, alanine, 2-aminobutyric acid, diethylalanine, leucine, allo-isoleucine, isoleucine, norleucine, valine, norvaline, 3-methyl-aspartic acid, beta-hydroxyaspartic acid, 3,3-dimethyl aspartic acid or aspartate, and wherein preferably D1 and D19 are independently of each other selected from glycine, D-aspartate or L-aspartate, and wherein more preferably D1 and D19 are independently of each other selected from glycine or L-aspartate;
R14, R15 and R16 are independently of each other an arginine derivative, wherein said arginine derivative is independently of each other selected from arginine, 5-methyl-arginine, c-gamma-hydroxy arginine and homoarginine, citrulline, 2-amino-4-guanidinobutryric acid, 2-amino-3-guanidinopropionic acid, lysine, ornithine, or 2,8-diaminooctanoic acid, wherein preferably said arginine derivative is a D-stereoisomer or L-stereoisomer, further preferably an L-stereoisomer, and wherein again further preferably R14, R15 and R16 are independently of each other D-arginine or L-arginine;
Y10 is selected from a D-stereoisomer or L-stereoisomer of phenylalanine, histidine, tryptophan or tyrosine, wherein preferably Y10 is D-tyrosine or L-tyrosine; and
G12 is glycine.

4. The peptide according to any one of the preceding claims, wherein at least one of said amino acid sequence (I) has at least 73 % sequence identity, preferably at least 78 % sequence identity, more preferably at least 84 % sequence identity, even more preferably at least 89 % sequence identity, again more preferably at least 94 % sequence identity to the amino acid sequence of DRRRFNSADYKGPRRRKAD (SEQ ID NO: 52).

5. The peptide according to any one of the preceding claims, wherein at least one of said amino acid sequence (I), wherein preferably all of said at least one amino acid sequence (I), is the amino acid sequence of DRRRFNSADYKGPRRRKAD (SEQ ID NO: 52) or an amino acid sequence, wherein up to 6 amino acids, preferably up to 5, further preferably up to 4, again further preferably up to 3, again further preferably up to 2, and again further preferably one amino acid of SEQ ID NO: 52 is/are exchanged, and wherein the up to 6 amino acid exchanges are independently of each other selected from:
D at positions 1 and 19 are independently of each other optionally exchanged by (i) a deletion, (ii) an amino acid having a negative net charge at pH 7, wherein preferably said amino acid having a negative net charge at pH 7 is a D-amino acid or L-amino acid, and further preferably an L-amino acid having a negative net charge at pH 7, or (iii) glycine or a glycine derivative, wherein the glycine derivative is selected from N-acetylglycine, ethyl acetamidoacetate, 2-aminobutyric acid, vinylglycine, benzylglycine, 2-allylglycine, glycine C1-C3 alkylester, N-formylglycine C1-C3 alkylester, C1-C3 N-alkylglycine, C2-C3 N-alkenylglycine, N-formylglycine, ethyl acetamidoacetate, alanine, 2-aminoheptanoic acid, 5-bromo-isoleucine, tertleucine, 3-chloro-alanine, cyclohexylglycine, 3-cyclopentyl-alanine, diethylalanine, trifluoro-alanine, 3-fluoro-valine, homoleucine, 3-methyl-alloisoleucine, allo-isoleucine, isoleucine, 5-fluoro-leucine, leucine, norleucine, norvaline, 2-amino-4,4-difluorobutanoic acid, 5,5,5-trifluoro-leucine or valine, wherein preferably said glycine derivative is a D-stereoisomer or L-stereoisomer, further preferably an L-stereoisomer, and wherein again further preferably D at positions 1 and 19 are independently of each other optionally exchanged by glycine;
R at positions 14, 15 and 16 are independently of each other optionally exchanged by a basic amino acid, preferably by a basic D-amino acid or a basic L-amino acid, and further preferably by a basic L-amino acid;
amino acids at positions 2, 3, 4, 5, 6, 7, 8, 9, 11, 13, 17 and 18 are independently of each other optionally exchanged by any amino acid, preferably by a D-amino acid or L-amino acid, and further preferably by an L-amino acid, or a deletion, wherein at most one of positions 2, 3, 4, 5, 6, 7, 8, 9, 11, 13, 17 and 18 is optionally exchanged by a deletion;
Y at position 10 is optionally exchanged by an aromatic amino acid, preferably by an aromatic D-amino acid or an aromatic L-amino acid, and further preferably by an aromatic L-amino acid; and
G at position 12 is optionally exchanged by a glycine derivative, wherein said glycine derivative is selected from 2-aminobutyric acid, vinylglycine, benzylglycine, allylglycine, glycine C1-C3 alkylester, C1-C3 alkylglycine, C2-C3 alkenylglycine, formylglycine, N-formylglycine or ethyl acetamidoacetate, wherein preferably said glycine derivative is a D-stereoisomer or L-stereoisomer, further preferably an L-stereoisomer.

6. The peptide according to any one of the preceding claims, wherein at least one of X2, X3, X4, X5, X6, X7, X8, X9, X11, X13, X17 or X18 is independently of each other D-alanine or L-alanine, preferably at least one of X4, X5, X6, X7, X8, X9, X11, X13, X17 or X18 is independently of each other D-alanine or L-alanine.

7. The peptide according to any one of the preceding claims, wherein X2 and X3 are independently of each other D-arginine or L-arginine, and wherein preferably X2, X3 and X4 are independently of each other D-arginine or L-arginine.

8. The peptide according to any one of the preceding claims, wherein said amino acid sequence (I) is an amino acid sequence selected from SEQ ID NO: 1 to 7, 9, 10, 12 to 21, 23, 25, 29 to 37, 39 to 44, 46 to 52 or 60 to 87, and wherein preferably said amino acid sequence (I) is an amino acid sequence selected from SEQ ID NO: 1 to 7, 12, 13, 17 to 21, 23, 29, 30, 43, 48, 50 to 52, 60, 64 to 68, 69, 77, 78, 84, 86 or 87.

9. The peptide according to any one of the preceding claims, wherein said peptide comprises at least two of said amino acid sequence (I), and wherein preferably said amino acid sequence (I) is an amino acid sequence selected from SEQ ID NO: 1 to 7, 9, 10, 12 to 21, 23, 25, 29 to 37, 39 to 44, 46 to 52 or 60 to 87, and wherein further preferably said amino acid sequence (I) is an amino acid sequence selected from SEQ ID NO: 1 to 7, 12, 13, 17 to 21, 23, 29, 30, 43, 48, 50 to 52, 60, 64 to 68, 69, 77, 78, 84, 86 or 87.

10. The peptide according to any one of the preceding claims comprising
(i) an N-terminus selected from a free amine group or a modified N-terminus, wherein said modified N-terminus includes a modification selected from (a) acetylation of said N-terminus of the peptide; (b) addition of at least one, preferably exactly one N-terminal compound to said N-terminus of the peptide; (c) addition of at least one, preferably exactly one N-terminal compound to said N-terminus of the peptide and acetylation of an N-terminus of said N-terminal compound; (d) addition of at least one, preferably exactly one N-terminal D-amino acid to said N-terminus of the peptide; or (e) addition of at least one, preferably exactly one N-terminal D-amino acid to said N-terminus of the peptide and acetylation of an N-terminus of said D-amino acid; wherein preferably said N-terminus is a modified N-terminus, and wherein further preferably said modified N-terminus is (d) addition of at least one, preferably exactly one N-terminal D-amino acid to said N-terminus of the peptide; and
(ii) a C-terminus which is selected from a free carboxyl group or a modified C-terminus, wherein said modified C-terminus includes a modification selected from (a') amidation of said C-terminus of the peptide; (b') addition of at least one, preferably exactly one C-terminal compound to said C-terminus of the peptide; (c') addition of at least one, preferably exactly one C-terminal compound to said C-terminus of the peptide and amidation of the C-terminus of said C-terminal compound; (d') addition of at least one, preferably exactly one C-terminal D-amino acid to said C-terminus of the peptide; or (e') addition of at least one, preferably exactly one C-terminal D-amino acid to said C-terminus of the peptide and amidation of the C-terminus of said D-amino acid, wherein preferably said C-terminus is a modified C-terminus, and wherein further preferably said modified C-terminus is (e') addition of at least one, preferably exactly one C-terminal D-amino acid to said C-terminus of the peptide and amidation of the C-terminus of said D-amino acid.

11. The peptide according to claim 10, wherein said C-terminal compound or said N-terminal compound, preferably said N-terminal compound, is a cell penetrating peptide; and wherein preferably said cell penetrating peptide is polyarginine or a trans-activating transcriptional activator peptide (TAT).

12. A pharmaceutical composition comprising at least one peptide according to any one of the preceding claims.

13. The pharmaceutical composition of claim 12, comprising one or more pharmaceutically active agents selected from an anti-infective, an antiseptic or a combination of at least one anti-infective and at least one antiseptic; wherein preferably said one or more pharmaceutically active agent is an anti-infective, wherein further preferably said anti-infective is an antibiotic; and wherein again further preferably said antibiotic is effective in reducing *Pseudomonas aeruginosa*.

14. A peptide as claimed in any one of claims 1 to 11 for use as a medicament in the treatment or prevention of a disease, wherein preferably said disease is a microbial, bacterial, fungal or protozoan infection, and wherein more preferably said disease is a microbial infection, wherein again more preferably said microbial infection is associated with or caused by *Pseudomonas aeruginosa* in a patient having cystic fibrosis.

15. A nucleic acid encoding the peptide as claimed in any one of the claims 1 to 11, preferably the nucleic acid comprises or preferably consists of at least one nucleic acid sequence selected from SEQ ID NO: 54 to 58.


SEQUENCE LISTING

<110>  UNIVERSITÄT BASEL

<120>  C-DI-GMP BINDING PEPTIDES

<130>  P5110EP00

<160>  87

<170>  PatentIn version 3.5

<210>  1
<211>  36
<212>  PRT
<213>  Artificial Sequence

<220>
<223>  Artificial peptide

<400>  1

Ser Lys Pro Arg Glu Trp Val Glu Ala Val Ala Tyr Val Gly Pro Asp
1               5                   10                  15

Arg Arg Arg Phe Asn Ser Ala Asp Tyr Lys Gly Pro Arg Lys Arg Lys
            20                  25                  30

Ala Asp Ala Ser
        35

<210>  2
<211>  36
<212>  PRT
<213>  Artificial Sequence

<220>
<223>  Artificial peptide

<400>  2

Ser Lys Pro Arg Glu Trp Val Glu Ala Val Ala Tyr Val Gly Pro Asp
1               5                   10                  15

Arg Arg Arg Phe Asn Ser Ala Asp Tyr Lys Gly Pro Arg Arg Arg Lys
            20                  25                  30

Ala Asp Ala Ser
        35

<210>  3
<211>  29
<212>  PRT
<213>  Artificial Sequence

<220>
<223>  Artificial peptide

<400>  3

Trp Val Glu Ala Val Ala Tyr Val Gly Pro Asp Arg Arg Arg Phe Asn
1               5                   10                  15

Ser Ala Asp Tyr Lys Gly Pro Arg Arg Arg Lys Ala Asp
            20                  25

<210>  4
<211>  20
<212>  PRT
<213>  Artificial Sequence

<220>
<223>  Artificial peptide

<400>  4

Asp Arg Arg Arg Phe Asn Ser Ala Asp Tyr Lys Gly Pro Arg Arg Arg
1               5                   10                  15

Lys Ala Asp Trp
            20

<210>  5
<211>  20
<212>  PRT
<213>  Artificial Sequence

<220>
<223>  Artificial peptide

<400>  5

Trp Asp Arg Arg Arg Phe Asn Ser Ala Asp Tyr Lys Gly Pro Arg Arg
1               5                   10                  15

Arg Lys Ala Asp
            20

<210>  6
<211>  23
<212>  PRT
<213>  Artificial Sequence

<220>
<223>  Artificial peptide

<400>  6

Tyr Val Gly Pro Asp Arg Arg Arg Phe Asn Ser Ala Asp Tyr Lys Gly
1               5                   10                  15

Pro Arg Arg Arg Lys Ala Asp
            20

<210>  7
<211>  23
<212>  PRT
<213>  Artificial Sequence

<220>
<223>  Artificial peptide

<400>  7

Trp Val Gly Pro Asp Arg Arg Arg Phe Asn Ser Ala Asp Tyr Lys Gly
1               5                   10                  15

Pro Arg Arg Arg Lys Ala Asp
            20

<210>  8
<211>  15
<212>  PRT
<213>  Artificial Sequence

<220>
<223>  Artificial peptide

<400>  8

Arg Arg Arg Phe Asn Ser Ala Asp Tyr Lys Gly Pro Arg Arg Arg
1               5                   10                  15

<210>  9
<211>  18
<212>  PRT
<213>  Artificial Sequence

<220>
<223>  Artificial peptide

<400>  9

Arg Arg Arg Phe Asn Ser Ala Asp Tyr Lys Gly Pro Arg Arg Arg Lys
1               5                   10                  15

Ala Asp

<210>  10
<211>  17
<212>  PRT
<213>  Artificial Sequence

<220>
<223>  Artificial peptide

<400>  10

Asp Arg Arg Arg Phe Asn Ser Ala Asp Tyr Lys Gly Pro Arg Arg Arg

1                   5                   10                  15

Lys

<210>  11
<211>  16
<212>  PRT
<213>  Artificial Sequence

<220>
<223>  Artificial peptide

<400>  11

Asp Arg Arg Arg Phe Asn Ser Ala Asp Tyr Lys Gly Pro Arg Arg Arg
1               5                   10                  15

<210>  12
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  12

Asp Arg Arg Arg Phe Asn Ser Ala Asp Tyr Lys Gly Pro Arg Arg Arg
1               5                   10                  15

Lys Ala Asp

<210>  13
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  13

Ala Arg Arg Arg Phe Asn Ser Ala Asp Tyr Lys Gly Pro Arg Arg Arg
1               5                   10                  15

Lys Ala Asp

<210>  14
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  14

Asp Ala Arg Arg Phe Asn Ser Ala Asp Tyr Lys Gly Pro Arg Arg Arg
1               5                   10                  15

Lys Ala Asp

<210>  15
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  15

Asp Arg Ala Arg Phe Asn Ser Ala Asp Tyr Lys Gly Pro Arg Arg Arg
1               5                   10                  15

Lys Ala Asp

<210>  16
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  16

Asp Arg Arg Ala Phe Asn Ser Ala Asp Tyr Lys Gly Pro Arg Arg Arg
1               5                   10                  15

Lys Ala Asp

<210>  17
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  17

Asp Arg Arg Arg Ala Asn Ser Ala Asp Tyr Lys Gly Pro Arg Arg Arg
1               5                   10                  15

Lys Ala Asp

<210>  18
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  18

Asp Arg Arg Arg Phe Ala Ser Ala Asp Tyr Lys Gly Pro Arg Arg Arg
1               5                   10                  15

Lys Ala Asp

<210>  19
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  19

Asp Arg Arg Arg Phe Asn Ala Ala Asp Tyr Lys Gly Pro Arg Arg Arg
1               5                   10                  15

Lys Ala Asp

<210>  20
<211>  19
<212>  PRT
<213>  Artificial Sequence

<220>
<223>  Artificial peptide


<220>
<221>  MOD_RES
<222>  (1)..(1)
<223>  N-terminal acetylation

<220>
<221>  MOD_RES
<222>  (19)..(19)
<223>  C-terminal amidation

<400>  20

Asp Arg Arg Arg Phe Asn Ser Leu Asp Tyr Lys Gly Pro Arg Arg Arg
1               5                   10                  15


Lys Ala Asp




<210>  21
<211>  19
<212>  PRT
<213>  Artificial Sequence

<220>
<223>  Artificial peptide


<220>
<221>  MOD_RES
<222>  (1)..(1)
<223>  N-terminal acetylation

<220>
<221>  MOD_RES
<222>  (19)..(19)
<223>  C-terminal amidation

<400>  21

Asp Arg Arg Arg Phe Asn Ser Ala Ala Tyr Lys Gly Pro Arg Arg Arg
1               5                   10                  15


Lys Ala Asp




<210>  22
<211>  19
<212>  PRT
<213>  Artificial Sequence

<220>
<223>  Artificial peptide


<220>
<221>  MOD_RES
<222>  (1)..(1)
<223>  N-terminal acetylation

<220>
<221>  MOD_RES
<222>  (19)..(19)
<223>  C-terminal amidation

<400>  22

Asp Arg Arg Arg Phe Asn Ser Ala Asp Ala Lys Gly Pro Arg Arg Arg
1               5                   10                  15


Lys Ala Asp




<210>  23
<211>  19
<212>  PRT
<213>  Artificial Sequence

<220>
<223>  Artificial peptide


<220>
<221>  MOD_RES
<222>  (1)..(1)
<223>  N-terminal acetylation

<220>
<221>  MOD_RES
<222>  (19)..(19)
<223>  C-terminal amidation

<400>  23

Asp Arg Arg Arg Phe Asn Ser Ala Asp Tyr Ala Gly Pro Arg Arg Arg
1               5                   10                  15


Lys Ala Asp




<210>  24
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  24

Asp Arg Arg Arg Phe Asn Ser Ala Asp Tyr Lys Ala Pro Arg Arg Arg
1               5                   10                  15

Lys Ala Asp

<210>  25
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  25

Asp Arg Arg Arg Phe Asn Ser Ala Asp Tyr Lys Gly Ala Arg Arg Arg
1               5                   10                  15

Lys Ala Asp

<210>  26
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  26

Asp Arg Arg Arg Phe Asn Ser Ala Asp Tyr Lys Gly Pro Ala Arg Arg
1               5                   10                  15

Lys Ala Asp

<210>  27
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  27

Asp Arg Arg Arg Phe Asn Ser Ala Asp Tyr Lys Gly Pro Arg Ala Arg
1               5                   10                  15

Lys Ala Asp

<210>  28
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  28

Asp Arg Arg Arg Phe Asn Ser Ala Asp Tyr Lys Gly Pro Arg Arg Ala
1               5                   10                  15

Lys Ala Asp

<210>  29
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  29

Asp Arg Arg Arg Phe Asn Ser Ala Asp Tyr Lys Gly Pro Arg Arg Arg
1               5                   10                  15

Ala Ala Asp

<210>  30
<211>  19
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (19)..(19)
<223>  C-terminal amidation

<400>  30

Asp Arg Arg Arg Phe Asn Ser Ala Asp Tyr Lys Gly Pro Arg Arg Arg
1               5                   10                  15

Lys Leu Asp

<210> 31
<211> 19
<212> PRT
<213> Artificial Sequence

<220>
<223> Artificial peptide

<220>
<221> MOD_RES
<222> (1)..(1)
<223> N-terminal acetylation

<220>
<221> MOD_RES
<222> (19)..(19)
<223> C-terminal amidation

<400> 31

Asp Arg Arg Arg Phe Asn Ser Ala Asp Tyr Lys Gly Pro Arg Arg Arg
1               5                   10                  15

Lys Ala Ala

<210> 32
<211> 18
<212> PRT
<213> Artificial Sequence

<220>
<223> Artificial peptide

<220>
<221> MOD_RES
<222> (1)..(1)
<223> N-terminal acetylation

<220>
<221> MOD_RES
<222> (18)..(18)
<223> C-terminal amidation

<400> 32

Asp Arg Arg Phe Asn Ser Ala Asp Tyr Lys Gly Pro Arg Arg Arg Lys
1               5                   10                  15

Ala Asp

<210>  33
<211>  18
<212>  PRT
<213>  Artificial Sequence

<220>
<223>  Artificial peptide


<220>
<221>  MOD_RES
<222>  (1)..(1)
<223>  N-terminal acetylation

<220>
<221>  MOD_RES
<222>  (18)..(18)
<223>  C-terminal amidation

<400>  33

Asp Arg Arg Arg Asn Ser Ala Asp Tyr Lys Gly Pro Arg Arg Arg Lys
1               5                   10                  15


Ala Asp




<210>  34
<211>  18
<212>  PRT
<213>  Artificial Sequence

<220>
<223>  Artificial peptide


<220>
<221>  MOD_RES
<222>  (1)..(1)
<223>  N-terminal acetylation

<220>
<221>  MOD_RES
<222>  (18)..(18)
<223>  C-terminal amidation

<400>  34

Asp Arg Arg Arg Phe Ser Ala Asp Tyr Lys Gly Pro Arg Arg Arg Lys
1               5                   10                  15


Ala Asp




<210>  35
<211>  18
<212>  PRT
<213>  Artificial Sequence

<220>
<223>  Artificial peptide


<220>
<221>  MOD_RES
<222>  (1)..(1)
<223>  N-terminal acetylation

<220>
<221>  MOD_RES
<222>  (18)..(18)
<223>  C-terminal amidation

<400>  35

Asp Arg Arg Arg Phe Asn Ala Asp Tyr Lys Gly Pro Arg Arg Arg Lys
1               5                   10                  15


Ala Asp




<210>  36
<211>  18
<212>  PRT
<213>  Artificial Sequence

<220>
<223>  Artificial peptide


<220>
<221>  MOD_RES
<222>  (1)..(1)
<223>  N-terminal acetylation

<220>
<221>  MOD_RES
<222>  (18)..(18)
<223>  C-terminal amidation

<400>  36

Asp Arg Arg Arg Phe Asn Ser Asp Tyr Lys Gly Pro Arg Arg Arg Lys
1               5                   10                  15


Ala Asp




<210>  37
<211>  18
<212>  PRT
<213>  Artificial Sequence

<220>
<223>  Artificial peptide


<220>

<221>  MOD_RES
<222>  (1)..(1)
<223>  N-terminal acetylation

<220>
<221>  MOD_RES
<222>  (18)..(18)
<223>  C-terminal amidation

<400>  37

Asp Arg Arg Arg Phe Asn Ser Ala Tyr Lys Gly Pro Arg Arg Arg Lys
1               5                   10                  15

Ala Asp

<210>  38
<211>  18
<212>  PRT
<213>  Artificial Sequence

<220>
<223>  Artificial peptide

<220>
<221>  MOD_RES
<222>  (1)..(1)
<223>  N-terminal acetylation

<220>
<221>  MOD_RES
<222>  (18)..(18)
<223>  C-terminal amidation

<400>  38

Asp Arg Arg Arg Phe Asn Ser Ala Asp Tyr Lys Pro Arg Arg Arg Lys
1               5                   10                  15

Ala Asp

<210>  39
<211>  19
<212>  PRT
<213>  Artificial Sequence

<220>
<223>  Artificial peptide

<220>
<221>  MISC_FEATURE
<222>  (1)..(1)
<223>  D-amino acid Asp

<220>

<221>  MISC_FEATURE
<222>  (19)..(19)
<223>  D-amino acid Asp

<400>  39

Xaa Arg Arg Arg Phe Asn Ser Ala Asp Tyr Lys Gly Pro Arg Arg Arg
1               5                   10                  15

Lys Ala Xaa

<210>  40
<211>  21
<212>  PRT
<213>  Artificial Sequence

<220>
<223>  Artificial peptide

<220>
<221>  MISC_FEATURE
<222>  (1)..(1)
<223>  D-amino acid Arg

<220>
<221>  MISC_FEATURE
<222>  (21)..(21)
<223>  D-amino acid Ala

<400>  40

Xaa Asp Arg Arg Arg Phe Asn Ser Ala Asp Tyr Lys Gly Pro Arg Arg
1               5                   10                  15

Arg Lys Ala Asp Xaa
            20

<210>  41
<211>  21
<212>  PRT
<213>  Artificial Sequence

<220>
<223>  Artificial peptide

<220>
<221>  MOD_RES
<222>  (1)..(1)
<223>  N-terminal acetylation

<220>
<221>  MISC_FEATURE
<222>  (1)..(1)
<223>  D-amino acid Arg

<220>

<221>  MOD_RES
<222>  (21)..(21)
<223>  C-terminal amidation

<220>
<221>  MISC_FEATURE
<222>  (21)..(21)
<223>  D-amino acid Ala

<400>  41

Xaa Asp Arg Arg Arg Phe Asn Ser Ala Asp Tyr Lys Gly Pro Arg Arg
1               5                   10                  15


Arg Lys Ala Asp Xaa
            20


<210>  42
<211>  21
<212>  PRT
<213>  Artificial Sequence

<220>
<223>  Artificial peptide


<220>
<221>  MISC_FEATURE
<222>  (1)..(1)
<223>  D-amino acid Arg

<220>
<221>  MISC_FEATURE
<222>  (21)..(21)
<223>  D-amino acid Ala

<220>
<221>  MOD_RES
<222>  (21)..(21)
<223>  C-terminal amidation

<400>  42

Xaa Asp Arg Arg Arg Phe Asn Ser Ala Asp Tyr Lys Gly Pro Arg Arg
1               5                   10                  15


Arg Lys Ala Asp Xaa
            20


<210>  43
<211>  19
<212>  PRT
<213>  Artificial Sequence

<220>
<223>  Artificial peptide


<220>

<221>  MOD_RES
<222>  (1)..(1)
<223>  FITC coupled to Asp

<220>
<221>  MOD_RES
<222>  (19)..(19)
<223>  C-terminal amidation

<400>  43

Asp Arg Arg Arg Phe Asn Ser Ala Asp Tyr Lys Gly Pro Arg Arg Arg
1               5                   10                  15

Lys Ala Asp

<210>  44
<211>  24
<212>  PRT
<213>  Artificial Sequence

<220>
<223>  Artificial peptide

<220>
<221>  MOD_RES
<222>  (1)..(1)
<223>  FITC-coupled Arg

<220>
<221>  MOD_RES
<222>  (24)..(24)
<223>  C-terminal amidation

<400>  44

Arg Arg Arg Arg Arg Arg Arg Arg Arg Phe Asn Ser Ala Asp Tyr Lys
1               5                   10                  15

Gly Pro Arg Arg Arg Lys Ala Asp
            20

<210>  45
<211>  9
<212>  PRT
<213>  Artificial Sequence

<220>
<223>  Poly-arginine peptide

<220>
<221>  MOD_RES
<222>  (1)..(1)
<223>  FITC-coupled Arg

<220>

<221>  MOD_RES
<222>  (9)..(9)
<223>  C-terminal amidation

<400>  45

Arg Arg Arg Arg Arg Arg Arg Arg Arg
1               5


<210>  46
<211>  25
<212>  PRT
<213>  Artificial Sequence

<220>
<223>  Artificial peptide


<220>
<221>  MOD_RES
<222>  (1)..(1)
<223>  FITC coupled Arg

<220>
<221>  MOD_RES
<222>  (25)..(25)
<223>  C-terminal amidation

<400>  46

Arg Arg Arg Arg Arg Arg Asp Arg Arg Arg Phe Asn Ser Ala Asp Tyr
1               5                   10                  15


Lys Gly Pro Arg Arg Arg Lys Ala Asp
            20                  25


<210>  47
<211>  26
<212>  PRT
<213>  Artificial Sequence

<220>
<223>  Artificial peptide


<220>
<221>  MOD_RES
<222>  (1)..(1)
<223>  FITC-coupled Arg

<220>
<221>  MOD_RES
<222>  (26)..(26)
<223>  C-terminal amidation

<400>  47

Arg Arg Arg Arg Arg Arg Arg Asp Arg Arg Arg Phe Asn Ser Ala Asp
1               5                   10                  15

Tyr Lys Gly Pro Arg Arg Arg Lys Ala Asp
            20                  25

<210>  48
<211>  27
<212>  PRT
<213>  Artificial Sequence

<220>
<223>  Artificial peptide

<220>
<221>  MOD_RES
<222>  (1)..(1)
<223>  FITC-coupled Arg

<220>
<221>  MOD_RES
<222>  (27)..(27)
<223>  C-terminal amidation

<400>  48

Arg Arg Arg Arg Arg Arg Arg Arg Asp Arg Arg Arg Phe Asn Ser Ala
1               5                   10                  15

Asp Tyr Lys Gly Pro Arg Arg Arg Lys Ala Asp
            20                  25

<210>  49
<211>  28
<212>  PRT
<213>  Artificial Sequence

<220>
<223>  Artificial peptide

<220>
<221>  MOD_RES
<222>  (1)..(1)
<223>  FITC-coupled Arg

<220>
<221>  MOD_RES
<222>  (28)..(28)
<223>  C-terminal amidation

<400>  49

Arg Arg Arg Arg Arg Arg Arg Arg Arg Asp Arg Arg Arg Phe Asn Ser
1               5                   10                  15

Ala Asp Tyr Lys Gly Pro Arg Arg Arg Lys Ala Asp
            20                  25

<210>  50
<211>  30
<212>  PRT
<213>  Artificial Sequence

<220>
<223>  Artificial peptide


<220>
<221>  MOD_RES
<222>  (1)..(1)
<223>  FITC-coupled Tyr

<220>
<221>  MOD_RES
<222>  (30)..(30)
<223>  C-terminal amidation

<400>  50

Tyr Gly Arg Lys Lys Arg Arg Gln Arg Arg Arg Gly Arg Arg Arg Phe
1               5                   10                  15


Asn Ser Ala Asp Tyr Lys Gly Pro Arg Arg Arg Lys Ala Asp
            20                  25                  30


<210>  51
<211>  31
<212>  PRT
<213>  Artificial Sequence

<220>
<223>  Artificial peptide


<220>
<221>  MOD_RES
<222>  (1)..(1)
<223>  FITC-coupled Tyr

<220>
<221>  MOD_RES
<222>  (31)..(31)
<223>  C-terminal amidation

<400>  51

Tyr Gly Arg Lys Lys Arg Arg Gln Arg Arg Arg Asp Gly Arg Arg Arg
1               5                   10                  15


Phe Asn Ser Ala Asp Tyr Lys Gly Pro Arg Arg Arg Lys Ala Asp
            20                  25                  30


<210>  52
<211>  19
<212>  PRT
<213>  Artificial Sequence

<220>
<223>  Artificial peptide

<400>  52

Asp Arg Arg Arg Phe Asn Ser Ala Asp Tyr Lys Gly Pro Arg Arg Arg
1               5                   10                  15

Lys Ala Asp

<210>  53
<211>  11
<212>  PRT
<213>  Artificial Sequence

<220>
<223>  Artificial peptide

<400>  53

Tyr Gly Arg Lys Lys Arg Arg Gln Arg Arg Arg
1               5                   10

<210>  54
<211>  108
<212>  DNA
<213>  Artificial Sequence

<220>
<223>  Artificial nucleic acid

<400>  54
agtaagccgc gcgaatgggt cgaggccgtc gcctatgtcg ggcctgatcg ccgtcgtttc      60

aactcggcgg actataaggg gccgcgcaag cgcaaggcgg acgccagc                  108

<210>  55
<211>  108
<212>  DNA
<213>  Artificial Sequence

<220>
<223>  Artificial nucleic acid

<400>  55
agtaagccgc gcgaatgggt cgaggccgtc gcctatgtcg ggcctgatcg ccgtcgtttc      60

aactcggcgg actataaggg gccgcgcagg cgcaaggcgg acgccagc                  108

<210>  56
<211>  57
<212>  DNA
<213>  Artificial Sequence

<220>
<223>  Artificial nucleic acid

<400>  56
gatcgccgtc gtttcaactc ggcggactat aaggggccgc gcaggcgcaa ggcggac          57


<210>  57
<211>  81
<212>  DNA
<213>  Artificial Sequence

<220>
<223>  Artificial nucleic acid

<400>  57
cgtagacgac gccgtcggcg tagggatcgc cgtcgtttca actcggcgga ctataagggg      60

ccgcgcaggc gcaaggcgga c                                                81


<210>  58
<211>  93
<212>  DNA
<213>  Artificial Sequence

<220>
<223>  Artificial nucleic acid

<400>  58
tatggcagga agaagcggag acagcgacga agagatggtc gccgtcgttt caactcggcg      60

gactataagg ggccgcgcag gcgcaaggcg gac                                   93


<210>  59
<211>  19
<212>  PRT
<213>  Artificial Sequence

<220>
<223>  c-di-GMP binding peptide


<220>
<221>  MISC_FEATURE
<222>  (1)..(1)
<223>  Position 1 is (i) a deletion, (ii) an amino acid having a
       negative net charge at pH 7, or (iii) glycine or a glycine
       derivative.

<220>
<221>  MISC_FEATURE
<222>  (2)..(9)
<223>  Positions 2 to 9 are independently of each other any amino acid
       (X2 to X9) or a deletion, wherein at most one of X2, X3, X4, X5,
       X6, X7, X8, X9, X11, X13, X17 or X18 is a deletion.

<220>
<221>  MISC_FEATURE
<222>  (10)..(10)
<223>  Position 10 is an aromatic amino acid.

<220>
<221>  MISC_FEATURE
<222>  (11)..(11)

<223>  Position 11 is any amino acid (X11) or a deletion, wherein at
       most one of X2, X3, X4, X5, X6, X7, X8, X9, X11, X13, X17 or X18
       is a deletion.

<220>
<221>  MISC_FEATURE
<222>  (12)..(12)
<223>  Position 12 is glycine or a glycine derivative.

<220>
<221>  MISC_FEATURE
<222>  (13)..(13)
<223>  Position 13 is any amino acid (X13) or a deletion, wherein at
       most one of X2, X3, X4, X5, X6, X7, X8, X9, X11, X13, X17 or X18
       is a deletion.

<220>
<221>  MISC_FEATURE
<222>  (14)..(16)
<223>  Positions 14 to 16 are independently of each other any basic
       amino acid.

<220>
<221>  MISC_FEATURE
<222>  (17)..(18)
<223>  Positions 17 and 18 are independently of each other any amino
       acid (X17 and X18) or a deletion, wherein at most one of X2, X3,
       X4, X5, X6, X7, X8, X9, X11, X13, X17 or X18 is a deletion.

<220>
<221>  MISC_FEATURE
<222>  (19)..(19)
<223>  Position 19 is (i) a deletion, (ii) an amino acid having a
       negative net charge at pH 7, or (iii) glycine or a glycine
       derivative.

<400>  59

Xaa Xaa Xaa Xaa Xaa Xaa Xaa Xaa Xaa Xaa Xaa Xaa Xaa Xaa Xaa Xaa
1               5                   10                  15

Xaa Xaa Xaa

<210>  60
<211>  19
<212>  PRT
<213>  Artificial Sequence

<220>
<223>  Artificial peptide

<400>  60

Ala Arg Arg Arg Phe Asn Ser Ala Asp Tyr Lys Gly Pro Arg Arg Arg
1               5                   10                  15

Lys Ala Asp

<210>  61
<211>  19
<212>  PRT
<213>  Artificial Sequence

<220>
<223>  Artificial peptide

<400>  61

Asp Ala Arg Arg Phe Asn Ser Ala Asp Tyr Lys Gly Pro Arg Arg Arg
1               5                   10                  15

Lys Ala Asp

<210>  62
<211>  19
<212>  PRT
<213>  Artificial Sequence

<220>
<223>  Artificial peptide

<400>  62

Asp Arg Ala Arg Phe Asn Ser Ala Asp Tyr Lys Gly Pro Arg Arg Arg
1               5                   10                  15

Lys Ala Asp

<210>  63
<211>  19
<212>  PRT
<213>  Artificial Sequence

<220>
<223>  Artificial peptide

<400>  63

Asp Arg Arg Ala Phe Asn Ser Ala Asp Tyr Lys Gly Pro Arg Arg Arg
1               5                   10                  15

Lys Ala Asp

<210>  64
<211>  19
<212>  PRT
<213>  Artificial Sequence

<220>
<223>  Artificial peptide

<400>  64

Asp Arg Arg Arg Ala Asn Ser Ala Asp Tyr Lys Gly Pro Arg Arg Arg
1               5                   10                  15

Lys Ala Asp

<210>  65
<211>  19
<212>  PRT
<213>  Artificial Sequence

<220>
<223>  Artificial peptide

<400>  65

Asp Arg Arg Arg Phe Ala Ser Ala Asp Tyr Lys Gly Pro Arg Arg Arg
1               5                   10                  15

Lys Ala Asp

<210>  66
<211>  19
<212>  PRT
<213>  Artificial Sequence

<220>
<223>  Artificial peptide

<400>  66

Asp Arg Arg Arg Phe Asn Ala Ala Asp Tyr Lys Gly Pro Arg Arg Arg
1               5                   10                  15

Lys Ala Asp

<210>  67
<211>  19
<212>  PRT
<213>  Artificial Sequence

<220>
<223>  Artificial peptide

<400>  67

Asp Arg Arg Arg Phe Asn Ser Leu Asp Tyr Lys Gly Pro Arg Arg Arg
1               5                   10                  15

Lys Ala Asp

<210>  68
<211>  19
<212>  PRT
<213>  Artificial Sequence

<220>
<223>  Artificial peptide

<400>  68

Asp Arg Arg Arg Phe Asn Ser Ala Ala Tyr Lys Gly Pro Arg Arg Arg
1               5                   10                  15

Lys Ala Asp

<210>  69
<211>  19
<212>  PRT
<213>  Artificial Sequence

<220>
<223>  Artificial peptide

<400>  69

Asp Arg Arg Arg Phe Asn Ser Ala Asp Tyr Ala Gly Pro Arg Arg Arg
1               5                   10                  15

Lys Ala Asp

<210>  70
<211>  19
<212>  PRT
<213>  Artificial Sequence

<220>
<223>  Artificial peptide

<400>  70

Asp Arg Arg Arg Phe Asn Ser Ala Asp Tyr Lys Gly Ala Arg Arg Arg
1               5                   10                  15

Lys Ala Asp

<210>  71
<211>  18
<212>  PRT
<213>  Artificial Sequence

<220>
<223>  Artificial peptide

<400>  71

Asp Arg Arg Phe Asn Ser Ala Asp Tyr Lys Gly Pro Arg Arg Arg Lys
1               5                   10                  15

Ala Asp

<210>  72
<211>  18
<212>  PRT
<213>  Artificial Sequence

<220>
<223>  Artificial peptide

<400>  72

Asp Arg Arg Arg Asn Ser Ala Asp Tyr Lys Gly Pro Arg Arg Arg Lys
1               5                   10                  15

Ala Asp

<210>  73
<211>  18
<212>  PRT
<213>  Artificial Sequence

<220>
<223>  Artificial peptide

<400>  73

Asp Arg Arg Arg Phe Ser Ala Asp Tyr Lys Gly Pro Arg Arg Arg Lys
1               5                   10                  15

Ala Asp

<210>  74
<211>  18
<212>  PRT
<213>  Artificial Sequence

<220>
<223>  Artificial peptide

<400>  74

Asp Arg Arg Arg Phe Asn Ala Asp Tyr Lys Gly Pro Arg Arg Arg Lys
1               5                   10                  15

Ala Asp

<210>  75
<211>  18
<212>  PRT
<213>  Artificial Sequence

<220>
<223>  Artificial peptide

<400>  75

Asp Arg Arg Arg Phe Asn Ser Asp Tyr Lys Gly Pro Arg Arg Arg Lys
1               5                   10                  15

Ala Asp

<210>  76
<211>  18
<212>  PRT
<213>  Artificial Sequence

<220>
<223>  Artificial peptide

<400>  76

Asp Arg Arg Arg Phe Asn Ser Ala Tyr Lys Gly Pro Arg Arg Arg Lys
1               5                   10                  15

Ala Asp

<210>  77
<211>  19
<212>  PRT
<213>  Artificial Sequence

<220>
<223>  Artificial peptide

<400>  77

Asp Arg Arg Arg Phe Asn Ser Ala Asp Tyr Lys Gly Pro Arg Arg Arg
1               5                   10                  15

Ala Ala Asp

<210>  78
<211>  19
<212>  PRT
<213>  Artificial Sequence

<220>
<223>  Artificial peptide

<400>  78

Asp Arg Arg Arg Phe Asn Ser Ala Asp Tyr Lys Gly Pro Arg Arg Arg
1               5                   10                  15

Lys Leu Asp

<210>  79
<211>  19
<212>  PRT
<213>  Artificial Sequence

<220>
<223>  Artificial peptide

<400>  79

Asp Arg Arg Arg Phe Asn Ser Ala Asp Tyr Lys Gly Pro Arg Arg Arg
1               5                   10                  15

Lys Ala Ala

<210>  80
<211>  21
<212>  PRT
<213>  Artificial Sequence

<220>
<223>  Artificial peptide

<400>  80

Arg Asp Arg Arg Arg Phe Asn Ser Ala Asp Tyr Lys Gly Pro Arg Arg
1               5                   10                  15

Arg Lys Ala Asp Ala
            20

<210>  81
<211>  24
<212>  PRT
<213>  Artificial Sequence

<220>
<223>  Artificial peptide

<400>  81

Arg Arg Arg Arg Arg Arg Arg Arg Arg Phe Asn Ser Ala Asp Tyr Lys
1               5                   10                  15

Gly Pro Arg Arg Arg Lys Ala Asp
            20

<210>  82
<211>  25
<212>  PRT
<213>  Artificial Sequence

<220>
<223>  Artificial peptide

<400>  82

Arg Arg Arg Arg Arg Arg Asp Arg Arg Arg Phe Asn Ser Ala Asp Tyr
1               5                   10                  15

Lys Gly Pro Arg Arg Arg Lys Ala Asp
            20                  25

<210>  83
<211>  26
<212>  PRT
<213>  Artificial Sequence

<220>
<223>  Artificial peptide

<400>  83

Arg Arg Arg Arg Arg Arg Arg Asp Arg Arg Arg Phe Asn Ser Ala Asp
1               5                   10                  15

Tyr Lys Gly Pro Arg Arg Arg Lys Ala Asp
            20                  25

<210>  84
<211>  27
<212>  PRT
<213>  Artificial Sequence

<220>
<223>  Artificial peptide

<400>  84

Arg Arg Arg Arg Arg Arg Arg Arg Asp Arg Arg Arg Phe Asn Ser Ala
1               5                   10                  15

Asp Tyr Lys Gly Pro Arg Arg Arg Lys Ala Asp
            20                  25

<210>  85
<211>  28
<212>  PRT
<213>  Artificial Sequence

<220>
<223>  Artificial peptide

<400>  85

Arg Arg Arg Arg Arg Arg Arg Arg Arg Asp Arg Arg Arg Phe Asn Ser
1               5                   10                  15

Ala Asp Tyr Lys Gly Pro Arg Arg Arg Lys Ala Asp
            20                  25

<210>  86
<211>  30
<212>  PRT
<213>  Artificial Sequence

<220>
<223>  Artificial peptide

<400>  86

Tyr Gly Arg Lys Lys Arg Arg Gln Arg Arg Arg Gly Arg Arg Arg Phe
1               5                   10                  15

Asn Ser Ala Asp Tyr Lys Gly Pro Arg Arg Arg Lys Ala Asp
            20                  25                  30

<210>  87
<211>  31
<212>  PRT
<213>  Artificial Sequence

<220>
<223>  Artificial peptide

<400>  87

Tyr Gly Arg Lys Lys Arg Arg Gln Arg Arg Arg Asp Gly Arg Arg Arg
1               5                   10                  15

Phe Asn Ser Ala Asp Tyr Lys Gly Pro Arg Arg Arg Lys Ala Asp
            20                  25                  30